(19) **Europäisches Patentamt / European Patent Office / Office européen des brevets**

(11) **EP 4 687 934 B1**

(12) **EUROPEAN PATENT SPECIFICATION**

(45) Date of publication and mention of the grant of the patent:
**18.03.2026 Bulletin 2026/12**

(21) Application number: **24837477.9**

(22) Date of filing: **23.12.2024**

(51) International Patent Classification (IPC):
*A61K 36/11* (2006.01)  *G01N 33/50* (2006.01)
*G01N 33/575* (2026.01)  *A61K 33/06* (2006.01)
*A61K 35/57* (2015.01)  *A61K 35/614* (2015.01)
*A61K 36/07* (2006.01)  *A61K 36/09* (2006.01)
*A61K 36/48* (2006.01)  *A61K 36/736* (2006.01)
*A61K 36/752* (2006.01)  *A61K 36/88* (2006.01)
*A61P 19/10* (2006.01)

(52) Cooperative Patent Classification (CPC):
(C-Sets available)
**A61P 19/10; A61K 33/06; A61K 35/57;
A61K 35/614; A61K 36/071; A61K 36/09;
A61K 36/11; A61K 36/48; A61K 36/736;
A61K 36/752; A61K 36/88; G01N 33/5008**   (Cont.)

(86) International application number:
**PCT/IB2024/063120**

(87) International publication number:
**WO 2025/149823 (17.07.2025 Gazette 2025/29)**

(54) **A NATURAL MATRIX MIXTURE FOR THE TREATMENT OF BONE FRAGILITY**

NATÜRLICHE MATRIXMISCHUNG ZUR BEHANDLUNG VON KNOCHENBRÜCHIGKEIT

MÉLANGE DE MATRICE NATURELLE POUR LE TRAITEMENT DE LA FRAGILITÉ OSSEUSE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.01.2024 PCT/IB2024/050280
09.05.2024 PCT/IB2024/054526
17.06.2024 PCT/IB2024/055892**

(43) Date of publication of application:
**11.02.2026 Bulletin 2026/07**

(60) Divisional application:
**25212685.9 / 4 696 314**

(73) Proprietor: **Bios-Therapy, Physiological Systems for
Health S.p.A.
52037 Sansepolcro (AR) (IT)**

(72) Inventors:
• **MERCATI, Valentino
52037 Sansepolcro (AR) (IT)**
• **LUCCI, Jacopo
52037 Sansepolcro (AR) (IT)**

(74) Representative: **Predazzi, Valentina
Società Italiana Brevetti S.p.A.
Piazza di Pietra, 39
00186 Roma (IT)**

(56) References cited:
WO-A1-2023/203531    KR-A- 20100 056 329
US-A1- 2009 098 222

- **JOHNSON JEYAKUMAR S. ET AL: "A comparative study of surface modified nanohydroxyapatite using PVA polymer extracted from Seashells, Coral Skeletons and Eggshells for biomedical applications", SURFACES AND INTERFACES, vol. 42, 1 November 2023 (2023-11-01), pages 103401, XP093266045, ISSN: 2468-0230, Retrieved from the Internet <URL:https://pdf. sciencedirectassets.com/314133/ 1-s2.0-S2468023023X00078/ 1-s2.0-S246802302300771X/main.pdf? hash=d3b27c336beb177eef226 be4272547768f6cbd0dc48f32926fe1744cf6ec d585&host=68042c943591013ac2b2430a89b270 f6af2c76d8dfd086a07176afe7c76c2c61&pii= S246802302300771X& tid=spdf-6ac67008-c07b-4671-a742-19f> DOI: 10.1016/j.surfin.2023.103401**

(52) Cooperative Patent Classification (CPC): (Cont.)

C-Sets
**A61K 33/06, A61K 2300/00;
A61K 35/57, A61K 2300/00;
A61K 35/614, A61K 2300/00;
A61K 36/071, A61K 2300/00;
A61K 36/09, A61K 2300/00;
A61K 36/11, A61K 2300/00;
A61K 36/48, A61K 2300/00;
A61K 36/736, A61K 2300/00;
A61K 36/752, A61K 2300/00;
A61K 36/88, A61K 2300/00**

**Description**

## TECHNICAL FIELD OF THE INVENTION AND BACKGROUND ART

The present invention relates to the exclusive selection and use of native matrices, appropriately processed through specific processes and methods, to create final products intended for therapeutic or beneficial purposes, for preventing deviations from balanced physiological states or for restoring physiological states such as in the bone fragility area. Every phase of the production process of such products is under the aegis of the One Health principle (which is a principle that recognizes the interconnectedness of human health, animal health, and environmental health), therefore, the use of artificial forces or substances is not permitted.

Indeed, in the field of the present invention, a fundamental requirement is represented by the fact that the final product, that is a product comprising or consisting of one or more natural matrices, must maintain the natural intelligence i.e. the imprint of the domain of the living to which each constituent of the product belongs, thereby maintaining a network capable of interconnecting and recognizing itself with other networks, whether natural or artificial, that is, originally natural networks that have acquired a degree of artificiality due to interaction with artificial components. This interconnection is deemed to be fundamental to rebalance any disturbances in the network of events that are active in each interacting biological system. Every matrix identified will present bio-physical specifications such that it will represent an invention on its own.

Each network will be characterized by the establishment of connections within the matrices in the final product and within the physiological actions exerted by the product on the receiving organism. The production validation of the product can be carried out and confirmed using probabilistic models based on the link between the conservation of a physiological activity profile and descriptors of the matrix per se generated using multiple bio-physical analytical systems, including spectroscopy (NIR and other techniques), spectrophotometry (target and untarget metabolomics), and paper or X-ray crystallography (fractal measurements). Although useful, the conventional molecular chemical definitions of individual substances contained in matter cannot be used for validating this kind of products as they are not representative of the overall effectiveness and quality thereof.

The selection of matrices intended for administration must be validated according to the updated and specific current taxonomic criteria for the animal, plant and mineral kingdoms. In case of use in combination with natural physical phenomena, it will be necessary to validate the relationship between action and effectiveness on a case-by-case basis, considering sound effects (music or other forms) and those in the field of wave-particles, including those of a quantum nature.

In the current state of the art, it will not always be possible to outline a fully described mechanism of action; however, it will be possible to validate the action and reaction in the interconnection of the respective networks, already validated at a biophysical level.

The invention aims to select and provide new entities or products, as well as systems capable of rebalancing, activating or limiting physiological functions in specific metabolic states of living organisms, that are always in continuous transformation.

The preparations conceived in this way can rebalance the psycho-neuro-endocrine-immunological system, considered as a single system that governs and manages all the other systems.

The invention contributes to a new state of the art that goes beyond the alchemical technologies in the medical field, the beginning of which can be traced back to the early years of the sixteenth century, bringing products and processes back to the conceptual One Health objective already mentioned. The invention proposes a new declination of artificial technologies and of those that naturally self-assemble matter, recognizing existing rules or finding new ones in order to guarantee the constitution of entities that can be validated, mainly based on the concept of validating of their effect and activity on other organisms. The latter, being living beings in continuous transformation, require an evaluation of their physiological state within defined intervals, which is a concept that today falls within the personalized medicine. The present invention fits into the concept of scientificity, understood as the set of knowledge that can demonstrably validate the effects of theoretical modes of action. Today, these methods find application in the establishment of interconnections between all forms of life, in a context in which technological innovations advance at such a pace as to risk compromising the interconnection between human-generated (artificial) intelligence and natural.

Since the activities of the invention herein disclosed are not currently covered by the state of the art, it will be necessary to consider the entire product cycle, from the end user to the social context concerned, under the concept of One Health.

The operational paradigm within which the invention was prepared has been herein denominated as "Bios Physiological Health".

This paradigm aims to introduce into the field of medical art an innovative approach for the treatment and self-management of health using natural matrices, alone or in combination, in order to rebalance the normal physiological states of various living entities, including humans, through endogenous physiological actions triggered by the product. It is a matter of identifying, selecting and assembling natural entities which possess emerging properties, validable through the final product's physiological mode of an action and other methods evolved in recent decades.

A reading of the context according to both technical-scientific and humanistic canons, integrated in their transversality, constitutes the foundation of the proposed invention. Although some of the properties of each matrix part of the product may already be known the emerging properties of the new composition are unexpected.

Of particular relevance is the role of determining the genetic and epigenetic aspects determining the network representing natural matrices and their description at the level of their specific isotopic abundance.

In order to fulfil the Bios Physiological Health paradigm, each phase of processing, from the selection of the reproductive material to the agricultural and industrial phases, up to the methods of use, must preserve as much as possible the integrity of the native programming heritage, inserted into the natural intelligence of each entity of creation, at least as far as known in our terrestrial dimension. It will be essential to validate matrices coming from epigenetic realities similar to the reference one, recognized as a Reference Standard for its specific emerging properties on the metabolism of other living beings, including humans. By way of example, one of the factors that negatively influences epigenetic differentiations is represented by different soil conditions, together with circadian, monthly and annual variations. To preserve the properties of the natural system, which are the only ones that can claim physiological interconnection with the whole of creation, it is not possible to use substances derived from alchemical processes, such as distillation, other processes of synthesis or hemisynthesis, or products derived from genetic modifications or genetically modified organisms. A new interpretation of the mysteries of natural programming, responsible for the vital evolution of organic and inorganic matter, is needed. The consolidation of scientific evolution in recent decades allows us to reposition the understanding of the genesis of a progress based on reductionist determinism, founded on the development of alchemical processes starting from the beginning of the 16th century, which in medicine, with Paracelsus, marked the beginning of the current evolutionary process, known as the Anthropocene.

The term Anthropocene describes the current phase of human evolution and can be dated back to different eras. If considered in the context of this invention, the key date can only be 1492, which represents the end of the humanistic/neoplatonic period of the early Renaissance. This period was represented politically by Cosimo the Elder and Lorenzo de' Medici, with artists and scientists such as Piero della Francesca, Luca Pacioli, Leonardo and Dürer. In the 16th century, alchemical research seen as a human possibility of dominion over nature, has evolved until today under the aegis of artificial intelligence as opposed to the natural one, inspired from the biblical thought according to which "man will dominate over all creation", with the aim of improving divine creation.

1492 is a symbolic date: in that year Lorenzo de' Medici and Piero della Francesca died, while Columbus discovered America. The human species abandoned the fifteenth-century Neoplatonic path to follow the Judeo-Catholic one, where the alchemical practices of Paracelsus applied to medicine marked the transition to the Renaissance mannerism of the 1500s, which, up to the present day, has led us towards a full-blown and irreversible sixth extinction.

The present invention, with the demonstration of feasibility of the resulting industrial discoveries in the medical field, but in principle adaptable to any production field, intends to address the change in evolutionary paradigms. We often talk about defending biodiversity without ever addressing the real problem, guiltily obscured, of the billions of tons of exogenous and non-biodegradable artificial substances released into the planetary system, with the certainty of irreversibly poisoning the sources of life, while the "carpe diem" approach prevails over the survival instinct of the species.

The invention is presented primarily in the patent context, with the hope of opening new areas of research that explore and share natural intelligence, rather than artificial intelligence, which, can do very little to stop or slow down the sixth extinction, or to lay the foundations of an alternative progress to the current one. The inventor Valentino Mercati, together with his collaborator Jacopo Lucci, has undertaken the path of researching in nature itself what can be useful to the living systems, and has developed a knowledge in the agricultural and industrial production system for over 40 years, presenting numerous patent applications following this operational strategy. The patents filed in the past relating to the present inventive process are essentially based on instrumental and diagnostic readings based on principles related to chemistry for the connection of physiological actions with the emerging properties of natural matrices and the innate defences of each individual living being with which they interconnect.

The analyses that followed and inspired the approach herein disclosed were unthinkable just a few decades ago, due to the technological impossibility of reading the genetic and epigenetic information written in the cells of every living organism, and the role of atomic isotopic differentiations in the molecular self-assembly and interconnections of every single unity/individuality with the "universe". The conceptual difficulty in moving from the reassuring management parameters of the artificiality of molecules -at least partly purified and linked by powerful thermodynamic forces that allow strong bonds such as covalent ones, which acts on reduced molecular scopes of other organisms- to natural matrices, mysterious by definition and, still viewed today as part therapeutically unreliable, is extremely high.

If a new inventive interpretation is needed for a new medical state of the art after five centuries of alchemical reductionism, this interpretation must connect the most distant concepts and processes in a single application field. This is due, as already expressed, to a philosophical legacy that questions the human condition: the human species was created like all the others by the original vital intelligence with the purpose of life itself, as far as we can assume, to dominate on creation, or has it been experimentally endowed with different faculties from other organisms, already favorably inserted into creation, to constitute a new ecological niche at the service of the universe?

The answer to this dilemma does not arise for the current invention: humanity will have to return to the Neoplatonic thought of the early Renaissance, and the experimental duality of the human species must emancipate itself from the mindset of dominance in order to share its unique faculties within the universe with all of creation. Humanity will need to reconsider the warning of Leonardo da Vinci: "Man can only create his own offspring..." and reflect on the melancholic thoughts of wise figures like Piero della Francesca, Luca Pacioli, and Dürer, regarding the impossibility of understanding and representing the beauty of creation and deciphering its mysteries.

The era has arrived for the acquisition of new research centers in molecular and cellular biology, with an indispensable focus on bioinformatics and the new physical sciences. Today, the inventor can base research strategies and socioeconomic applications in new therapeutic fields, particularly those that are complex and/or chronic-degenerative, where the restoration of metabolic balance for organisms either naturally or artificially disturbed will become an integral part of a future that is already present.

The present invention represents a new vision of the medical art, which reconsiders scientific evolution from a perspective different from reductionist determinism. This alternative progress, in conflict with universal or planetary rules, will have to rely not so much on artificial intelligence and technological advancement, but on the evolution of the laws that regulate our universe and life itself. The transition from the artificial treatment of singled out symptoms, even if seen from a systemic perspective with modern techniques of systems biology, to a holistic approach that embraces the whole, represents the basis of the present progress.

In particular, the present invention discloses and analyses a product consisting of natural matrices from equisetum, acacia, malphigia, coral calcium, egg shell calcium, agave, cetraria, agaricus and calcium citrate, which adjuvate bone homeostasis helping the organism to re-establish a correct bone metabolism by correcting the behavior of stem cells and re-establishing the correct balance between the cell populations that allow bone growth or remodeling. By adjuvating the correct functioning of bone cells, the product achieves beneficial results, obtaining a rewiring of the differentiation process in a direction that favors the bone lineage over the adipocyte one, as well as inducing an improvement of systemic metabolism. The present invention also relates to the use of said product as a template in a method for determining the presence of native natural intelligence in a therapeutic or beneficial product, said product comprising or consisting of natural matrices, through the validation of its therapeutic or beneficial emerging properties.

MATTOLI LUISA ET AL: "Natural complex substances: From molecules to the molecular complexes. Analytical and technological advances for their definition and differentiation from the corresponding synthetic substances",PHYTOCHEMISTRY, vol. 215, 22 July 2023 discloses the use of AMS for analysing, whether a product comes from a natural biological or other source.

It proposes the use of accelerator mass spectrometry (AMS) i.e. 14C testing to distinguish bio-based material form petroleum-based material.

US 2016/024503 A1 (KALLURI RAGHU [US] ET AL) discloses measuring exosomes and miRNA but the method involves detecting biomarkers and not establishing that a product itself is a natural matrix.

## SUMMARY OF THE INVENTION

The invention relates to a method for determining the presence of native natural intelligence in a therapeutic or beneficial product, said product comprising or consisting of natural matrices, through the validation of its therapeutic or beneficial emerging properties, the method comprising the following steps

a. assessing the product or composition naturality by:

1. measuring the 14C activity in said product or composition with ISO-16620-2;2015 (AMS) method,
2. assessing the presence of miRNAs in said product or composition,
3. assessing the presence of exosomes in said product or composition,

b. assessing the presence of therapeutic or beneficial functional resilience between different batches of said product by comparing, batch to batch, the modulation of one or more biological activities underlying the product's desired therapeutic or beneficial effect on a relevant altered physiological state and/or on the pathological condition treated by said product or composition in a cell-based assay whose read-out is representative of the modulation of said one or more biological activities;

c. assessing from the read-out of said cell-based assay, whether the modulation of said biological activities underlying the desired therapeutic or beneficial effect results in the modulation of a whole altered physiological state or pathological condition; and

determining that said product or composition is itself a natural matrix, representing native natural intelligence, wherein native natural intelligence is present when
the measured value for the 14C activity is of 99.82 ± 0,22 % percent, miRNAs, exosomes and therapeutic or functional resilience are detected, and
said modulation in c. results in the modulation of a whole altered physiological state or pathological condition.

The development of the product disclosed in the invention, (in figures and examples also indicated as "product C") is based on the goal of creating a 100% natural matrices-based product with plants and minerals that can support the physiological bone metabolism, specifically in women during menopause which is, as explained above, a physiological transition process that affects various areas of the body and can be associated with potential pathological conditions such as osteoporosis and metabolic syndrome. The adipose tissue, due to epigenetic stimuli that have emerged in modern times and were previously absent or not as invasive, plays an increasingly important role during this transition.

The product herein disclosed, thanks to its emerging properties and its natural matrices composition, is capable of a network (product) over networks (recipient) mechanism and interacts with the physiological metabolic pathways that occur in the body of a woman in menopause or undergoing this transition (pre or peri menopause: Premenopause is the time between a woman's first period and the onset of perimenopause. Perimenopause is the transition phase into menopause that typically lasts about six years). Said product is capable of correctly stimulating the differentiation of mesenchymal stem cells, promoting the development of the osteoblastic cell line and inhibiting the formation of osteoclasts and adipocytes, thus contributing to establishing a virtuous balance in bone metabolism processes. Additionally, it is capable of providing the necessary calcium for the correct deposition of hydroxyapatite crystals within the bone matrix. Results also show that the can reduce, also by modulating osteocalcin expression, both the amount and inflammatory status of adipose tissue, suggesting an anti-adipogenic effect, as well as improve glucose tolerance, indicating further desirable effects in the pathophysiological framework of interest: its local and systemic action can intervene in the dysfunctional loop that occurs in systemic inflammation and metabolic dysregulation, affecting the differentiation of mesenchymal stem cells into white adipocytes and their subsequent accumulation.

The reduction of bone resorption induced by the product, as disclosed in the examples and in the figures, plays a fundamental role in the maintenance and repair of the bone itself, promoting the rebalancing of bone density and structure. In addition, the product of the invention, has shown a systemic metabolic regulation effect, inducing the expression levels of Osteocalcin (OCN), a hormone also responsible for modulating insulin metabolism. Osteocalcin is expressed and secreted by mature osteoblasts and acts by stimulating insulin secretion from pancreatic β-cells and enhancing insulin sensitivity in muscle and white adipose tissue, leading to a reduction in blood glucose levels and the regulation of overall energy expenditure.

During the development of the product, modern technologies and experimental models representative of the physiological transition process of women in menopause from a systemic perspective were applied, in order to define the biological activity of the product. The data obtained and disclosed in the examples and figures demonstrate a dual synergistic and systemic effect of the product which makes it significantly more desirable compared to classic calcium and vitamin D supplements which are only able to partially support the physiological changes of the body. In addition, as evident from the experiments and figures, its beneficial/therapeutic activity is provided through a physiological mechanism of action, meaning that the product interacts with the body according to systems already familiar to it, rather than according to foreign principles imposed by an exogenous entity such as a synthetic molecule. This is why the product of the invention, in addition to containing precursors of Vitamin D and sources of calcium, has been designed in order to interact with the pool of mesenchymal stem cells in the bone and adipose tissue, enabling it to recapitulate all the necessary elements to rebalance proper bone turnover, essential for the formation of a solid and functional structure. Overall, hence, the effects of the product have a systemic projection, contributing to the rebalancing of metabolic disorders and latent inflammation that affect women in menopause on a systemic scale.

These characteristics make the product significantly better than traditional calcium and Vitamin D supplements, which only partially support the physiological changes in the body. Its interaction with mesenchymal stem cells in both bone and adipose tissue, can recalibrate all the necessary elements to restore proper bone turnover, essential for creating a solid and functional structure, and, at the systemic level, to rebalance metabolic dysregulation and reduce inflammation.

Hence, thanks to the presence of complex plant matrices and natural calcium from various sources, the product herein disclosed clearly demonstrates that the complexity of a physiological process can only find the correct synergy when accompanied by a similar level of complexity.

The method of the invention has been thoroughly tested on a product consisting of natural matrices with the following formula

| Natural matrices components | Product composition for a total of 100% |
|---|---|
| Equisetum | 1.4-2.6% |

(continued)

| Natural matrices components | Product composition for a total of 100% |
| --- | --- |
| Acacia | 1.5-2.8% |
| Malpighia | 1.4-2.6% |
| Coral skeleton | 22.4-41.6% |
| Avian Egg shell | 21-39% |
| Agave | 8.4-15.6% |
| Cetraria | 1.4-2.6% |
| Agaricus | 3.2-6% |
| Calcium citrate | 9-17% |

## DETAILED DESCRIPTION OF THE FIGURES

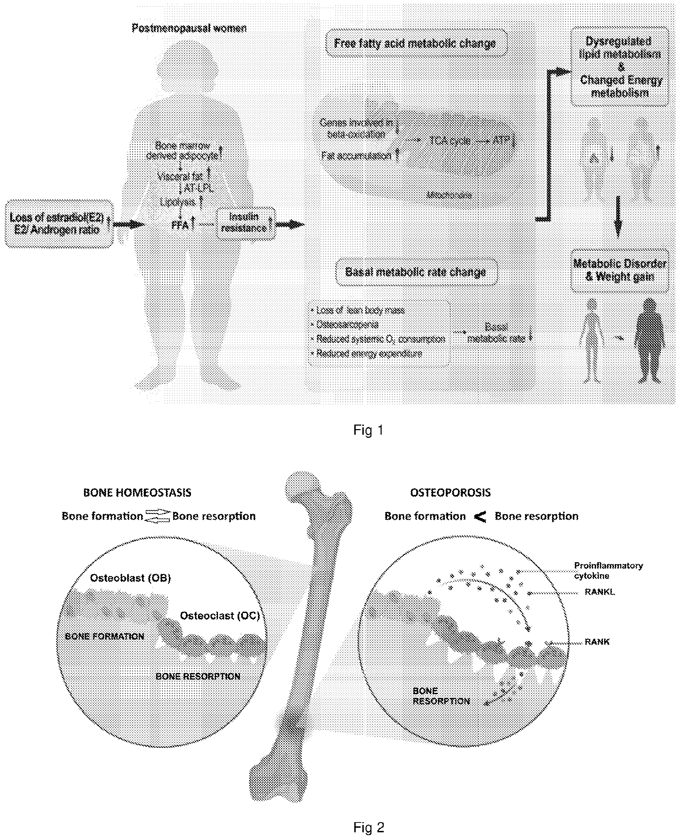

**Figure 1. Lipid metabolism imbalance in postmenopausal women.** Changes in energy metabolism and lipid metabolism that contribute to weight gain in postmenopausal women: Women undergo hormonal changes during menopause, including a reduction in estrogen and an increase in circulating androgens. These changes make postmenopausal women susceptible to changes in body composition, muscle loss and abdominal obesity. In particular, the decline in estrogen leads to an increase in bone marrow-derived adipocytes, which contributes to higher levels of visceral fat in postmenopausal women. Increased lipolysis of visceral fat by adipose tissue lipoprotein lipase produces excess free fatty acids, which can lead to insulin resistance and metabolic disease. The loss of estrogen down-regulates genes involved in β-oxidation, preventing the efficient use of free fatty acids as an energy source. In addition, older women show increased fat accumulation due to the upregulation of genes involved in adipogenesis. As a result, ATP production by β-oxidation decreases while lipid synthesis metabolism increases.

**Figure 2. Bone remodelling.** Bone remodelling is a crucial process that maintains the strength and health of human bones. This process involves bone resorption, controlled by osteoclasts, and bone formation, controlled by osteoblasts. Osteoporosis occurs when there is an imbalance in this process, with bone resorption exceeding bone formation resulting in an inability to ensure proper mineralization of the cellular matrix and a reduction in bone mass and bone functionality. Osteoporosis can lead to fractures that cause pain, disability, reduced quality of life and, in severe cases, death.

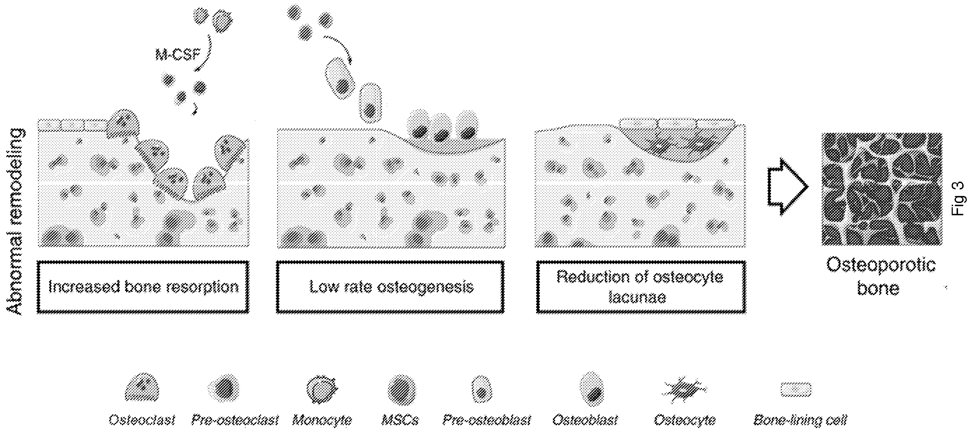

**Figure 3. Bone remodeling in osteoporosis (abnormal bone remodelling).** Abnormal bone remodelling process: Key factors influencing bone formation include a) differentiation of mesenchymal stem cells (MSCs) into osteoblasts, b) MSC differentiation into osteoclasts and adipocytes, and c) deposition of bone matrix. The figure shows abnormal remodelling with increased resorption and reduced osteogenesis, leading to osteoporotic bone. In abnormal remodelling, osteoclasts play a crucial role by excessively breaking down bone tissue, while adipocytes contribute to the imbalance by promoting fat cell formation over bone cell formation. As a result, bone becomes fragile and loses its structure and function.

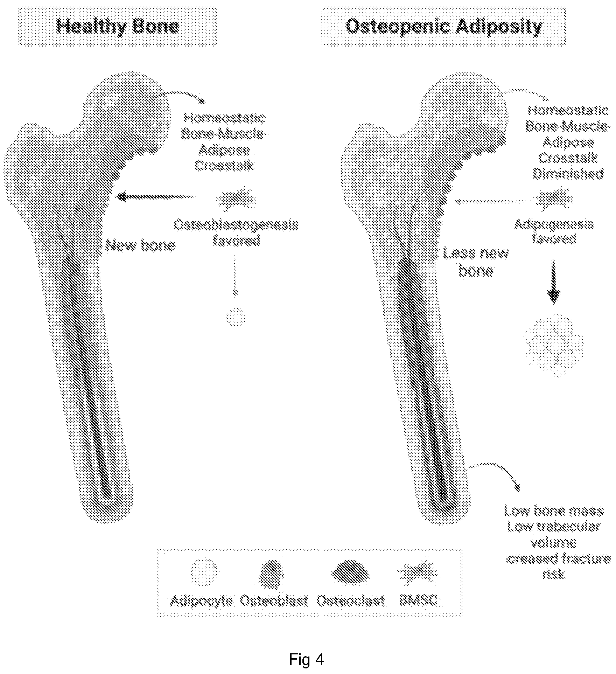

**Figure 4. Influence of adipocyte on bone fragility.** Osteopenic adiposity is characterised by the infiltration of fat into bone, including adipocytes derived from MSC differentiation that is unbalanced relative to the bone cell component, together with bone loss. Inflammation plays an important role in the development of osteopenic adiposity and has been extensively studied. Disruption of communication between bone and adipose tissue is thought to contribute to this condition. The accumulation of fatty tissue in the bone affects the structure of the bone and makes it weaker.

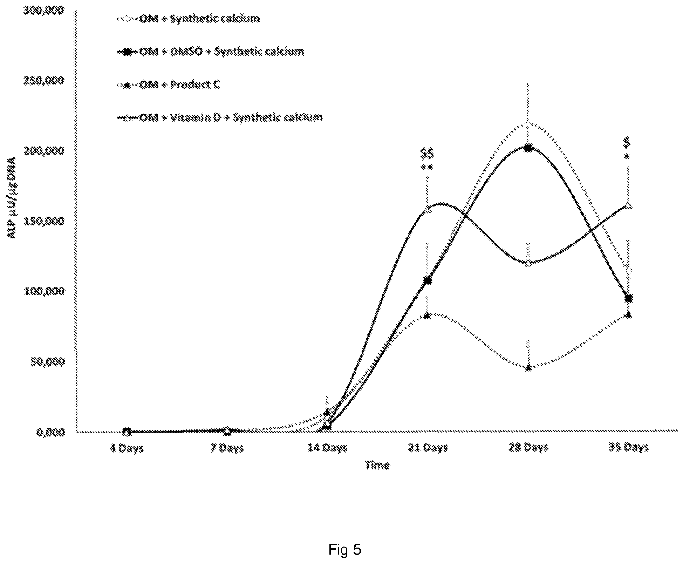

**Figure 5. ALP activity in osteoinductive medium (OM).** The graph illustrates alkaline phosphatase (ALP) activity in hADMSCs cultured in OM, normalized to DNA content (ALP μU/μg DNA) over time (4 to 35 days) under different experimental conditions: Product C, synthetic calcium with or without DMSO vehicle, or Vitamin D + synthetic calcium (DMSO-vehicled). ALP enzyme activity data were calculated as mean $\pm$ standard deviation (SD) of the experimental quadruplicates at the 6 different osteoblast induction time points. Significances were calculated using Anova and Bonferroni test. * = $p < 0.05$ vs. OM + DMSO + Synthetic calcium; ** = $p < 0.01$ vs. OM + DMSO + Synthetic calcium; $ = $p < 0.05$ vs. OM + Synthetic calcium; $$ = $p < 0.01$ vs. OM + Synthetic calcium.

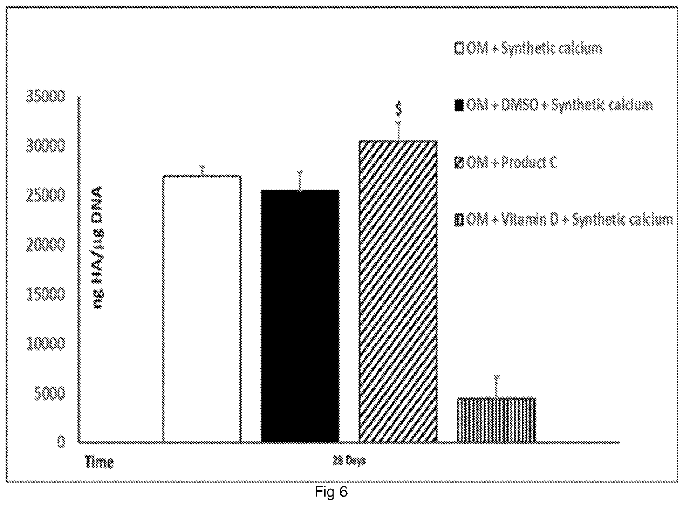

**Figure 6. Hydroxyapatite (HA) deposits in osteoinductive medium (OM).** Quantitative analyses of calcium mineralized deposits in hADMSCs cultured in OM in the presence of Product C, synthetic calcium with or without DMSO vehicle, or Vitamin D + synthetic calcium (DMSO-vehicled), from 4 to 35 days. Data were calculated as mean $\pm$ standard deviation (SD) of the experimental quadruplicates. Significances were calculated using Anova and Bonferroni test. $ = p < 0.05 vs. OM + Synthetic calcium.

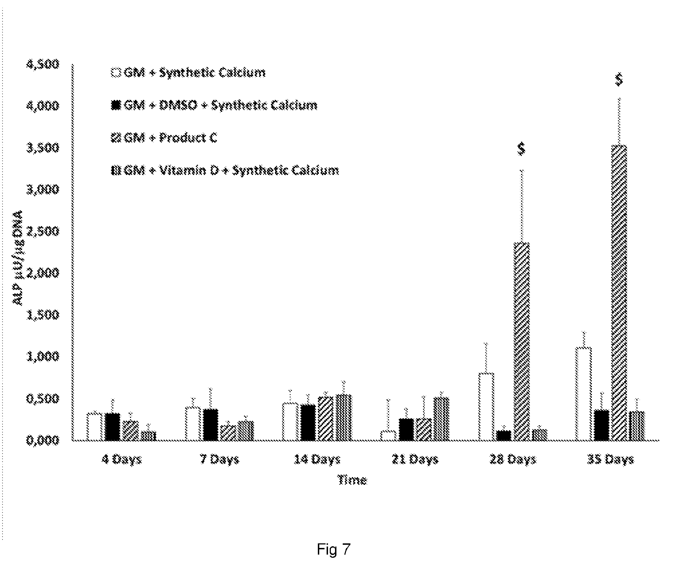

**Figure 7. ALP activity in non-osteoinductive medium (GM).** The graph illustrates alkaline phosphatase (ALP) activity in hADMSCs cultured in GM, normalized to DNA content (ALP U/$\mu$g DNA) over time (4 to 35 days) under different experimental conditions: Product C, synthetic calcium with or without DMSO vehicle, or Vitamin D + synthetic calcium (DMSO-vehicled). ALP enzyme activity data were calculated as mean $\pm$ standard deviation (SD) of the experimental quadruplicates at the 6 different osteoblast induction time points. Significances were calculated using Anova and Bonferroni test. $ = p < 0.05 vs. GM + Synthetic calcium.

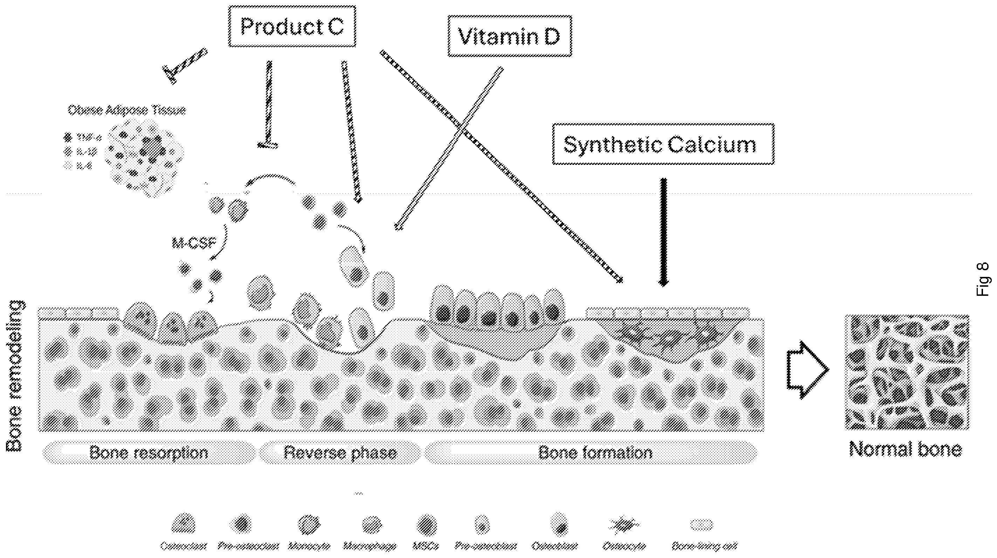

**Figure 8**. **Comparison of Product C activity vs Vitamin D and Calcium in osteoblast differentiation.** The figure shows that the data obtained in the experiments with Vitamin D certainly contributes, but it does not provide a differentiation stimulus by itself and does not directly provide material in the form of calcium to promote the formation of mineralised bone matrix. Synthetic calcium alone is not able to intervene in the differentiation process and can only provide calcium for bone formation. Product C alone is able to recapitulate the entire physiologically active process. The product is able to supply the calcium necessary for the correct deposition of hydroxyapatite crystals in the bone matrix, and also has the ability to correctly stimulate the differentiation of mesenchymal stem cells, favouring the development of the osteoblastic cell line and inhibiting the formation of osteoclasts and adipocytes, both locally and systemically.

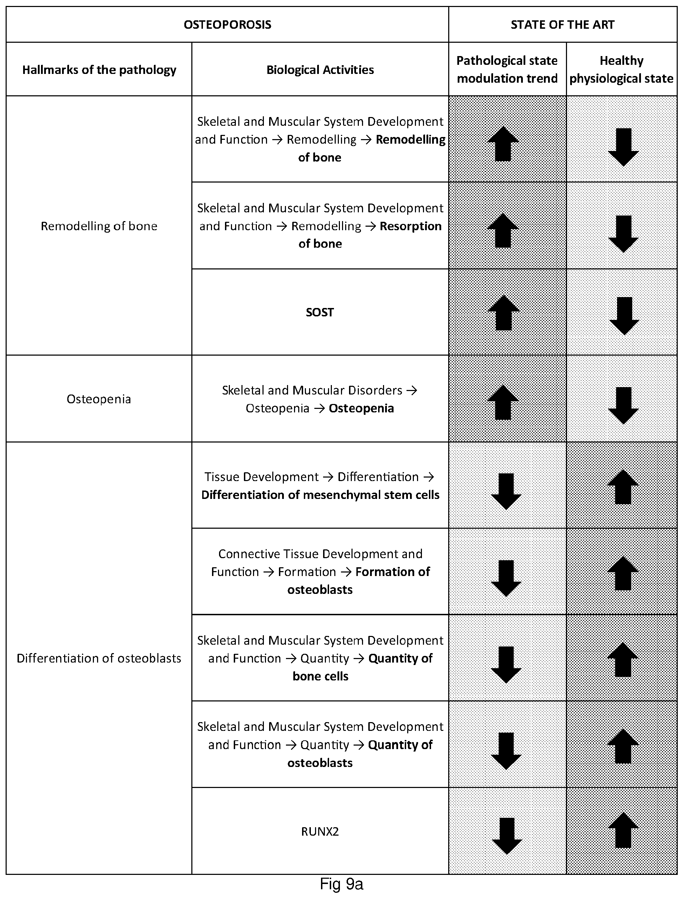

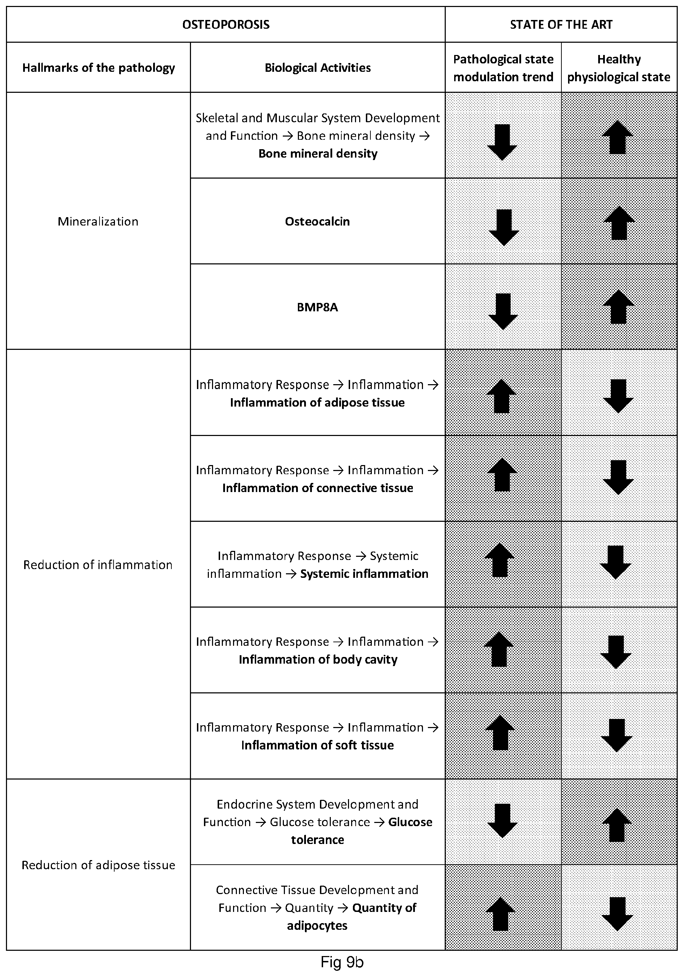

**Figure 9a and 9b** Exemplification of hallmarks of osteoporosis, (column 1), network of biological activities concurring to define pathological state (column 2) and modulations thereof in the pathological state (column 3). Modulation trend of the network of said biological activities concurring to a healthy physiological state (column 4). Dark grey: up modulation light grey: down modulation.

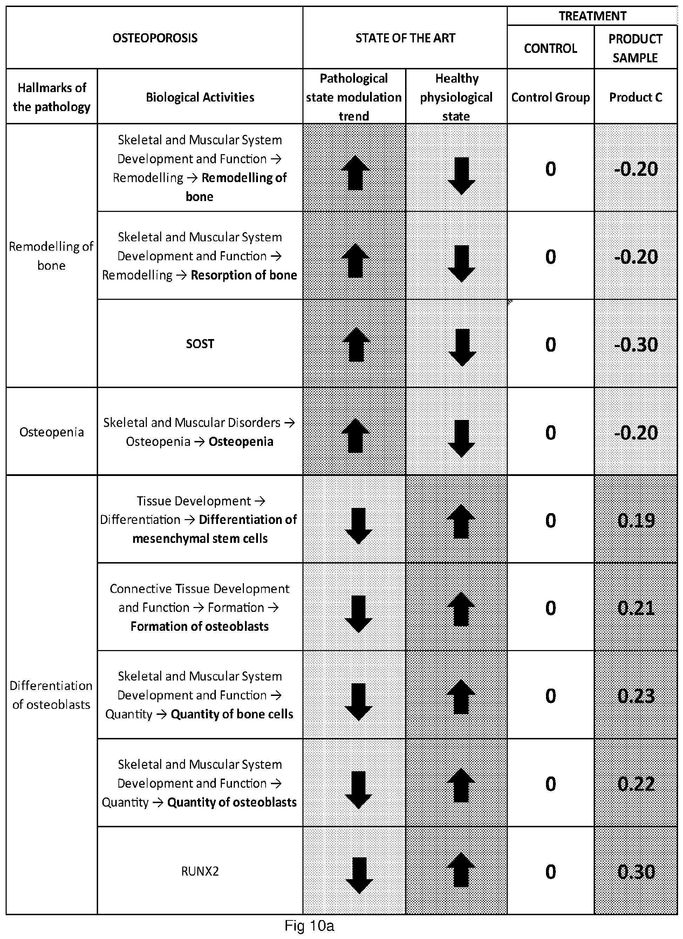

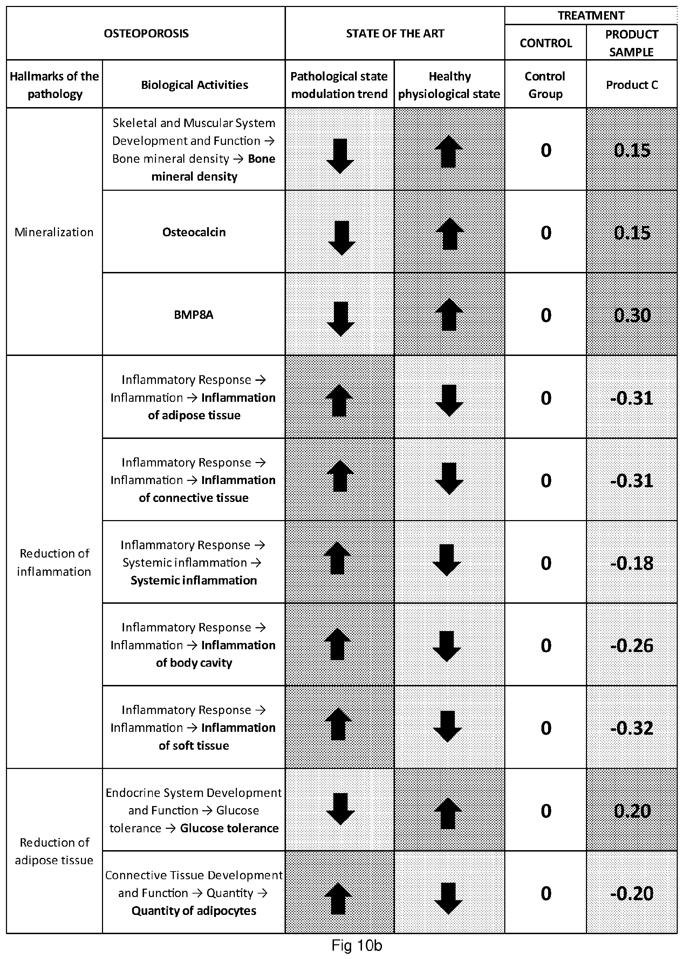

**Figure 10a and 10b** Modulation of a network of selected biological activities in a human, adipocyte-derived, mesenchymal stem cell lines (hADMSC), capable of differentiating into osteoblasts and mineralize the extracellular matrix (ECM) cell-based assay in osteoporosis: column 1 hallmarks, column 2 network of biological activities, column 3 modulation trend of the network of biological activities in the pathological state, column 4 modulation trend of the network of biological activities concurring to a healthy physiological state, column 5 suitably induced cells representing the network of biological activities modulation of the non-modulated state control, column 6 modulation induced by tested product (Product C). The cell-based assay shows that product sample modulates the whole network of selected activities according to the trend concurring to the healthy physiological state profile. The numbers reported in each square represent the value quantifying the modulation (z-score) calculated according to the examples representing the modulation of each biological activity observed.

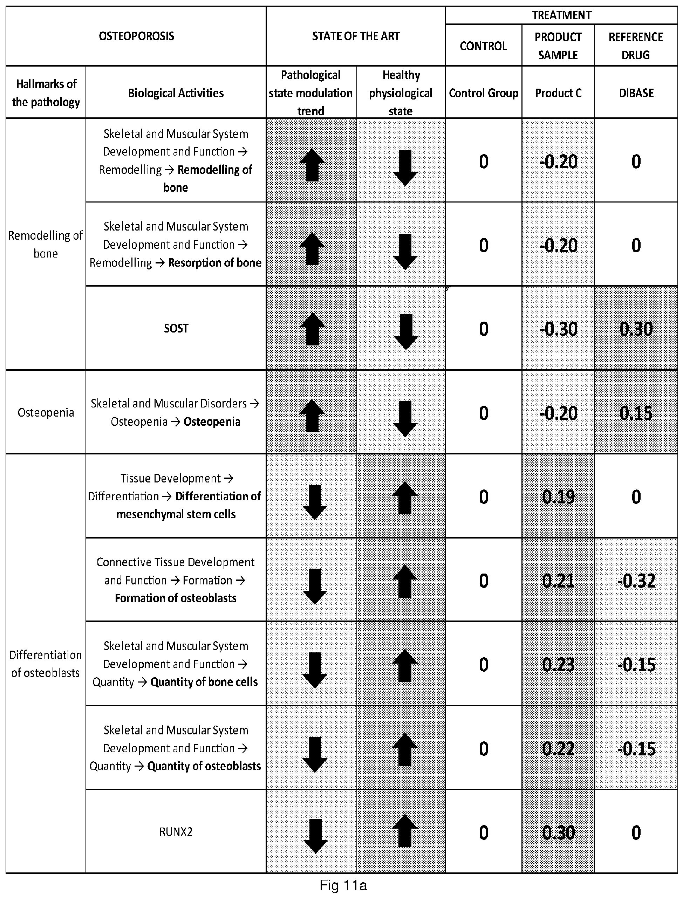

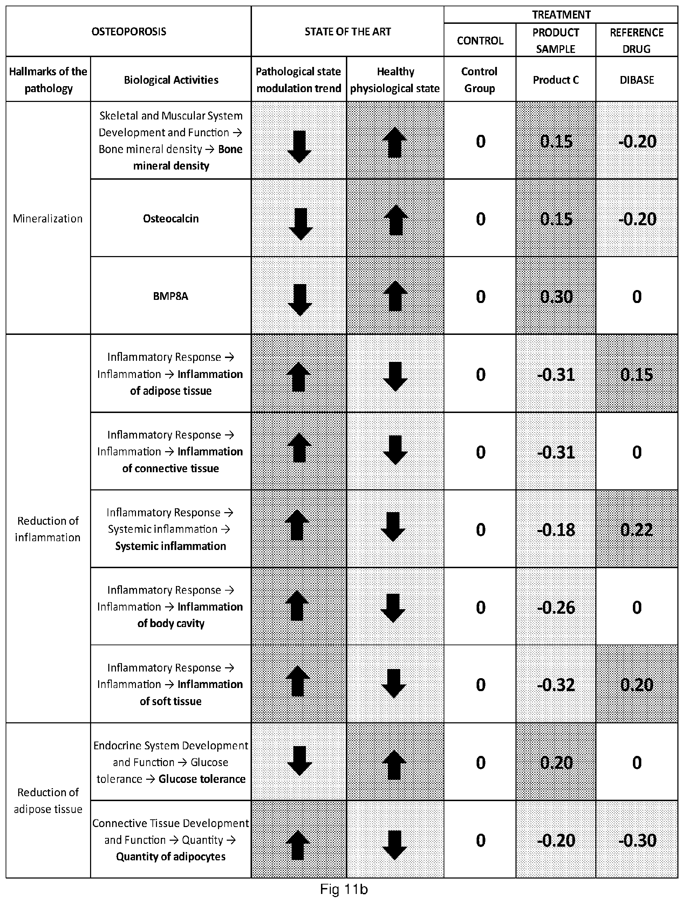

**Figure 11a and 11b** Modulation of a network of selected biological activities in a human, adipocyte-derived, mesenchymal stem cell lines (hADMSC), capable of differentiating into osteoblasts and mineralize the extracellular matrix (ECM) cell-based assay in osteoporosis: column 1 hallmarks, column 2 network of biological activities, column 3 modulation trend of the network of biological activities in the pathological state, column 4 modulation trend of the network of biological activities concurring to the healthy physiological state, column 5 suitably induced cells representing the biological activities network modulation of the non-modulated state control, column 6 modulation induced by tested product (Product C), column 7 modulations induced by a reference drug (DIBASE). The cell-based assay shows that product sample modulates all the selected activities of the network with a modulation trend according to the one concurring to the healthy physiological state profile. On the other hand, the reference drug is not able to effectively modulate all the whole network of biological activities needed to define the healthy physiological state The numbers reported in each square represent the value quantifying the modulation (z-score) calculated according to the examples representing the modulation of each biological activity observed.

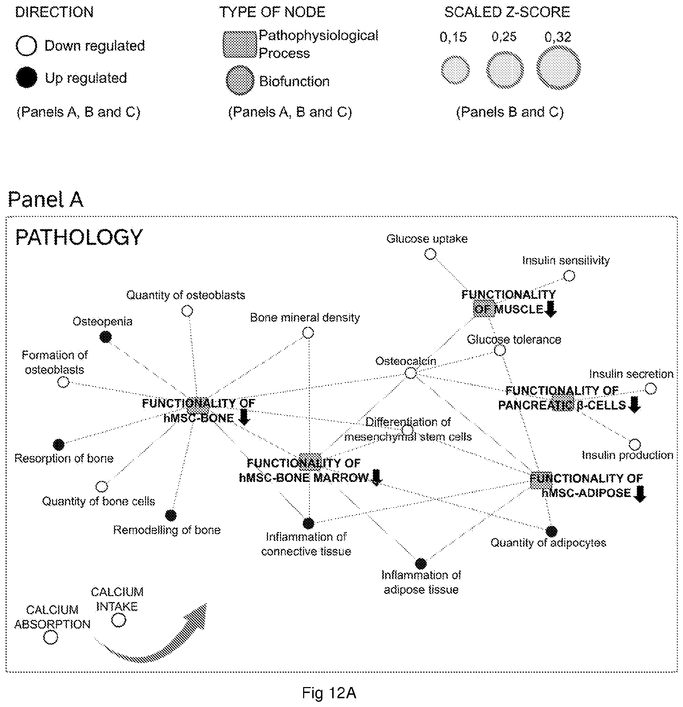

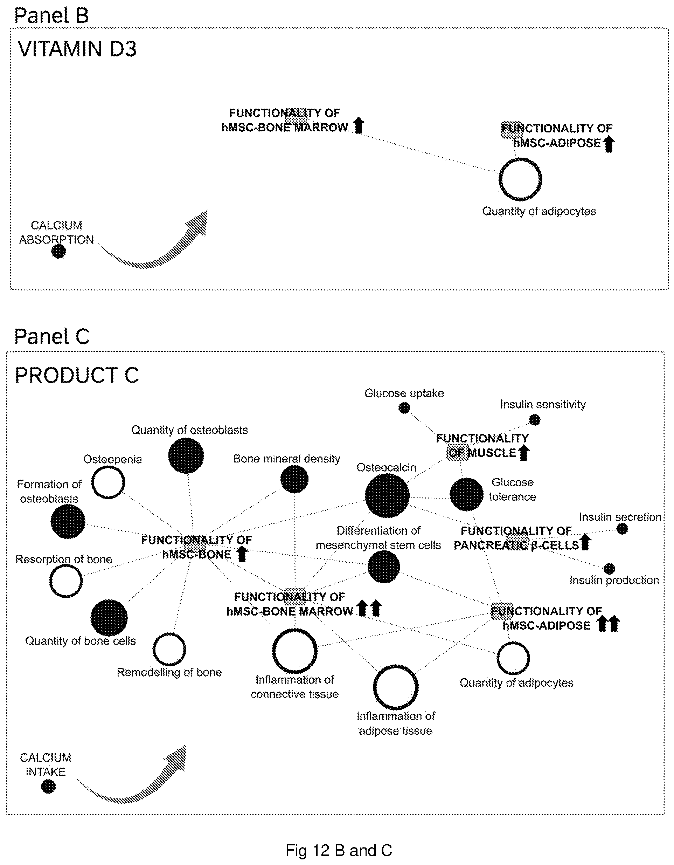

**Figure 12.** network analysis of osteoporosis (12 Panel A) and treatment with reference drug (12 Panel B) vs. treatment with product C (12 Panel C). The gray squares represent both the fundamental nodes characterizing the pathophysiological or altered physiological state and the specific sites through which the pathology interconnects with the body. The arrows next to the nodes indicate the specific modulation for each situation described, and the intensity is shown through multiples of the arrows themselves. The gray squares are in turn linked to the network of biological activities, the modulation of which, has been demonstrated experimentally. These biological activities are represented by black (upregulation) or white (down modulation) circles whose amplitude is directly proportional to the magnitude of their experimentally proved modulation. The network analysis shows that the product C influences the body in a systemic way, modulating a higher number of desired activities, according to the modulation trend concurring to the healthy physiological state, than treatment with reference drug.

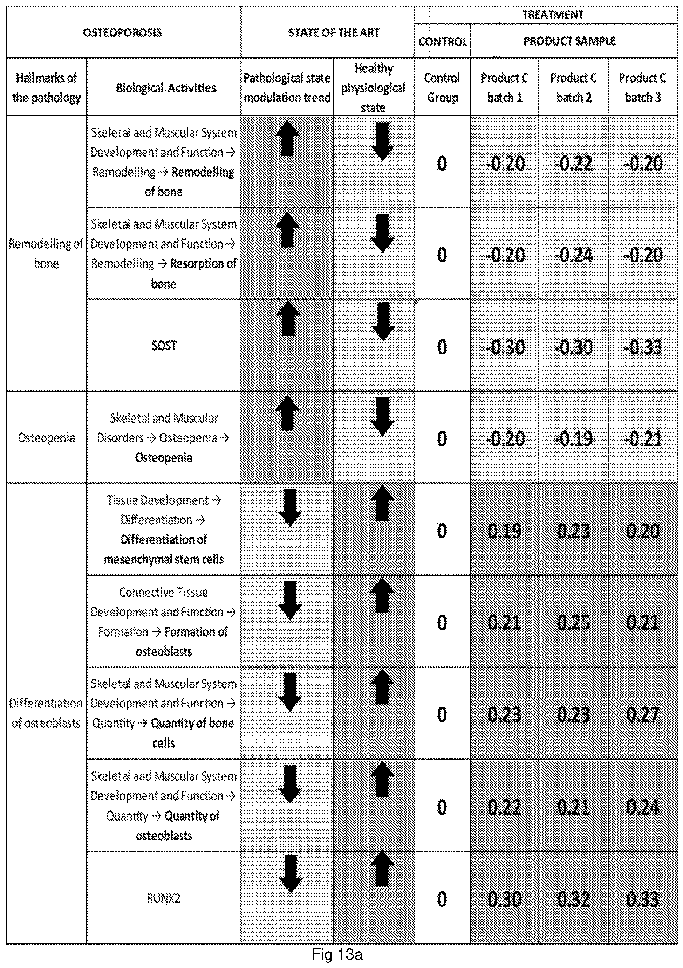

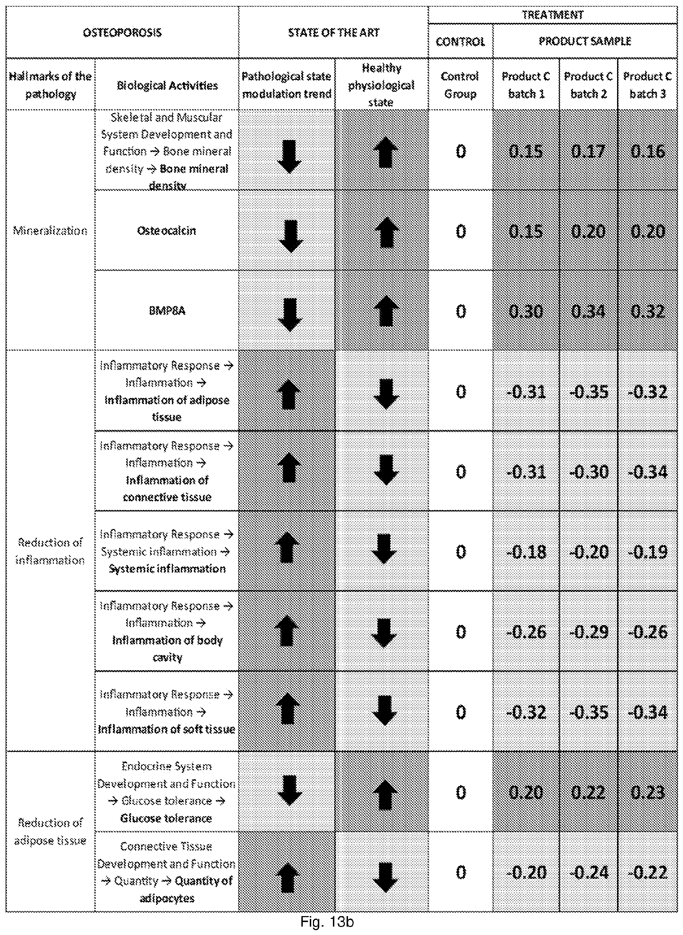

**Figure 13a and 13b.** Modulation of a network of selected biological activities in a human, adipocyte-derived, mesenchymal stem cell lines (hADMSC), capable of differentiating into osteoblasts and mineralize the extracellular matrix (ECM) cell-based assay in osteoporosis: column 1 hallmarks, column 2 network of biological activities, column 3 modulation trend of the network of biological activities in the pathological state, column 4 modulation trend of the network of biological activities concurring to the healthy physiological state, column 5 suitably induced cells representing the biological activities network modulation of the non-modulated state control, column 6 modulation induced by batch 1 of Product C, column 7 modulation induced by batch 2 of Product C, column 8 modulation induced by batch 3 of Product C. The cell-based assay shows that all the tested product batches modulate the network of all the selected activities according to modulation trend concurring to the healthy physiological state profile (modulation of a state and functional resilience). On the other hand, the reference drug is not able to effectively modulate all the biological activities needed to define the healthy physiological state (it does not act on the network). The numbers reported in each square represent the value quantifying the modulation (in terms of z-score) calculated according to the examples representing the modulation of each biological activity of the network observed.

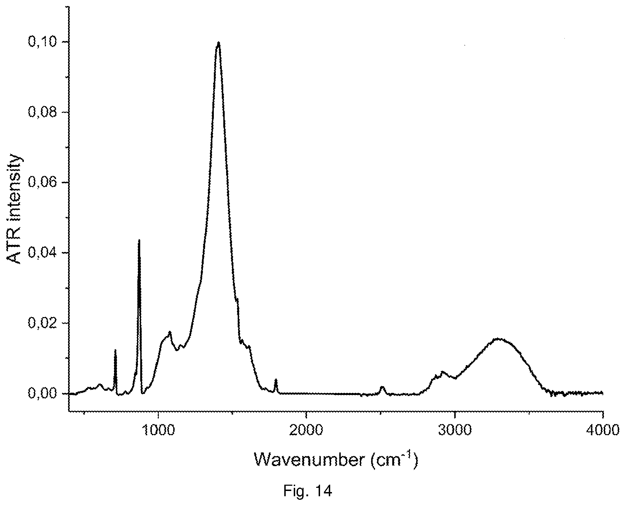

**Figure 14** FTIR spectrum of batch 1 of Product C.

## GLOSSARY

Unless otherwise defined herein, scientific, and technical terms used in connection with the present invention shall have the meanings that are commonly understood by those of ordinary skill in the art. Further, unless otherwise required by context, singular terms shall include pluralities and plural terms shall include the singular.

In the present specification, the term "synthetic calcium" refers to a compound comprising calcium which has been prepared synthetically through chemical reaction in a laboratory.

In the present specification, the term "calcium citrate" refers to a compound obtained by reacting calcium, for example coral calcium, with lemon juice.

In any point of the present specification or of the claims, the expression "comprising" or "comprise(s)" can be replaced by "consisting of" or "consist(s) of".

A "natural matrix" in the present application refers to a material consisting of a network represented by a broad number of components/constituents obtained (e.g. extracted) directly from a member of the natural kingdom or a naturally occurring portion thereof (i.e., from a natural raw source), without significant processing or synthetic alteration, wherein "without significant processing or synthetic alteration" is intended that no denaturing processes are used for obtaining the matrix from the raw source. In other words, the natural raw source is processed only by manual, mechanical or gravitational means e.g. by dissolution in water or other naturally occurring solvents, such as water, water-alcohol solutions etc.; by flotation; by extraction with water or other naturally occurring solvents; by steam distillation or by heating solely to remove water or any other naturally occurring solvent; or extracted from air by any means and with the provision that "natural matrix" excludes said member of the natural kingdom or a naturally occurring portion thereof as such. In particular, according to the invention, a natural matrix is a 100% natural and biodegradable material, consisting of natural components that have not been denatured by the process for the production of the matrix from the starting raw materials without intentional addition of synthetic products along the whole process. In the present description, 100% biodegradable is considered as "readily biodegradable" according to an OECD biodegradability test. These features guarantee the maintenance of the matrix effect which is conferred to the matrix by the presence of structural interactions by its components (material interactions) and functional interactions that become evident upon exposure of a biological system to the natural matrix (immaterial interactions). In other words, a natural matrix, or a mixture of natural matrices, are materials obtained from entities that are self-assembled in nature and processed so to preserve their native bio-physical characteristics which determine their physiological interaction with other living organisms, such as the human organism. Their emerging properties can be expressed by contributing to the rebalancing of metabolic processes or states of the receiving organism and/or of some organs or tissues alongside the physiological actions that will be activated in each specific context. According to the present invention the natural matrix can be from a material obtained from any source in the life kingdoms i.e., Monera, Protista, Fungi, Plantae and Animalia. The term hence encompasses a plant natural matrix, an animal natural matrix, a fungi natural matrix, a protista (archaea or bacteria) natural matrix, a monera natural matrix. A natural matrix may also comprise natural inorganic materials such as minerals obtained from natural raw materials. A synonym of natural matrix or one or more natural matrices in the present description is "complex natural system" or "natural material" as defined below.

An example of naturally occurring portion of an organism may be represented by e.g., roots, leaves, bark, fruit, flower, of a plant or sections thereof, organs, tissues.

In any part of the description the general term natural matrix can be substituted with:

a plant natural matrix or a natural matrix obtained from a plant,
an animal natural matrix or a natural matrix obtained from an animal or from an animal product such as eggs or milk,
a fungi natural matrix or a natural matrix obtained from a fungus,
a protista natural matrix or a natural matrix obtained from a protista,
a monera natural matrix or a natural matrix obtained from a monera,
or with a plant material and/or extract, an extract from an animal tissue or organ, fungi
and/or a fungi extract, or a mixture thereof wherein the extraction process does not encompass denaturing steps (e.g., temperature or the use of denaturing solvents). Plant is synonymous with herb.

The term "natural" matrix emphasizes the retaining the integrity and complexity of networks of constituents/-components as in the original natural source due to the absence of denaturing treatments for the obtainment thereof. A natural matrix hence does not encompass compositions of natural origin that are enriched in specific molecules of artificial synthesis or isolated from a natural raw material. In addition, a natural matrix is obtainable only with processes that do not act through extensive processing or chemical modification, isolation, purification, or molecular extraction.

Due to the supramolecular self-assembly of the constituents/components of a natural matrix and the presence of functional interactions among them, the whole matrix behaves as a complex network that does not interact with a single target molecule but that interacts with a network of recipients (also organised as a network) in the receiving organism. Therefore, the interaction natural matrix-receiving organism is not, as for common pharmaceutical APIs the result of a point-to-point interaction, but the result of an "interactor" networks (i.e., the matrix)-"receiver" network (i.e., the organism to whom the matrix is administered) interaction.

The term natural matrix can be also substituted in any part of the description and claims with complex natural system.

Nowhere in the description and in the claims the term natural matrix can be interpreted as "a product of nature" as such, rather, a natural matrix is a product obtained from a natural organism and processed (e.g. extracted) therefrom by techniques that do not substantially alter biological structure and the relevant supramolecular and functional interconnections among the components within the matrix as mentioned above, i.e., without the use of denaturing techniques and that does not comprise additional isolated or synthesised molecules or classes of molecules.

Emerging properties according to the present description and to the art, the term defines the properties of a natural matrix or of a natural material according to the present specification, i.e., properties that are not represented by the mere sum of properties of each singled out constituent/component of said matrix/material but by the both functional and structural interactions among all constituents/components of the matrix/material that are also the result of the supramolecular self-assembly of said components/constituents within the matrix/material itself.

"Emerging properties" hence refer to technical effects, such as therapeutic or homeostasis - adjuvating properties (i.e., beneficial effect), that the interactions and relationships among the constituents/components of a natural matrix exert on a receiving living system. By definition, emergent properties are properties that are not immediately evident or even predictable based solely on the individual characteristics of each constituent/component of the matrix. Instead, they "emerge" when all the constituents/components of the matrix networks interact with one another and with the living system receiving network in a dynamic and complex way. Emerging properties have been broadly discussed in the art in various scientific and systems-oriented fields, including physics, chemistry, biology, and complex systems theory.

Emerging properties are hence properties that cannot be predicted a priori by the quali-quantitative knowledge of each component of a given composition or matrix and that, consequently, cannot be ascribed to one or more specific API. Hence, although multidrug compositions can show unpredicted synergic effects, the properties of said compositions are still ascribed to the specific APIs and quantities thereof contained therein.

In the case of emerging properties, characteristic of natural matrices, the observed emerging properties cannot be reconducted to specific APIs and are maintained in different batches of a given matrix or a given mixture of matrices notwithstanding the different quali-quantitative composition of said batches (functional resilience see below).

Synthetic according to the present description has the meaning conventionally accepted in chemistry. Conventionally, in chemistry, the term "synthetic" refers to the origin or source of a material or substance. Synthetic substances or materials are produced by man through artificial synthesis i.e., through laboratory chemical reactions usually by reacting simpler chemicals to create more complex ones through processes that often use different pathways, temperature conditions, pressure conditions, energy sources and/or catalysers from those used by living organisms.

Examples: Synthetic substances or materials include plastics, pharmaceutical drugs, and many industrial chemicals. For example, nylon is a synthetic polymer made through chemical synthesis, and aspirin is a synthetic drug produced through specific chemical reactions.

Functional resilience (also indicated as "redundancy") according to the present description is intended as a therapeutic or beneficial (homeostasis adjuvant) resilience of a therapeutic or beneficial product comprising or consisting of one or more natural matrices; the term describes the maintenance of the therapeutic or beneficial properties of different batches of a given product comprising (or consisting of one or more natural matrices) notwithstanding the different batch to batch qualitative and quantitative composition, which is necessarily present (inherent) in products comprising or consisting of one or more natural matrices. As known by the skilled person, each time a different batch of starting raw material is used, the resulting natural matrix has a unique quali-quantitative composition at the molecular level which is typical of the individual diversity between living organisms also of the same species.

A healthy physiological state refers to the condition of an organism's body, organ, apparatus, system or body district, and its internal processes when they are functioning optimally and within normal parameters for that individual, i.e., the state to which homeostasis tends. A healthy physiological state, in the context of one or more biological activities known to contribute to hallmarks of a given disease or pathological condition or of an altered physiological state, refers to the state in which said one or more biological activities are operating optimally and within normal (healthy) parameters. This state is characterized by the absence of significant aberrant cellular or molecular processes associated with the specific disease under consideration. When the modification trend of one or more biological activities which concurs to a pathological, pre-pathological condition is known, the healthy physiological state can be considered represented by the opposite modification trend for each of said activities.

The term considers the hallmarks of a particular disease, which are distinctive features or characteristics that are typically observed in individuals affected by that disease. These hallmarks can include specific cellular behaviours, molecular pathways, canonical pathways, or physiological responses that play a key role in the development or progression of the disease.

In summary, a healthy physiological state in the context of a specific disease or pathological/altered condition is a state in which the one or more biological activities related to the known hallmarks of that disease or pathological condition are modulated in a direction that is consistent with a non-pathological/non-altered state, in other words, opposed to the pathological/altered state.

A healthy physiological state according to the invention, therefore, also indicates the direction of the modulation of one or more biological activities that are known hallmarks of a pathological condition in homeostasis, i.e., before the onset of a pathological condition, in other words the homeostatic direction of the modulation of one or more biological activities ascribed to a specific system, district, apparatus or organ of a healthy subject.

Altered physiological states and altered homeostasis are closely related concepts that describe deviations from the normal functioning and balance of the body's internal environment. While they overlap, there are some distinctions between the two terms:

Altered Physiological States: This term encompasses a broad range of changes in the body's normal functioning, including disruptions in organ systems, biochemical processes, and cellular functions. Altered physiological states can result from various factors such as disease, injury, medication, environmental factors, and psychological stress. Examples include fever, inflammation, hormonal imbalances, and impaired organ function.

Homeostasis (altered): Homeostasis refers to the body's ability to maintain a stable internal environment despite external or pre-pathological changes. This stability is achieved through regulatory mechanisms that control variables such as body temperature, blood pressure, pH balance, and blood glucose levels within narrow ranges. Altered homeostasis occurs when these regulatory mechanisms fail to maintain balance, leading to deviations from the body's normal set points. These deviations can be temporary or chronic and may involve compensatory mechanisms to restore balance. In summary, altered physiological states describe the observable changes in the body's normal functioning, while altered homeostasis refers to the underlying disruption of the body's regulatory mechanisms that maintain internal stability.

An altered homeostasis underlies altered physiological states, as disruptions in homeostatic mechanisms that can lead to physiological imbalances and manifestations of illness or dysfunction. A product adjuvating homeostasis is a product that adjuvates the body to restore the stability of its internal environment when altered.

Hallmark of a disease or of a pathological or medical condition according to the present description has the meaning conventionally used in the art. Hallmarks of a disease are known to be indicators that can mark the progression or control of a given disease or pathological or pre-pathological condition and taken together are usually representative of the general pathological state associated to a given pathology. These hallmarks (also called 'key indicators') are typically a set of features or patterns that a physician would monitor, over time, to track the onset, the progression or regression of a particular illness. In summary, a hallmark of a disease is a defining feature or characteristic whose modification is indicative of a given pre-medical or medical condition, aiding in its identification, diagnosis, monitoring and understanding. By way of example, for neurodegenerative diseases (NDDs) at least the following eight hallmarks of NDD are known in the art: (pathological protein) aggregation, synaptic and neuronal network (dysfunction), (aberrant) proteostasis, cytoskeleton (abnormalities), (altered) energy homeostasis, DNA and RNA (defects), inflammation (increase), and neuronal cell death (increase). In cancer research, the hallmarks of cancer are a set of distinctive characteristics that are commonly found in cancer cells. These hallmarks include (sustained) proliferative signalling, (evasion of) growth suppressors, (resistance to) cell death, (enabling) replicative immortality, (inducing) angiogenesis, and (activating) invasion and metastasis.

Hallmarks of a disease, parameters related to said hallmarks (e.g., biomarkers), one or more biological activities associated to said hallmarks etc. are a framework to study a disease or a pathological or medical condition using an integrated/ holistic approach.

The hallmarks of an altered physiological state typically include observable changes in various aspects of the body's functioning, which may manifest through symptoms, signs, or laboratory findings.

Altered physiological states typically reflect disruptions in the body's homeostatic mechanisms, leading to deviations from normal physiological parameters. These imbalances may involve alterations in temperature regulation, fluid and electrolyte balance, acid-base balance, glucose metabolism, or other regulatory processes. Overall, the hallmarks of an altered physiological state provide valuable clues for healthcare providers to identify the underlying cause, assess severity, and guide appropriate interventions to restore normal functioning and promote recovery.

A reference drug is a drug that is commonly selected or chosen as the standard or preferred treatment for a specific medical condition or illness. It is often established based on factors such as its effectiveness, safety profile, cost, and clinical experience. The reference drug serves as a benchmark for comparison with other drugs, especially when assessing generic versions, new treatments, or alternative therapies. It is typically the first drug of choice recommended by medical guidelines or by healthcare providers for treating a particular condition.

Native natural intelligence, represents the intrinsic ability of natural matrices to conserve and transmit biological and physico-chemical information necessary for interacting and integrating with other living networks, using logics inherent to the living organism that receives them, as they are already known to it, and therefore endogenous in relation to it. This intelligence is an expression of natural autopoiesis, that is, the ability to self-organize and adapt to environmental stimuli without artificial intervention, which would transmit a message according to point-like logics and through mediators unknown to the living organism receiving them, and therefore exogenous in relation to it.

The expression Physiological Interconnection, defined as "endogenous" physiological interconnection, describes the ability of a natural matrix to interact in a harmonious and functional way with the biological systems of the recipient, stimulating internal (endogenous) responses to restore balanced physiological states. This interaction is based on natural dynamics, without artificial interventions, and represents a reciprocal dialogue between the matrix and the organism, promoting self-regulation and physiological recovery.

Self-assembled entities in nature defines complex systems made up of multiple components that spontaneously organize into functional structures through chemical-physical interactions that occur in natural environments and conditions. These systems, found in living organisms or natural matrices, exhibit emergent properties that arise from their dynamic interactions and cannot be replicated artificially.

When referring to a subject in need of a beneficial or therapeutic treatment, the description relates to a human being, either affected by a pathological condition, or in a condition (e.g. age, weigth, sex etc.) of being at risk of developing a pathological condition.

## DETAILED DESCRIPTION OF THE INVENTION

The present invention relates to a method for determining the presence of native natural intelligence in a therapeutic or beneficial product, said product comprising or consisting of natural matrices, through the validation of its therapeutic or beneficial emerging properties, the method comprising the following steps

a. assessing the product or composition naturality by:

1. measuring the 14C activity in said product or composition with ISO-16620-2;2015 (AMS) method,
2. assessing the presence of miRNAs in said product or composition,
3. assessing the presence of exosomes in said product or composition,

b. assessing the presence of therapeutic or beneficial functional resilience between different batches of said product by comparing, batch to batch, the modulation of one or more biological activities underlying the product's desired therapeutic or beneficial effect on a relevant altered physiological state and/or on the pathological condition treated by said product or composition in a cell-based assay whose read-out is representative of the modulation of said one or more biological activities;

c. assessing from the read-out of said cell-based assay, whether the modulation of said biological activities underlying the desired therapeutic or beneficial effect results in the modulation of a whole altered physiological state or pathological condition; and

determining that said product or composition is itself a natural matrix, representing native natural intelligence, wherein native natural intelligence is present when the measured value for the 14C activity is of 99.82 $\pm$ 0,22 % percent,

miRNAs, exosomes and therapeutic or functional resilience are detected, and said modulation in c. results in the modulation of a whole altered physiological state or pathological condition.

The method has been validated on a product, consisting of 100% natural matter, exerting a therapeutic or beneficial effect in the treatment of an altered bone metabolism and/or bone pathological conditions, said product, having a physiological (as opposed to pharmacological) mode of action.

In fact, to act with a physiological mechanism of action, a product must be 100% natural. Natural materials, such as products comprising or consisting of natural matrices, are entities which maintain at least in part the autopoietic properties of their starting materials which belong to the living domain, and display own properties which are represented by networks of material and immaterial relationships, that interact with the network of relationships of the treated subject (networks-to-network interactions) thereby recapitulating an interaction with features and complexities that are physiological-like. Therefore, according to the present description, a product comprising or consisting of one or more natural matrices is a product which is 100% natural, which means that the product does not contain additional artificial substances, i.e., substances of chemical synthesis made by man through laboratory processes.

In addition, according to the present description, a product comprising one or more natural matrix, does also not contain any added isolated molecule, e.g., excipient/s or active principle/s even if of natural origin.

To note, natural materials are fundamentally different from "substances", including substances of natural origin. Since they are not represented by their individual components, they need a dedicated model. Therefore, to describe natural materials, it is necessary to extend the reductionist approach and use the innovations of the last century. Conceptually, this means referring to systems theory. From an experimental point of view, preclinical evidence involves systems biology approaches such as omics sciences (e.g., transcriptomics) and bioinformatics evaluations.

These allow appropriate assessments of the matrix (the acting networks), the human body (the receiving network) and allow to consider the interaction between the two as a "networks over a network" interaction. A mechanism that accompanies, in each specific context, the coordinated redundancy and resilience that characterize physiology corresponds to a 'physiological mechanism of action' and could be characterized by a network paradigm, distinct from the targeted and non-targeted models that describe the PhIM and the mechanical/chemical/physical mechanism, respectively.

In particular, according to the invention, a natural matrix is a 100% natural and biodegradable material, consisting of natural components that have not been denatured by the process for the production of the matrix from the starting raw materials without intentional addition of synthetic products along the whole process.

As already stated, it is mandatory that the matrices are obtained through non-denaturing processes, so that the components of the matrices are not artificially denatured, however, when desired, the presence of additional indicators of maintenance of features that are present in the original raw material can be verified. In addition, a 100% natural product, is a product expected to be 100% biodegradable. In the present description, 100% biodegradable is considered as "readily biodegradable" according to an OECD biodegradability test. These features guarantee the maintenance of the matrix effect which is conferred to the matrix by the presence of structural interactions by its components (material interactions) and functional interactions that become evident upon exposure of a biological system to the natural matrix (immaterial interactions).

The description, in order to validate the method claimed, discloses examples carried out on a product as defined in the table below.

| Natural matrices components | Product composition for a total of 100% w/w |
| --- | --- |
| Equisetum flowering tops | 1.4-2.6% w/w |
| Acacia | 1.5-2.8% w/w |
| Malpighia fruits | 1.4-2.6% w/w |
| Coral skeleton | 22.4-41.6% w/w |
| Avian Egg shell | 21-39% w/w |
| Agave leaves | 8.4-15.6% w/w |
| Cetraria stems | 1.4-2.6% w/w |
| Agaricus (edible) | 3.2-6% w/w |
| Calcium citrate (Coral skeleton plus lemon juice) | 9-17% w/w |

the product having, e.g. the following formula,

| Natural matrices components | Product composition for a total of 100% w/w |
| --- | --- |
| Equisetum flowering tops | 1.7-2.3% w/w |
| Acacia | 1.7-2.4% w/w |
| Malpighia fruits | 1.7-2.3% w/w |
| Coral skeleton | 25-37% w/w |
| Avian Egg shell | 25-34% w/w |
| Agave leaves | 10-14% w/w |
| Cetraria stems | 1.7-2.3% w/w |

(continued)

| Natural matrices components | Product composition for a total of 100% w/w |
|---|---|
| Agaricus (edible) | 4-5.2% w/w |
| Calcium citrate (Coral skeleton plus lemon juice) | 11-15% w/w |

or the formulas below:

Formula A

| Natural matrices components | Product composition for a total of 100% w/w |
|---|---|
| Equisetum flowering tops | 1.80% w/w |
| Acacia | 2.35% w/w |
| Malpighia fruits | 1.60% w/w |
| Coral skeleton | 31.40% w/w |
| Avian Egg shell | 31.00% w/w |
| Agave leaves | 11.70% w/w |
| Cetraria stems | 2.30% w/w |
| Agaricus (edible) | 5.00% w/w |
| Calcium citrate (Coral skeleton plus lemon juice) | 12.85% w/w |

or formula B

| Natural matrices components | Product composition for a total of 100% w/w |
|---|---|
| Equisetum flowering tops | 2.30% w/w |
| Acacia | 1.60% w/w |
| Malpighia fruits | 2.10% w/w |
| Coral skeleton | 35.10% w/w |
| Avian Egg shell | 29.10% w/w |
| Agave leaves | 10.60% w/w |
| Cetraria stems | 1.70% w/w |
| Agaricus (edible) | 4.90% w/w |
| Calcium citrate (Coral skeleton plus lemon juice) | 12.60% w/w |

or formula C

| Natural matrices components | Product composition for a total of 100% w/w |
|---|---|
| Equisetum flowering tops | 2.00% w/w |
| Acacia | 2.15% w/w |
| Malpighia fruits | 2.00% w/w |
| Coral skeleton | 32.00% w/w |
| Avian Egg shell | 30.20% w/w |
| Agave leaves | 12% w/w |

(continued)

| Natural matrices components | Product composition for a total of 100% w/w |
| --- | --- |
| Cetraria stems | 2.00% w/w |
| Agaricus (edible) | 4.65% w/w |
| Calcium citrate (Coral skeleton plus lemon juice) | 13.00% w/w |

For the experiments disclosed herein Equisetum was *Equisetum Arvense,* said Acacia was *Acacia senegal,* said Malpighia was *Malpighia punctifolia,* said Coral was caribbean coral, said Avian was *Gallus gallus* and said Cetaria was *Cetaria islandica.*

Calcium citrate in the product of the present description, is calcium citrate obtained by the natural reaction occurring when coral skeleton is put in contact with lemon juice. In particular, the calcium citrate is made by diluting lemon juice 1:1 (volume) with water, adding coral powder and mixing for 5 hours and then freeze-drying.

The product tested was prepared in dry form, and consists of 6 powders and 3 lyophilized extracts in the w/w % indicated in the tables above.

The powders are Calcium carbonate from coral, calcium carbonate from eggshell, fine powder from *Agave* leaves, fine powder from *Cetaria* thallus, fine powder from edible mushroom, *Agaricus,* and gum Arabic, *Acacia,* mixed together at room temperature until the mixture is homogenous.

The freeze-dried extracts are from *Equisetum* flowering tops, acerola, *Malpighia* fruitsand calcium citrate (as defined above),

For the preparation, the homogeneous mixture of powders is then mixed together at room temperature with the freeze dried extracts until the mixture is homogenous. When the product is prepared in the form of tablets, the bulk mixture is pressed by direct compression to obtain the finished product tablet.

The powders are: Calcium carbonate from coral, *Caribbean coral,* calcium carbonate from eggshell, fine powder from *Agave sisalana* leaves, fine powder from *Cetaria islandica* thallus, fine powder from champignon mushroom, *Agaricus bisporus,* and gum Arabic, *Acacia senegal,* and the freeze-dried extracts are: freeze dy extract of *Equisetum arvense* flowering tops, acerola, *Malpighia punctifolia* and calcium citrate.

The solvent used in the preparation of the extracts of the invention is water, preferably purified water by means of an Industrial Water Treatment Plants produced from drinking water.

Water extracts of plant materials are known to the skilled person. A non limiting example of extract preparation that can be applied mutatis mutandis also to acerola, si the following;

Freeze-dried extract of *Equisetum arvense* flowering tops: dried *Equisetum arvense* flowering tops, were subjected to extraction by water 100% (*v/v*) [drugs solvent ratio: 1/18] for 2h at 70°C and filtered to remove solid exhausted material. The resulting clarified extract was concentrated under vacuum, until reaching the concentration factor of 10:1 (v:v, initial extract volume compared to the volume after the evaporation step), and then freeze-dried for 72 h. The resulting extract was stored until use at room temperature, away from light and moisture.

The product or the composition as herein disclosed and/or claimed is for beneficial or medical use, e.g., for use in the treatment or in adjuvating the treatment of a bone fragility condition in a subject in need thereof.

In fact, as stated in the summary of the invention, the Applicant's *in vitro* studies allowed to compare the efficacy profile of the mixture of the present invention with the treatment with synthetic calcium and vitamin D alone, evaluating its ability to induce differentiation (increase in alkaline phosphatase, ALP, activity) of mesenchymal stem cells (hADMSCs), isolated from adipose tissue of patients, into mature osteoblasts capable of mineralising the cellular matrix (increase in the deposition of HA crystals) and counteracting bone fragility.

The treatment with Vitamin D, together with the osteoinducing stimulus provide by an osteoinductive medium (OM) showed a statistically greater increase in ALP activity compared to its control (OM + DMSO + Ca) indicating a possible synergistic effect with OM and confirming its ability to induce the differentiation of stem cells towards osteoblasts (Figure 5).

Despite the increase in activity, Vitamin D is not able to induce the correct stimulus for functional mineralization (Figure 6).

Even treatment with synthetic calcium, in osteoinductive medium, showed an increase in ALP activity (Figure 5), with the typical "bell-shaped" trend characterized by a peak in enzymatic activity levels at 28 days of treatment, which led, unlike the treatment with Vitamin D, to the correct formation of extracellular matrix and substantial hydroxyapatite (HA) deposits (Figure 6).

Analogously to the treatments with Vitamin D and synthetic calcium, treatment with the mixture of the invention together with the osteoinductive stimulus was also able to promote the increase in ALP activity (Figure 5), showing the characteristic bell-shaped trend with a peak in activity at 21 days of treatment, thus indicating an early stimulation of the differentiation process compared to the OM medium added with synthetic calcium alone (Synthetic calcium).

The product, in addition to showing the ability to mediate the commitment of the mesenchymal cell towards the bone lineage, is able to induce the mineralization process early, in a complete manner, favoring the deposition of hydroxyapatite (HA) crystals (Figure 6). In fact, after 28 days of treatment, the mixture of the invention induces a statistically significant increase in hydroxyapatite crystals compared to synthetic calcium. Not only does the mixture herein described act as a support for the differentiation stimulus towards the osteoblast, in the presence of an osteoinducing stimulus, and as a calcium donor capable of mineralizing the bone extracellular matrix, but, in the GM medium without osteoinducing agents, it leads to a statistically greater increase in ALP activity compared to synthetic Calcium, suggesting that it is capable of inducing the differentiation of mesenchymal cells into mature osteoblasts by itself (Figure 7). On the contrary, treatments with Calcium and Vitamin D do not determine increases in ALP activity, confirming their inability to induce cellular differentiation in the absence of an osteoinducing stimulus.

In line with what is already known, treatments with Vitamin D and synthetic Calcium, although fundamental, interpret and satisfy in a non-exhaustive way the real needs of the organism, inserting themselves into the metabolic pathways of the individual in a punctual fashion without achieving a satisfactory effect. In fact, they are not able to recapitulate the physiological differentiation of a mesenchymal stem cell into an osteoblast, in order to support the formation of bone.

Vitamin D certainly contributes, but alone cannot provide a differentiating stimulus and does not directly provide material in the form of calcium to promote the formation of mineralized bone matrix (Figure 8).

Synthetic calcium alone is not able to intervene in the differentiation process and is only able to provide material for the formation of bone (Figure 8).

The mixture of the invention alone is able to recapitulate the entire physiologically active process (Figure 8).

The molecular mechanisms underlying the activity of the mixture of herein described in inducing a correct metabolic stimulus declined at the phenotypic level in the increase of ALP and HA, were analyzed by evaluating the modulation of the gene expression of mesenchymal stem cells following treatment with the mixture of the invention together with the differentiating stimulus. The product induces in vitro significant effects on stem cells that are precursors of both bone cells and adipose cells, promoting in the bone a clear differentiation of mesenchymal stem cells into mature osteoblasts capable of mineralizing the extracellular matrix. Consequently, with respect to the adipose tissue, the mixture of the invention promotes a reduction of fat both locally in the bone and at the systemic level, decreasing the tendency of mesenchymal stem cells to differentiate into mature adipocytes. The synergy of these actions results in a decrease in bone fragility and an increase in bone quality, due to the induction of beneficial effects at both ends of the fat/bone axis.

As can be observed in the heatmap (Figure 10), treatment with the mixture herein described determines modulations of the expression profiles that underlie the decrease in bone remodeling, bone resorption and osteopenia, in addition to an increased differentiation of osteoblasts and an increase in mineralization in terms of increased bone mineral density.

In particular, the product described promotes the differentiation of osteoblasts through the modulation of specific proteins linked to osteogenesis such as the induction of RUNX2 and the inhibition of sclerostin (SOST) known to have a function on the inhibition of osteoblast activity, consistently with what was also observed through the analysis of the enzymatic activity of ALP.

The product also induces the increase of bone synthesis markers such as osteocalcin (OCN) and BMPs (bone morphogenetic proteins) underlying the increase in osteoblastic activity and the formation of new bone matrix, consistently with what was also observed through the measurement of the concentration of HA crystals.

The reduction in the bone resorption process induced by the mixture of the invention plays a fundamental role in the maintenance and repair of the bone itself, promoting the rebalancing of bone density and structure. In particular, the mixture of the invention promotes a reduction in the expression levels of Sclerostin (SOST), known to have a function on the inhibition of osteoblast activity, thus enhancing bone formation.

In addition, the mixture of described has demonstrated a systemic action of metabolic regulation, inducing the expression levels of Osteocalcin (OCN) (Figure 10) a hormone also responsible for modulating the insulin metabolic asset. Osteocalcin is expressed and secreted by mature osteoblasts and acts by stimulating insulin secretion from pancreatic $\beta$-cells and promoting insulin sensitivity in muscle and white adipose tissue, resulting in a reduction in blood glucose levels.

The results also show the potential of the mixture of described to reduce both the quantity and the inflammatory process of adipose tissue, suggesting an anti-adipogenic effect and an improvement in tolerance, thus identifying further desirable effects in the physiopathological framework of interest.

The local and systemic action of the mixture described is therefore able to enter the dysfunctional loop that is established in conditions of systemic inflammation and metabolic dysregulation, interfering with the differentiation of mesenchymal stem cells towards white adipocytes and their consequent accumulation.

In other words, the mixture described, thanks to its emerging properties, using a network mechanism, is able to interact with the physiological metabolic pathways that are established in the body of a woman in menopause or who is facing this transition. The product is able not only to provide the calcium necessary for the correct deposition of hydroxyapatite crystals within the bone matrix, but also has the ability to correctly stimulate the differentiation of mesenchymal stem cells, promoting the development of the osteoblastic cell line and inhibiting the formation of osteoclasts and adipocytes, thus helping to establish a virtuous balance in bone metabolism processes. Furthermore, the improvement of the secretory profile of osteocalcin suggests the induction of beneficial effects also at the systemic level of organs such as the pancreas, muscle tissue and adipose cells.

This dual synergistic and systemic effect of the product makes it markedly desirable compared to the classic calcium and vitamin D supplements that are commonly used, as they are only able to partially support the physiological change of the organism. This support is instead supported by the mixture of the invention with a physiological mechanism, that is, able to interact with the body according to the canons already known to the latter and not according to alien canons, imposed by an exogenous entity such as a synthetic molecule. This is because the mixture of the invention, in addition to containing both precursors of Vitamin D and sources of calcium, above all interacts with the pool of mesenchymal stem cells in the bone and adipose tissue, allowing to recapitulate all the elements necessary to rebalance the correct turnover of the bone, useful for the formation of a solid and functional structure. Furthermore, the effects of the product reach a systemic projection helping to rebalance metabolic disorders and the latent inflammation that on a systemic scale afflict women in perimenopause. The mixture herein described, thanks to the presence of complex plant matrices and natural calcium from various sources, clearly demonstrates how the complexity of a physiological process can be correctly supported according to canons already known to it only if placed in relation to a 100% natural therapeutic solution of an equally highly complex nature.

Hence, the product or composition as herein defined is suitable, for use in the treatment or in adjuvating the treatment of a bone fragility condition, wherein said subject is at risk of developing a bone fragility condition.

According to the description, the subject is a human being with a bone fragility condition or a human being at risk of developing a bone fragility condition.

As bone metabolism is also strictly interconnected with fat metabolism, as explained above, in a particular embodiment of the invention, said bone fragility is associated with a fat increase in said subject.

Said fat increase is intended as a significant fat increase, i.e. a fat increase that goes beyond the normal weigth fluctuations occurring averagely in said subject.

A typical trigger of fat increase and bone fragility, by way of example, can be associated to obesity, metabolic syndrome, premenopause, perimenopause or menopause, andropause.

Furthermore, said bone fragility condition can be selected among osteoporosis, osteopenia.

In the extensive characterisation and study work on the product described herein, the Applicant also identified conventional hallmarks of osteoporosis and related biological activities whose modulation (up or down modulation) underlies the pathological condition, and defined as modulation trend of said activities toward the healthy physiological state as opposed to the modulation observed in the altered or pathological condition (see figure 9).

The applicant also verified that the desired modulation of all of said activities was achieved with the product described herein, thereby validating the therapeutic effectiveness of the product (Fig. 10). In addition, the Applicant compared the modulation of said activities exerted by the product with the modulation exerted by a reference drug used for treating osteoporosis such as Dibase (Vitamin D 10'000 U.I./ml) and discovered that the reference drug was not capable of: reducing the remodelling of the bone, reducing osteopoenia, inducing the differentiation of osteoblasts, restoring mineralization, reducing inflammation and is partially capable of reducing adipose tissue. Hence, overall, the administration of vitamin D does not modify the biological activities underlying the osteoporosis pathological condition with the modulation trend tending to the healthy phyisiological state (fig. 11).

Finally, the inventors also found, surprisingly, that different batches of the product tested (see examples, product C batches 1, 2 and 3), notwithstanding their quali-quantitative different chemical composition, showed therapeutic/beneficial functional resilience by modulating the biological activities as described above, with the same pattern and with similar modulation values.

In fact, although different batches of a product comprising one or more natural matrices are, by definition, batches wherein the qualitative and quantitative composition is necessarily variable as thoroughly discussed above, according to one embodiment, a qualitative and/or quantitative analysis of each batch was performed to demonstrate the existing qualitative and/or quantitative difference in the molecular composition of each batch (Fig. 13). This can be achieved through conventional techniques; a non-limiting example comprises chromatography. spectrophotometry, atomic absorption spectroscopy (AAS), atomic emission spectroscopy (AES), inductively coupled plasma (ICP) techniques, chromatography coupled with detectors, and the like or combinations thereof. The analysis can be focused on a limited number of selected classes of substances (e.g., figure 13) or on all the components of the product.

As expected for products comprising or consisting of natural matrices, although each batch was prepared following standardised procedures to obtain a priori a high degree of homogeneity between different batches, the detailed quali-quantitative analysis of all the tested batches clearly demonstrated relevant batch-to-batch quali-quantitative differences that would have lead, with conventional validation methods used for synthetic or isolated drugs, to the discard of batches that were indeed therapeutically valid, as well as the impossibility to hypothesise the presence of an API.

It is herein reminded that, due to their own nature, natural matrices are variable in their composition even when obtained from the same kind of raw source, by way of example, the skilled person knows very well that a natural matrix obtained from an individual of a plant species, will never be absolutely identical to another natural matrix obtained from a different individual of the same plant species, even among plants in the same field, due to the genetic and epigenetic variability of each living organism.

Following the experiment reported in figure 13, notwithstanding the different qualitative and quantitative chemical composition of all the batches analysed, the authors surprisingly found that, in all the batches tested, the different molecular entities within each matrix appeared to interact in a redundant manner with each other both functionally and possibly structurally providing the same therapeutic or beneficial (homeostasis-adjuvant) effect despite their differences in quali-quantitative molecular composition.

Indeed, the batches showed a functional (therapeutical or beneficial) resilience despite the variability in their qualitative and quantitative molecular composition.

In other words, the authors surprisingly found that different batches of the same product showed a consistent regulation (in terms of trend and magnitude) of all the examined biological activities, relevant to the desired therapeutical or beneficial effect, notwithstanding batch-to-batch qualitative and quantitative composition differences, herein also defined as "functional resilience effect". The observed maintenance of the biological activity is likely due to the fact that, as said above, the emerging properties of a natural matrix are due to the matrix networks acting as a whole entity with distinctive properties, and may not be ascribable to each single molecule as if it were in isolation, the therapeutic action being through a non-pharmacological mechanism of action different from the classical therapeutic products based on the pharmacological relationship between structure and activity (SAR) which is the most relevant relationship in classical pharmacological activities between an active pharmaceutical ingredient (API) and the receptor targeted by said API, which is considered at the level of single molecules.

This agrees with the likelihood that the products analysed by the inventors, may exert their therapeutic or beneficial action by acting on a whole pathophysiological or altered physiological state.

The product or composition as herein defined, exerts its therapeutic or beneficial effect through a physiological mechanism of action, by adjuvating the reinstatement of bone homeostasis through a network of biological activities on the altered physiological state underlying said bone fragility condition and by showing therapeutic or beneficial functional resilience among different batches of said product or composition, said functional resilience being intended as the maintenance of the therapeutic or beneficial properties of different batches of said product or composition, notwithstanding their different batch to batch qualitative and quantitative composition.

In particular, due to all the reasoning on natural matrices provided above, together with all the experimental data gathered by the Applicant, it can be said that the product or composition according herein described is itself a natural matrix, representing native natural intelligence, said natural intelligence being the only capable to allow a physiological endogenous interconnection with other entities which are self-assembled in nature, such as the human species.

It is herein reminded that native natural intelligence, represents the intrinsic ability of natural matrices to conserve and transmit biological and physico-chemical information necessary for interacting and integrating with other living networks, using logics inherent to the living organism that receives them, as they are already known to it, and therefore endogenous in relation to it. This intelligence is an expression of natural autopoiesis, that is, the ability to self-organize and adapt to environmental stimuli without artificial intervention, which would transmit a message according to point-like logics and through mediators unknown to the living organism receiving them, and therefore exogenous in relation to it.

According to the invention, the presence of native natural intelligence in a thereapeutic or beneficial product or coposition, wherein said product or composition comprises or consists of natural matrices, can be determined by validating that the product or composition is itself a natural matrix with emerging properties (therapeutic or beneficial) when its 14C activity measured with ISO-16620-2;2015 (AMS) method is of 99.82 $\pm$ 0,22 % percent, miRNAs and exosomes are detected in said product or composition, the product or composition shows batch-to-batch therapeutic or beneficial functional resilience between different batches of said product or composition and when the product or composition modulates a whole altered physiological state or pathological condition.

This can be done, with the product or composition of the invention, by performing the following steps on samples of said product or composition:

    a. assessing the product or composition naturality by:

        1. measuring the 14C activity with ISO-16620-2;2015 (AMS) method,
        2. assessing the presence of miRNAs in said product or composition,
        3. assessing the presence of exosomes in said product or composition,

    b. assessing the presence of therapeutic or beneficial functional resilience between different batches of said product or composition by comparing, batch to batch, the modulation of one or more biological activities underlying the product's desired therapeutic or beneficial effect on an altered bone metabolism and/or bone pathology in a cell-based assay whose read-out is representative of the modulation of said one or more biological activities;

c. assessing from the read-out of said cell-based assay, whether the modulation of said biological activities underlying the desired therapeutic or beneficial effect results in the modulation of a whole altered physiological state or pathological condition; and determining that said product or composition is itself a natural matrix, representing native natural intelligence when

the measured value for the 14C activity is of 99.82 $\pm$ 0,22 % percent,

miRNAs, exosomes and therapeutic or functional resilience are detected, and

said modulation in c. results in the modulation of a whole altered physiological state or pathological condition.

The product is determined to be natural when the measured value for the 14C activity is of 99.82 $\pm$ 0,22 % percent, miRNAs, exosomes are detected and to exert its activity through a physiological mechanism of action when it modfies a state and show therapeutic or beneficial functional resilience i.e. shows emerging properties. The sum of these features allows the determination of the product as a natural matrix itself, thereby representing native natural intelligence.

Assessment of the modification of a state and of functional resilience can be performed as follows.

The capability of a therapeutic or beneficial product of modifying a pathophysiological state or an altered physiological state (e.g., by adjuvating the homeostasis response of the organism) is a crucial feature for establishing its physiological mode of action. Indeed, a product acting with networks- to -network interaction is a product that is expected to modify a state rather than a single function when administered to a living organism.

In other words, this feature is likely to be satisfied by a therapeutic or beneficial product comprising one or more natural matrices or consisting of one or more natural matrices given the networks-network (product-receiver) interactions exerted by natural matrices. The Applicant's patent application PCT/IB2024/055892 discloses a method for defining the mode of action of a therapeutic or beneficial natural matrices-based product.

A product exerting a physiological mode of action is also expected to be 100% natural (see above) and to show the flexibility and self-administrating mechanisms that are observable in living organisms wherein different messages within the cell and among cells as well as different regulations of gene pathways can provide the same result notwithstanding the different messages triggered within the cell. For a therapeutic or beneficial product this equates to the functional resilience of different batches of the product (with a quali-quantitative variability in their chemical composition).

The present inventors determined whether product herein described exerts its therapeutic or beneficial effect by modifying a pathological state or an altered physiological (not yet pathological) state and whether the product shows or not functional resilience (i.e., the maintenance of the therapeutic or beneficial properties of different batches of a given product comprising one or more natural matrices, notwithstanding its different batch to batch qualitative and quantitative composition).

According to the invention, this can be verified by carrying out a cell-based assay whose readout is representative of the modulation of the selected biological activities as defined above as disclosed e.g. in the examples as described in the specification, and by analysing and interpreting the data obtained therefrom.

Assessment of functional resilience (therapeutic or beneficial).

As indicated in the glossary and in the specification above, in the present description and claims, functional resilience is the maintenance of a therapeutic or beneficial measurable efficiency in different batches of a product notwithstanding variability in their qualitative and quantitative molecular composition.

It is clear that the batches (such as the tested batches 1, 2, and 3 of Product C) are intended as batches that are identical in terms of manufacturing processes and type and amounts of each ingredient (as the main ingredients of the selected products are natural matrices, this means that each matrix in the product is produced from the same kind of starting materials and with the same procedures e.g. a given kind of extract from the same plant part of the same plant species), therefore, the variability in their qualitative and quantitative molecular composition cannot be ascribed to different manufacturing procedures or to different ingredients but can only originate from the inherent difference between natural matrices obtained with the same procedure from different organism/s of the same species. When different batches of the same product comprising one or more natural matrices maintain in a measurable and verifiable way their final modulatory activities underlying their therapeutic or beneficial properties irrespectively of their quali-quantitative composition, functional resilience of the product can be validated.

The invention hence also relates to a method for determining the presence of native natural intelligence in a therapeutic or beneficial product or composition, said product or composition comprising or consisting of natural matrices, through the validation of its therapeutic or beneficial emerging properties, the method comprising the following steps on samples of said product or composition:

a. verifying said product naturality by performing:

1. measuring of the 14C activity with ISO-16620-2;2015 (AMS) method
2. assessing the presence of miRNAs in said product or composition,
3. assessing the presence of exosomes in said product or composition,

b. assessing the presence of therapeutic or beneficial functional resilience betweeng different batches of said product by comparing, batch to batch, the modulation of one or more biological activities underlying the product's desired therapeutic or beneficial effect on a relevant altered physiological state and/or on the pathological condition treated by said product or composition in a cell-based assay whose read-out is representative of the modulation of said one or more biological activities;

c. assessing from the read-out of said cell-based assay, whether the modulation of said biological activities underlying the desired therapeutic or beneficial effect results in the modulation of a whole altered physiological state or pathological condition; and

determining that said product or composition is itself a natural matrix representing native natural intelligence, when the measured value for the 14C activity is of 99.82 $\pm$ 0,22 % percent, miRNAs, exosomes and therapeutic or functional resilience are detected, and said modulation in c. results in the modulation of a whole altered physiological state or pathological condition.

According to an embodiment of the invention the method further comprises, before performing the one or more cell-based assay: (1) providing a list of hallmarks representative of said altered metabolism and/or pathological state; (2) identifying for each of said hallmarks one or more biological activities modifications underlying said pathological state thereby pinpointing a network of biological activities whose modulation concurs to said pathological state and (3) identifying one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state.

According to a preferred embodiment said altered physiological state is bone metabolism and/or said pathological condition is osteoporosis and said hallmarks are selected from: remodelling of bone, osteopenia, differentiation of osteoblasts, mineralization of bones, reduction of inflammation, reduction of adipose tissues, preferably, wherein the biological activities of (2) for the remodelling of bone hallmark are selected from the biological activities depicted in figure 9.

More in detail

(1) providing a list of hallmarks representative of the pathological state associated to a pathology or to a pathology that can stem from said non-pathological altered physiological state, i.e., providing a list of hallmarks representative of the disease or pathophysiological condition treated by the product of interest or that may derive from the non-pathologically altered physiological state in which homeostasis is adjuvated by the beneficial product of interest;

(2). identifying for each of said hallmarks one or more biological activities modifications underlying said pathology and determining the modulation thereof representing the pathophysiological state associated to said pathology and assessing the opposite modulation as the modulation pattern of each of said activities representing a healthy physiological state; and

(3). identifying one or more marker and the modulation pattern thereof underlying the modification detectable in said pathological state for each of said one or more biological activities and setting for each of said parameters the modulation pattern opposite to the one identified, as the modulation pattern concurring to said healthy physiological state.

In case a therapeutic product is examined, according to an embodiment, the method of the invention may comprise the following steps:

(a) performing said at least one *in vitro* cell-based assay on the following groups of cells

(a1) at least one control group and at least two test groups of cells having the diseased phenotype relevant to the intended use of the therapeutic product; or

(a2) at least one group of cells with a healthy physiological phenotype; and at least one control group and at least two test groups of said cells with a healthy physiological phenotype wherein the diseased phenotype relevant to the intended use of the therapeutic product is induced,

and treating each of said test groups of cells with one of said different batches of therapeutic product;

(b) determining the modulation or modulation pattern of each of said parameters on each of said groups of cells of step (a) and calculating the respective modulation values for each of said one or more biological activities;

(c) comparing said modulation values, wherein:
said therapeutic product is shown to exert its therapeutic effect through a physiological mechanism when

at least 50% of said one or more biological activities for each hallmark are modulated by each product batch with the modulation trend of the network concurring to the healthy state, and the modulation values determined in (b) of each of said at least 50% one or more biological activities for said test groups of cells of (a1) differ, respectively, from the ones of said control group of cells of (a1) of at least 0.15, or
at least 50% of said one or more biological activities for each hallmark are modulated by each product batch with the modulation trend of the network concurring to the healthy state, and the modulation values determined in (b) of each of said at least 50% one or more biological activities for said test groups of cells of (a2) differ, respectively, from the ones of said control group of cells of (a2) by at least 15%, and
functional resilience of the product is demonstrated by the modulation values for each one or more biological activities of each said test groups of cells differing by less than 20% from the average of said values.

This means, that the modulation value of a given biological activity of the test group is confronted, respectively, with the modulation value of the same biological activity of the control group, therefore the difference of at least 0.15 or of at least 15% is the difference between the modulation value of a given activity in the treated group of cells with respect to the modulation value of the same activity in the group of cells representing the control baseline.

In other words, the method may also be described as a method of assessing whether a therapeutic product exerts its effect to treat a pathological state through a physiological mechanism of action comprising:

- providing different batches of a therapeutic product, the product comprising one or more natural matrix
- providing a list of hallmarks representative of said pathological state, identifying for each of said hallmarks a set of parameters whose modulation allows an evaluation of a network of biological activities underlying the therapeutic effect of the product tested and determining the modulation trend in terms of up or down modulation of said activities in said network in the diseased and healthy state;

(a) performing at least one *in vitro* cell-based assay on the following groups of cells

(1) at least one control group and at least two test groups of cells having the diseased phenotype targeted by the therapeutic product; or
(2) at least one group of cells with a healthy physiological phenotype; and at least one control group and at least two test groups of said cells with a healthy physiological phenotype wherein the diseased phenotype targeted by the therapeutic product is induced,
and treating each of said test groups of cells with one of said different batches of therapeutic product;

(b) determining the modulation or modulation pattern of each of said parameters on each of said groups of cells and calculating the respective modulation values for each of said biological activities;
(c) comparing said modulation values for each biological activity in each cell group of step (b), wherein:

the therapeutic product is shown to exert its therapeutic activity through a physiological mechanism of action when
at least 50% of said biological activities for each hallmark are modulated by each product batch with the modulation trend of the healthy state determined in (b) and the modulation values determined in (b) of each of said at least 50% one or more biological activities for said test groups of cells of (a) (1) differ, respectively, from the ones of said control group of (a) (1) of at least 0.15 or
at least 50% of said biological activities are modulated by each product batch with the modulation trend of the healthy state determined in (b) and the modulation values determined in (b) of each of said at least 50% one or more biological activities for said test groups of cells of (a) (2) differ, respectively, from the ones of said control group of (a) (2) by at least 15%,
and functional resilience of the product is demonstrated by the modulation values for each biological activity of each said test groups of cells differing by less than 20% from the average of said values.

The expression at least 50% of said one or more biological activities for each hallmark are modulated by each product batch with the modulation trend of the network concurring to the healthy state, means that, in case of a single biological activity for a given hallmark, in order to fulfill the requirement above, 100%, i.e., the single activity, must be modulated by the product tested, with the modulation trend concurring to the healthy state, The expression "the modulation values of each said at least 50% one or more biological activities determined in (b)" refers to the modulation values determined in (b) of that at least 50% of biological activities fulfilling the requirement of being modulated according to the modulation trend concurring to the healthy state. This applies, mutatis mutandis, to all the embodiments disclosed herein.

In case a beneficial product (i.e., a product exerting a beneficial effect on an altered but not yet pathological physiological state by adjuvating homeostasis) is examined, according to an embodiment, the method of the invention may comprise the following steps:

(a) performing said at least one *in vitro* cell-based assay the following groups of cells:

at least one control group of cells having healthy phenotype or at least one control group of cells wherein the aberrantly modulated phenotype targeted by said beneficial product is suitably induced, and at least two test groups of cells taken from said control group
and treating, each of said test groups of cells, with one of said different batches of beneficial product;

(b) determining the modulation or modulation pattern of each of said parameters on each of said groups of cells of step (a) and calculating the respective modulation values for each of said one or more biological activities;
(c) comparing said modulation values, wherein:

said beneficial product is shown to exert its homeostasis adjuvating effect through a physiological mechanism of action when
at least 50% of said one or more biological activities for each hallmark are modulated by each product batch with the modulation trend of the network concurring to the healthy state and the modulation values calculated in (b) of each of said at least 50% one or more biological activities for said test groups of cells differ, respectively, from the ones of said control group of cells of at least 0.15 and
functional resilience of the product is demonstrated by the modulation values for each one or more biological activities of each said test groups of cells differing by less than 20% from the average of said values.

This means, that the modulation value of a given biological activity of the test group is confronted, respectively, with the modulation value of the same biological activity of the control group, therefore the difference of at least 0.15 or of at least 15% is the difference between the modulation value of a given activity in the treated group of cells with respect to the modulation value of the same activity in the group of cells representing the control baseline.

In other words, the method can be defined as a method of assessing whether a beneficial product exerts its homeostasis adjuvating effect by regulating an altered physiological state comprising:

- providing different batches of a beneficial product adjuvating homeostasis, the product comprising one or more natural matrix
- providing a list of hallmarks representative of a pathological state that can stem from said altered physiological state, identifying for each of said hallmarks a set of parameters whose modulation allows an evaluation of a network of biological activities underlying the therapeutic effect of the product tested and determining the modulation trend in terms of up or down modulation of said activities in said network in the diseased and healthy state;

(a) performing at least one *in vitro* cell-based assay on the following groups of cells:

(1) at least one control group of cells having a healthy phenotype or at least one control group of cells wherein the aberrantly modulated phenotype targeted by said beneficial product is suitably induced, and at least two test groups of cells taken from said control group
and treating each of said test groups of cells with one of said different batches of beneficial product;

(b) determining the modulation or modulation pattern of each of said parameters on each of said groups of cells of step (a) and calculating the respective modulation values for each of said biological activities;
(c) comparing said modulation values for each biological function in each cell group of step (a), wherein:

the beneficial product is shown to exert its homeostasis adjuvating effect through a physiological mechanism when
at least 50% of said biological activities for each hallmark are modulated by each product batch with the modulation trend of the healthy state determined in (b) and the modulation values determined in (b) of each of said at least 50% one or more biological activities for said test groups of cells of (a)(1) differ, respectively, from the ones of said control group of (a)(1) of at least 0.15,
and functional resilience of the product is demonstrated by the modulation values for each biological activity of each said test groups of cells differing by less than 20% from the average of said values.

In a preferred embodiment, the control group, is considered as the reference modulation baseline and the quali-quantitative modulation value for each of said one or more biological activities is considered to be 0.

As previously indicated, the method of the invention comprises:(1) providing a list of hallmarks representative of the pathological state of interest (i.e., the pathological state treated by the product analysed or the pathological state that can stem from the altered physiological state on which the beneficial product analysed exerts its homeostasis adjuvating activity); (2) identifying for each of said hallmarks one or more biological activities modifications underlying said pathological state thereby pinpointing a network of biological activities whose modulation concurs to said pathological state and (3) identifying one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state.

All the embodiments above allow to determine whether a therapeutic or beneficial product exerts its therapeutic or beneficial effect by modifying a state or merely a limited number of activities or even a single function underlying the pathology an aberrant physiological state treated by said product and whether a therapeutic or beneficial product as the ones selected maintains a functional resilience as herein defined.

The modification of a state is a feature that cannot be obtained with a one-API pharmaceutical product, therefore, this feature rules out classical pharmaceutical modes of action. However, the modification of a state could, in principle, be obtained with a pharmaceutical product comprising a cocktail of APIs.

A physiological mode of action requires that the therapeutic or beneficial product regulates the state in a manner that involves an overall cellular response with a networks over network interaction, and not in a points over network interaction that is at the basis of APIs mode of action (be it a single API or a cocktail thereof).

This implies, besides the regulation of a state, also the capability of the product of acting with a functional resilience (either therapeutic or beneficial), i.e., providing the same batch to batch therapeutic/beneficial effect notwithstanding the different batch to batch quali-quantitative composition which, in other words, is the result of regulating the various parameters selected in variable ways and nevertheless providing a conserved functional result.

The present invention hence also provides modes for demonstrating the functional resilience of therapeutic or beneficial products.

As already explained in the glossary and in the description above, functional resilience (therapeutic or beneficial) is the capability of a given product of modulating one or more biological activities underlying a pathological state or an altered physiological state notwithstanding variability in the qualitative and quantitative composition of different batches of the same product and therefore notwithstanding the possibility of reaching the same final result triggering different signals within the cell i.e., showing bioequivalence intended as same final result. As already stated above is known that pharmaceutical (API based) products, with a different qualitative and quantitative composition are not considered bioequivalent.

The physiological mode of action, which implies an overall interaction with the cells of the subject treated and not with singled out cellular molecular targets, is the mode exerted by living organisms as the result of networks-network interactions. Physiological systems in the body often exhibit functional redundancy to maintain homeostasis and adapt to changes or disruptions, redundancy being a well-known physiological mechanism in the living for ensuring that a given goal is reached (e.g., a response of the organism in the production of various proteins, in the activation of various pathways etc.). When a therapeutic product interacts with these systems, it may engage multiple pathways or mechanisms, including redundant ones, to achieve its desired effect. This redundancy, which results in a functional resilience, contributes to the physiological mode of action of the product.

Functional resilience in therapeutic/beneficial products (intended as the ability of a therapeutic or beneficial product to maintain its intended functionality and effectiveness despite variability in its batch-to-batch qualitative and quantitative composition) is therefore an essential feature of a physiological mode of action.

The invention also relates, mutatis mutandis, to method of treatment or of adjuvating the treatment of a bone fragility condition wherein the product or the composition according the invention is administered, alone or in combination with an active principle in a beneficial of therapeutically effective amount to a subject in need thereof.

The following examples are intended to better illustrate the invention and to support it scientifically and are nowhere intended as limiting it's scope.

## EXAMPLES

### 1. COMPOSITIONOF THE TESTED FORMULATION

The experimental data reported below were produced with the following formulation of the product hereinafter also named "Product C"

| Coral calcium powder | 32% w/w |
| Egg shell calcium powder | 30.2% w/w |
| Coral calcium citrate powder | 13% w/w |
| Agaricus bisporus powder | 4.65% w/w |
| Equisetum arvense flowering tops dry extract | 2% w/w |
| Malpighia punicifolia | 2% w/w |
| Cetraria islandica powder | 2% w/w |
| Agave sisalana leaves powder | 12% w/w |
| Acacia senegal powder | 2.15% w/w |

Other formulations of the product of the invention within the ranges claimed were also tested and provided similar results (data not shown).

Three batches of product C were prepared, with the same formula, but starting from different lots of raw plant or natural material, namely, batch 1, batch 2 and batch 3.

In addition, permutations of the formula above, within the ranges claimed in claim 1, were also prepared and preliminary data confirmed their correct biological activities (i.e. biological activities comparable to product C) in cell based assays as reported below. Powders of Calcium carbonate from coral, *Caribbean coral,* calcium carbonate from eggshell, fine powder from *Agave sisalana* leaves, fine powder from *Cetaria islandica* thallus, fine powder from champignon mushroom, *Agaricus bisporus,* and gum Arabic, *Acacia senegal,* were mixed at room temperature until the mixture was homogeneous then the freeze-dried extracts of freeze dy extract of *Equisetum arvense* flowering tops, acerola, *Malpighia punctifolia* and calcium citrate were mixed and added at room temperature, in the weight to weight percentages disclosed above.

The solvent used in the preparation of the extracts of the invention was purified water by means of an Industrial Water Treatment Plants produced from drinking water.

Freeze-dried extract were prepared by subjecting each plant material to extraction by water 100% (*v/v*) for 2h at 70°C and filtered to remove solid exhausted material. The resulting clarified extracts were concentrated under vacuum, until reaching the concentration factor of 10:1 (v:v, initial extract volume compared to the volume after the evaporation step), and then freeze-dried for 72 h. The resulting extracst were stored until use at room temperature, away from light and moisture.

.The product thus prepared was then suitably dissolved and/or diluted where appropiate for the various assays disclosed below.

2. IN VITRO ASSAYS

An in vitro study was conducted on human pre-osteoblastic mesenchymal stem cells derived from adipose tissue (hADMSCs). These cells represent a convenient and easily obtainable (non-invasive) source of mesenchymal stem cells capable of differentiating, if appropriately induced, into the osteoblastic lineage. In vitro studies have shown that hADMSCs are capable of differentiating into mature, active osteoblasts and producing mineralized bone matrix when appropriately induced by culture in specific osteogenic induction medium (osteogenic/osteoinductive medium; OM) containing 10 nM dexamethasone, 0.2 mM ascorbic acid, and 10 mM $\beta$-glycerophosphate, along with a source of calcium and phosphate. Therefore, these cells represent an optimal model for studying osteoblast differentiation and functionality, the mineralization process, and for evaluating the effects of various substances on these two processes.

To assess the effects of Product C, synthetic Calcium, and Vitamin D on bone metabolism, in vitro experiments were conducted by treating hADMSCs at different experimental times (4, 7, 14, 21, 28, and 35 days of treatment).

The experiments focused on the evaluation of:

The ability to provide bioavailable calcium ions for the mineralization of the extracellular bone matrix (Measurement of hydroxyapatite (HA) crystals),

The potential to induce and/or enhance osteoblastic differentiation (Spectrophotometric assay of alkaline phosphatase (ALP) activity),

The transcriptional profile induced in the cells to assess pathways and biological functions potentially influenced by the treatment (Gene expression using microarray platform

The differentiation potential of the samples was evaluated under two different osteoinductive stimulation conditions - non-osteoinductive medium (GM) and osteoinductive medium (OM).

Non-osteoinductive medium GM:

(DMEM medium containing antibiotics/calcein/serum + beta-glycerophosphate and 2-phosphoascorbate, but lacking dexamethasone): This medium does not contain dexamethasone (an external osteoinductive agent) and, as such, allows the evaluation of whether the added formulation can independently induce osteoblastic differentiation, through measurement of ALP activity.

Osteoinductive medium OM:

(DMEM medium containing antibiotics/calcein/serum + beta-glycerophosphate and 2-phosphoascorbate and dexamethasone): This medium contains dexamethasone (an external osteoinductive agent) and allows the evaluation of whether the formulation may have a synergistic (or conversely inhibitory) effect on osteoblastic differentiation induced by dexamethasone.

The experimental model involved the addition of calcium to the medium at a concentration reflecting the physiological amount of calcium in the body, which is 1.4 mM. To achieve this condition, in the treatments involving Vitamin D (solubilized in DMSO as it is hydrophobic) and Calcium, appropriate quantities of synthetic calcium were added to both culture media (OM and GM).

hADMSCs cell lines were cultured into a 100 mm Petri dish at 37 _C in humified atmosphere with 5% $CO_2$ in growth medium (GM) [Ham's F12 Coon's modification medium supplemented with 10% FBS, 100 IU/mL penicillin, and 100 _g/mL streptomycin]. The medium was replaced with fresh GM every 3 days and once confluence was reached, the cells were detached by trypsinization and were plated on 24-well plates at a cell density of 1 x_ 104 cells/cm2 in GM until reaching the 70-80% confluence. Afterward, the medium was switched to osteogenic medium (OM) or maintained in GM both supplemented with treatments and incubated from 7 to 35 days. Treatment media was refreshed twice a week.

These cells were obtained with informed consent during general surgery from three different patients (PA42, PA59 and PA69) (Romagnoli et al, "In Vitro Behaviour of Human Adipose Tissue-Derived Stem Cells on Poly($\varepsilon$-caprolactone) Film for Bone Tissue Engineering Applications", BioMed Research International, vol. 2015, Article ID 323571, 12 pages, 2015. https://doi.org/10.1155/2015/323571). These cell lines have been characterized with respect to the main stemness markers of mesenchymal stem cells (CD44, CD105, and STRO1) and by studying their multipotency toward osteogenic phenotypes at the Department of Surgery and Translational medicine of the University of Florence.

The time schedule used for experimental setting is as follows:

2.1 ALP ASSAY AND CALCIUM MINERALISED DEPOSIT ASSAY (figures 5-7) At the end of every incubation time point, the cells were washed with DPBS (two times), fixed in 4% PFA/DPBS for 15 min, washed with ultrapure water (three times), dried, and preserved at 4∘C until the assay. Each experimental point was performed in quadruplicate.

ALP Assay

Each well was incubated with 500 $\mu$L of 4-methylumbelliferyl phosphate in 280mM Tris-HCl Buffer pH 9.0 for 15 min at 37∘C. The reaction was stopped by the addition of 2mL 0.1M NaOH. ALP activity was measured with a spectrofluorometer LS55 (PerkinElmer) at 365nm $\lambda$ excitation and 445nm $\lambda$ emission and expressed in $\mu$U/cm2 using a standard

curve of 4-methylumbelliferone 50 nM-10 $\mu$M in 280mMTris-HCl Buffer pH 9.0.

*Calcium Mineralized Deposits Assay.*

Each well was incubated with 2mL of 50mM NaEDTA for 30min at 37∘C. The solution was then transferred into the cuvette and the fluorescence measured with a spectrofluorometer LS55 (PerkinElmer) at 494nm $\lambda$ excitation and 517nm $\lambda$ emission and expressed in $\mu$g/cm2 using a standard curve of calcium mineralized deposits 25 ng/mL-500 $\mu$g/mL solubilized in 50mM NaEDTA.

The *in vitro* studies have allowed the efficacy profile of Product C to be compared with treatment with synthetic calcium alone and vitamin D alone, by evaluating its ability to induce differentiation (increased ALP activity) of mesenchymal stem cells (hADMSCs) isolated from patients' adipose tissue into mature osteoblasts, capable of mineralising the cellular matrix (increased deposition of HA crystals) and counteracting bone fragility. Treatment with vitamin D in combination with osteoinductive stimulus (OM) showed a statistically greater increase in ALP activity than its control (OM+DMSO+Ca), indicating a possible synergistic effect with OM and confirming its ability to induce stem cell differentiation towards osteoblasts (Fig. 5).

Treatment with synthetic calcium also showed, in osteoinductive medium, an increase in ALP activity, with the typical 'bell-shaped' pattern characterised by a peak in enzyme activity levels at 28 days of treatment (Fig. 5).

Similar to vitamin D and synthetic calcium treatments, Product C treatment in combination with osteoinductive stimulation was also able to promote an increase in ALP activity, showing the characteristic bell-shaped pattern with peak activity at 21 days of treatment, thus indicating an early stimulation of the differentiation process compared to OM supplemented with synthetic calcium alone (Fig. 5).

The bell-shaped curve observed (Fig. 5) in all groups reflects the dynamic nature of ALP activity during osteogenic differentiation, with a rise corresponding to early matrix maturation and a decline as cells transition to later stages of bone formation. For Product C, the earlier peak in ALP activity highlights its ability to anticipate key phases of the physiological process of differentiation and mineralization. This early response suggests a role in promoting the initial stages of osteogenesis, aligning with the physiological progression of bone formation.

Treatment with synthetic calcium in an osteoinductive medium showed, in contrast to vitamin D treatment, the correct formation of extracellular matrix and substantial deposition of hydroxyapatite (HA) (Fig. 6). Product C, in addition to its ability to mediate the commitment of mesenchymal stem cells to the bone lineage, is able to induce the mineralisation process early and completely, promoting the deposition of hydroxyapatite (HA) crystals. In fact, after 28 days of treatment, Product C induces a statistically significant increase in hydroxyapatite crystals compared to synthetic calcium (Fig. 5). This result indicates that the early peak in ALP activity observed for Product C (Fig. 5) translates into effective mineral deposition at the 28-day time point (Fig. 6). In contrast, OM + Vitamin D + Synthetic Calcium, despite its contribution to early ALP activity, does not translate into appreciable mineralization at day 28 (Fig. 6). The HA content remains markedly low compared to the other groups, highlighting that early differentiation alone is insufficient without effective downstream mineral deposition. This analysis underscores that early activation of osteogenic markers, as seen with Vitamin D, does not guarantee successful mineralization unless it is supported by mechanisms that drive the full differentiation process.

Both OM + DMSO + Synthetic Calcium and OM + Synthetic Calcium serve as strong positive controls, showing robust mineralization levels at 28 days. Product C achieves comparable results, further demonstrating its effectiveness in supporting the physiological process of bone differentiation and mineralization. In contrast, Product C effectively couples its early osteogenic activity with sustained mineralization capacity, suggesting a more comprehensive role in mimicking the physiological bone formation process.

The results obtained show that not only does Product C act as a support for the differentiation stimulus towards osteoblasts in the presence of an osteoinductive stimulus, and as a calcium donor capable of mineralising the extracellular matrix of bone, but in GM medium without osteoinductive agents it leads to a statistically greater increase in ALP activity than synthetic calcium, suggesting that it alone is capable of inducing the differentiation of mesenchymal cells into mature osteoblasts (Fig. 7). In contrast, calcium and vitamin D treatments do not increase ALP activity, confirming their inability to induce cell differentiation in the absence of an osteoinductive stimulus.

In particular, the results (Figure 7) show that in the GM medium, lacking osteoinductive agents, Product C leads to a statistically significant increase in ALP activity compared to synthetic calcium, suggesting that Product C alone can induce the differentiation of mesenchymal cells into mature osteoblasts. In contrast, treatments with Calcium and Vitamin D do not induce increases in ALP activity, confirming their inability to induce cellular differentiation in the absence of an osteoinductive stimulus.

In the osteoinductive medium, Product C also promotes an increase in ALP, showing a typical bell-shaped curve with a peak of activity at 21 days, indicating an early stimulation of the differentiation process compared to the OM medium with only synthetic calcium (Synthetic Calcium).

Similarly, (figure 6) treatment with Vitamin D shows the same ALP trend, but it induces a statistically greater increase compared to its control (OM+DMSO+Ca). Despite the increase in activity, the substance is unable to induce the correct stimulus for functional mineralization.

This stimulus was effectively provided by Product C, which showed a statistically significant increase in hydroxyapatite crystals at 28 days compared to synthetic calcium (figure 6).

3. GENE EXPRESSION ANALYSIS

At the end of described treatment periods, cells were washed with 100 μl PBS and lysed and collected in RLT buffer (Qiagen, 1053393) added with β-mercaptoethanol (Sigma, M3148) and DX reagent (Qiagen, 19088) for gene expression analysis experiments. Total RNA was extracted from cells lysates using an QIAsymphony RNA Kit (Qiagen,) with the QIAsymphony SP instrument (Qiagen).

The quality and quantity of RNA was determined by A230, A260, A280 and A320 measurements on Varioskan™ LUX multimode microplate reader (Thermo Scientific™). Integrity of RNA was checked using a 2100 expert_Eukaryote Total RNA Nano Kit (Agilent). Whole transcriptome expression profile was evaluated using a Human Clariom™ S Pico Assay HT (Applied Biosystems, ThermoFisher Scientific) on a GeneTitan MC Instrument (Applied Biosystems, Thermo-Fisher Scientific), following the manufacturer's instructions. Briefly, 6 ng of total RNA was used to generate cDNA, then fragmented and labelled cDNA was hybridized to a Human Clariom S 96-array plate for 17 h at 45°C. Arrays were washed, stained and then scanned using the GeneTitan MC Instrument (Applied Biosystems, ThermoFisher Scientific) and CEL Intensity files were generated by Affymetrix GeneChip Command Console Software (AGCC, ThermoFisher Scientific).

3.1 Transcriptomics data analysis

Data analysis was performed using Transcriptomic Analysis Console Software (TAC, ThermoFisher Scientific) that provides quality control analysis, performs normalization and summarization, based on the Signal Space Transformation-Robust Multi-Chip Analysis (SST-RMA) analysis algorithm, and provides a list of differentially expressed genes (Limma Bioconductor package, $p\text{-value} \leq 0.05$).

3.2. Bioinformatic modelling of experimentally observed transcriptomics data Ingenuity Pathways Analysis (IPA) (QIAGEN \Inc., https://www.qiagenbioinformatics.com/products/ingenuitypathway-analysis) was used, for each investigated batch to evaluate modulations of gene expression relevant for effects of interest.

IPA is an aggregator of scientific references that allows to search for information on genes/proteins and the construction of networks that predict the behaviour of biological systems according to their gene expression status.

4. EVALUATION OF TRANSCRIPTIONAL EFFECTS OF PRODUCT C TREATMENT ON OSTEOBLASTIC DIFFERENTIATION AND MINERALIZATION OF ADIPOSE TISSUE

As evidenced by ALP and HA dose evaluations, gene expression analysis also indicates that Product C appears to have significant effects on both bone and adipose tissue, promoting clear differentiation of mesenchymal stem cells into mature osteoblasts capable of mineralizing the extracellular matrix, while inducing a reduction in bone adipose tissue, leading to decreased bone fragility and improved bone quality.

As shown in the heatmap (Figures 10-11), Product C (batch 1) treatment leads to modulation of expression profiles underlying decreased bone remodeling, bone resorption, and osteopenia, in addition to increased osteoblastic differentiation and enhanced mineralization in terms of increased bone mineral density.

In particular, Product C promotes osteoblastic differentiation through the modulation of specific osteogenesis-related proteins, such as the induction of RUNX2 and inhibition of sclerostin (SOST), known for inhibiting osteoblast activity, consistent with the ALP enzyme activity analysis.

The product also induces an increase in bone synthesis markers such as osteocalcin (OCN) and BMPs (bone morphogenetic proteins), highlighting increased osteoblastic activity and the formation of new bone matrix, consistent with the measurement of HA crystal concentration.

A reduction in bone remodeling processes is also observed, which is important for the maintenance and repair of bone, potentially rebalancing bone density and structure. In particular, Product C seems to reduce sclerostin (SOST), known for inhibiting osteoblast activity, thereby enhancing bone formation.

Finally, Product C appears to reduce both the quantity and inflammatory processes in bone and adipose tissue and at systemic level, suggesting an anti-adipogenic effect, confirmed by improved glucose tolerance.

Hence, Product C demonstrates multiple effects on both bone and adipose tissue, contributing to bone formation through increased osteoblastic differentiation and mineralization, as well as reducing bone remodeling, adiposity, and metabolic regulation. All effects highlighted by gene expression data clearly distinguish and significantly demonstrate Product C advantageous effects compared to traditional products consisting solely of calcium and Vitamin D.

The assay was repeated on batches 2 and 3 of Product C and the resulting data are provided in Fig. 13 a and b.

5. DEFINITION OF THE PATHOPHYSIOLOGICAL HALLMARKS OF THE DISEASE WITH WHICH TO INTERROGATE IPA

The alteration of healthy physiological state features of state-of-the-art "Osteoporosis" were considered with particular attention to the following areas involved:

- Remodelling of bone
- Osteopenia
- Differentiation of osteoblasts
- Mineralization
- Reduction of inflammation
- Reduction of adipose tissue

This knowledge was used to interrogate IPA via the "IPA Bioprofiler" tool, using the following keywords: osteoporosis, postmenopausal osteoporosis, calcification of bone, osteoblast and osteoclast differentiation, bone mineral density.

The use of "IPA Bioprofiler" allowed to identify clusters of expressed genes causally linked to each of the identified one or more biological activities and the specific molecular pathway underpinning them. Information concerning the measured gene expression data (Fold change value cut-off $\leq -2$ and $\geq +2$ and p-value$\leq 0.05$) induced by each batch was then superimposed on the networks obtained, to define influenced genes and modulation of the connected biofunction.

Modulation of expressed genes were shown in different intensities of blue (signifying down-modulation) or red (signifying up-modulation). The resulting expected calculated impact, based on the literature, on the related biofunctions was determined by "IPA Molecule Activity Predictor" tool (MAP) and resumed in a heatmap visualization.

The colour and intensity thereof were transformed into numerical values. Figure 9 show the modulation trend of the selected biological activities underlying the identified hallmarks of osteoporosis in an altered physiological state (altered metabolism) of the bone or in the osteoporosis pathological condition and the modulation trend of said biological activities (opposite to the altered/pathological one) desired for the restoration of a healthy physiological state.

The results of the tests performed yielded a comparative study of the performance and of the mechanism of action of three different batches of Product C. The analysis revealed that while all batches could return reproducible biological effects, it was also possible to identify batch-specific fluctuations in the induced transcriptional pattern. Evidently the induction of slightly different transcriptional patterns still results in the same desirable regulation of one or more biological activities. This is due to a functional resilience caused by redundancy in the interactions between the components of the product and the body, whereby, by virtue of the multifocal mechanism of action, different batches differing in their quali-quantitative composition elicit the same effect (Fig. 13 a and b).

The analysed batches are therefore considered as having equivalent biological outputs since the induction and repression patterns are conserved.

The different transcriptional patterns and relative biological effects of different batches is to be intended as the hallmark of the inherent variability present in a preparation constituted of biological material. From the results summarised in figure 13 it is evident that the observed transcriptional patterns of the different batches elicit a very reproducible biological (functional resilience) effect leading to a general modification of the pathological processes and of the overall pathological state equivalence for all the batches reported in Figure 13.

6. DETAILED ANALYSIS OF PRODUCT C

A detailed analysis of Product C was performed, unless otherwise indicated the batch product used was batch 1.

The table below summarizes all the methods used.

| Class of Compounds | Characteristics | Type of Method | Short description |
|---|---|---|---|
| **Polysaccharides** | Polysaccharides $\geq$ 20.000 Dalton | HPLC-RID | Extraction of water-soluble polysaccharides and analysis by HPLC equipped with molecular exclusion column and refractive index detector.**(A)** |
| **Phenols** | Phenols polar | UHPLC-qToF | Sample extraction and reverse phase chromatography analysis by UHPLC coupled to a quadrupole-time-of-flight mass spectrometer. **(B)** |

(continued)

| Class of Compounds | Characteristics | Type of Method | Short description |
|---|---|---|---|
| **Tannins** | Tannins | UHPLC-qToF | Sample extraction and reverse phase chromatography analysis by UHPLC coupled to a quadrupole-time-of-flight mass spectrometer. **(B)** |
| **Terpenes** | Terpenes | HPLC-DAD | Analysis carried out by NEOTRON |
| **Organic acids** | Organic acids mono-, di-, tri-carboxylic | HPLC-UV | Sample extraction and analysis by HPLC coupled to UV detector. **(C)** |
| **Sugars and Derivatives** | Monosaccharides | IC-PAD | Sample extraction and analysis by ion chromatograph coupled to pulsed amperometric detector. **(D)** |
| | Disaccharides | IC-PAD | Sample extraction and analysis by ion chromatograph coupled to pulsed amperometric detector. **(D)** |
| **Lipids** | Fatty acids | HPLC-UV | Sample extraction and analysis by HPLC coupled to UV detector. **(E)** |
| Inorganic Compounds | Elements | ICP-MS, ICP-OES<br><br>IC-CD | Acid mineralization of the sample in a microwave oven and analysis of the conductively induced plasma by means of a single quadrupole mass spectrometer, or optical emission spectrometer. **(F1)**<br>Sample extraction and analysis by ion chromatograph coupled to conductometric detector. **(F2)** |
| | Anions | IC-CD | Sample extraction and analysis by ion chromatograph coupled to conductometric detector. **(F2)** |
| **miRNAs** | miRNA | Sequencing | After sample extraction protocol the samples were analysed by RNA-Seq technique **(Genomix4Life )** |

The results are summarised in the table below:

| METHOD | COMPOUNDS | Product C_Batch 1 (also 23D2227) (%) |
|---|---|---|
| A | **MACROMOLECULES > 20000 (POLYSACCHARIDES, Buffer Method), Total** | 2,6828 |
| | **PHENOLS, Total** | 0,0337903 |
| | **FLAVONOIDS, Total** | 0,0319882 |
| | **ANTHOCYANIDINS, Total** | 0,0072838 |
| B | Delphinidin | 0,0072838 |
| | **FLAVANONES, Total** | < LoQ |
| B | Hesperetin | ND |
| B | Hesperidin | <LoQ |
| B | Naringenin & Pinobanksin (AS: Naringenin) | <LoQ |
| B | Naringenin-7-O-glucoside | <LoQ |
| B | **FLAVONES, Total** | 0,0099425 |
| B | Genkwanin | <LoQ |
| B | Luteolin-4'-O-glucoside & Kaempferol-3-O-glucoside & Quercitrin (AS: Luteolin-4'-O-glucoside) | 0,0022669 |

(continued)

| METHOD | COMPOUNDS | Product C_Batch 1 (also 23D2227) (%) |
|---|---|---|
| B | Luteolin-7-O-Rutinoside | <LoQ |
| B | Nobiletin | <LoQ |
| B | Orientin & Homoorientin (AS: Homoorientin) | <LoQ |
| B | Scutellarein tetramethyl ether | <LoQ |
| B | Sinensetin | <LoQ |
| B | Tangeretin | <LoQ |
| B | Vicenin-2 | 0,0076756 |
| B | **FLAVONOLS, Total** | 0,0147619 |
| B | Quercetin | ND |
| B | Quercetin-3-O-glucopyranoside (Isoquercetin) | 0,012504 |
| B | Quercetin-3-O-rutinoside-7-O-glucoside | <LoQ |
| B | Rutin | 0,0022579 |
| | **PHENOLIC ACIDS, Total** | < LoQ |
| B | alpha-Resorcylic acid & Protocatechuic acid (AS: Protocatechuic acid) | <LoQ |
| | **PHENYLPROPANOIDS, Total** | 0,0018021 |
| | **HYDROXYCINNAMIC ACIDS, Total** | 0,0018021 |
| B | Caffeic acid | <LoQ |
| B | Cryptochlorogenic acid & Chlorogenic acid (AS: Chlorogenic acid) | 0,0018021 |
| B | Ferulic acid | <LoQ |
| B | Isoacteoside | <LoQ |
| B | Neochlorogenic acid | <LoQ |
| B | Verbascoside | <LoQ |
| | **MONOLIGNOLS, Total** | < LoQ |
| B | Eleutheroside B | <LoQ |
| | **TANNINS, Total** | < LoQ |
| | **TANNIN MONOMERS, Total** | < LoQ |
| | **CONDENSED TANNINS MONOMERS, Total** | < LoQ |
| B | Catechin | <LoQ |
| | **TERPENES, Total** | 0,0222 |
| | **TRITERPENES, Total** | 0,0222 |
| | **PHYTOSTEROLS, Total** | 0,0222 |
| NEOTRON: HPLC-DAD | Ergosterol | 0,0222 |
| | **ORGANIC ACIDS, Total** | 8,39616 |
| | **MONOCARBOXYLIC ACIDS, Total** | < LoQ |
| C | Acetic acid | <LoQ |
| C | Lactic acid | <LoQ |
| | **DICARBOXYLIC ACIDS, Total** | 2,22076 |

(continued)

| METHOD | COMPOUNDS | Product C_Batch 1 (also 23D2227) (%) |
|---|---|---|
| C | Fumaric acid | 0,02916 |
| C | Malic acid | 2,1916 |
| C | Succinic acid | <LoQ |
| C | Tartaric acid | <LoQ |
| | TRICARBOXYLIC ACIDS, Total | 6,1754 |
| C | Citric acid | 6,1754 |
| | SUGARS AND DERIVATIVES, Total | 5,09549892 |
| | MONOSACCHARIDES, Total | 4,53164501 |
| D | Arabinose | <LoQ |
| D | Fructose | 2,21704215 |
| D | Galactose | <LoQ |
| D | Glucose | 2,31460286 |
| D | Mannose | <LoQ |
| D | Rhamnose | <LoQ |
| | DISACCHARIDES, Total | 0,56385391 |
| D | Lactose | <LoQ |
| D | Maltose | <LoQ |
| D | Sucrose | 0,56385391 |
| | VITAMINS, Total | 0,19148 |
| | WATER SOLUBLE VITAMINS, Total | 0,19148 |
| C | Ascorbic acid (Vitamin C) | 0,19148 |
| | LIPIDS, Total | < LoQ |
| | FATTY ACIDS, Total | < LoQ |
| E | Propionic acid | <LoQ |
| | INORGANIC COMPOUNDS, Total | 28,9817119 |
| | ANIONS, Total | 0,33454945 |
| F2 | Nitrate | <LoQ |
| F2 | Nitrite | <LoQ |
| F2 | Phosphate | 0,10151375 |
| F2 | Sulfate | 0,2330357 |
| | MACROELEMENTS, Total | 28,5455538 |
| F1 | Calcium | 27,1786219 |
| F2 | Chloride | 0,09940538 |
| F1 | Magnesium | 0,29963747 |
| F1 | Phosphorus | 0,16727613 |
| F1 | Potassium | 0,741467 |
| F1 | Sodium | 0,05914598 |
| | MICROELEMENTS, Total | 0,01321692 |
| F1 | Chromium | 0,00029394 |

(continued)

| METHOD | COMPOUNDS | Product C_Batch 1 (also 23D2227) (%) |
|---|---|---|
| F1 | Cobalt | < LoQ |
| F1 | Copper | 0,00034911 |
| F2 | Iodide | <LoQ |
| F1 | Iron | 0,01004836 |
| F1 | Manganese | 0,00153219 |
| F1 | Molybdenum | < LoQ |
| F1 | Nickel | 0,00026527 |
| F1 | Selenium | < LoQ |
| F1 | Tin | < LoQ |
| F1 | Vanadium | 0,00010867 |
| F1 | Zinc | 0,00061937 |
| | **OTHER ELEMENTS, Total** | 0,0883917 |
| F1 | Aluminum | 0,01264075 |
| F1 | Antimony | < LoQ |
| F1 | Arsenic | < LoQ |
| F1 | Barium | 0,0031083 |
| F1 | Boron | 0,00073173 |
| F2 | Bromide | <LoQ |
| F1 | Cadmium | < LoQ |
| F1 | Gadolinium | < LoQ |
| F1 | Gallium | < LoQ |
| F1 | Gold | < LoQ |
| F1 | Iridium | < LoQ |
| F1 | Lead | < LoQ |
| F1 | Lithium | < LoQ |
| F1 | Lutetium | < LoQ |
| F1 | Mercury | < LoQ |
| F1 | Palladium | < LoQ |
| F1 | Platinum | < LoQ |
| F1 | Rubidium | 0,00098574 |
| F1 | Ruthenium | < LoQ |
| F1 | Strontium | 0,02223713 |
| F1 | Tellurium | < LoQ |
| F1 | Thallium | < LoQ |
| F1 | Thorium | < LoQ |
| F1 | Titanium | 0,04868804 |
| F1 | Uranium | < LoQ |

(continued)

| METHOD | COMPOUNDS | Product C_Batch 1 (also 23D2227) (%) |
|---|---|---|
| F1 | Ytterbium | < LoQ |
| *Note 1. Gray boxes indicate chemical macro classes.* *Note 2. %= compound concentration expressed as percentage of the whole matrix.* *Note 3. The term "Total" refers to the sum of the value of the various compounds which forms the corresponding group.* *Note 5. <LdQ= below the limit of quantification* | | |

In addition, qualitative miRNA characterization was also performed on ultracentrifuged samples from each batch and the results show high metabolomic complexity together with the presence of miRNAs typical of living matter.

| miRNA Family | miRNA |
|---|---|
| miR166 | ath-miR166a-3p |
| | ath-miR166b-3p |
| | ath-miR166c |
| | ath-miR166d |
| | ath-miR166e-3p |
| | ath-miR166f |
| | ath-miR166g |
| miR8175 | pseudomiR8175 1 |
| | pseudomiR8175 3 |
| | pseudomiR8175 4 |
| | pseudomiR8175 5 |
| | pseudomiR8175 6 |
| | pseudomiR8175 7 |
| | pseudomiR8175 8 |

## 7. IDENTIFICATION OF EXTRACELLULAR VESICLES AND miRNA CONTENT IN PRODUCT C

Flow Cytometry analysis of Product C identified the presence of particles compatible with extracellular vesicles. This suggests that Product C contains vesicles that could potentially play a role in cellular communication. Building on this, small RNA sequencing using the sRNAtoolbox framework was employed to explore the RNA content within these vesicle-sized particles. Among the identified sequences, two mature microRNAs (miRNAs) or their isoforms were found: miR8175 and miR166, which are prominent miRNAs from the well-annotated Arabidopsis thaliana genome.

### 7.1 miRNAs detection and role of the detected miRNAs in bone metabolism

The presence of RNA has been evaluated both quantitatively and qualitatively. RNA was extracted using a plant matrix specific kit (Rneasy PowerPlant kit) after homogenization with the use of QIAshredder columns before proceeding with the kit extraction protocol. The dimensional distribution of the RNA obtained was performed using Bioanalyzer 2100 with RNA 6000 Nano, RNA 6000 Pico and small RNA kits.

A size distribution of the total RNA between 4 and 150 nt was detected.

Role of ath-miR8175 in Mediating the Differentiation and Mineralization Capacities of Product C

The product has been characterized for the presence of miRNA, through NGS analysis of total RNA isolated from various biological matrices within the product.

A current and evolving topic in scientific research is the relevance of miRNAs present in foods and their role in human nutrition. Numerous studies emphasize the importance of these miRNAs in modulating metabolic processes in response to diet.

RNA analysis of the product C allowed for the identification of two mature miRNA sequences (miRNA), annotated in the Arabidopsis thaliana genome (the most annotated plant to date): miR8175 and miR166.

### 7.2 Detection of supramolecular structures in the Product C system natural matrix Dynamic Light Scattering

Dynamic Light Scattering (DLS) is a common technique for particle size analysis in the nanometer range. It measures the hydrodynamic size of particles by analysing light scattering from a laser passing through a solution. The intensity of scattered light fluctuates over time, reflecting the Brownian motion of particles, with smaller particles diffusing faster. DLS is used to measure particle size in colloidal samples, assess formulation stability, and detect aggregation. It is also ideal for analysing the size distribution of isolated exosomes and microvesicles.

The analysis was carried out by Alfatest Lab.

Sample preparation:

Product C Batch 1 (23D2227) was dispersed at arbitrary concentration of 1 mg/ml in 0.22 $\mu$m filtered demineralized water.

After dispersion sample was vortexed for 2 minutes to provide complete dispersion.

Analysis parameters:

Measurement cell: Plastic, (DTS0012)
Detector: Back scattering 173° (NIBS)
Laser wavelength: 633 nm
Measurement number: 3
Correlation time: adaptive
Measurement position: automatic
Attenuator: automatic
Temperature: 25 °C
Temperature equilibration time: 120 seconds
Dispersant: Water
Dispersant refractive index: 1,33
Dispersant viscosity: 0,8872 cP at 25 °C (water)

Sample was analysed at three different conditions:

- Unfiltered
- After filtration with 0.45 $\mu$m Nylon syringe filter
- After filtration with 0.1 $\mu$m Nylon syringe filter

0.1 $\mu$m filtration was performed on 0.45 $\mu$m filtered sample.

Before each filtration step, sample dispersions were vortexed for 30 seconds. Filtered dispersions were left at rest at room temperature for about 15 minutes and then analysed after a gentle manual stirring.

Results:

Averaged results of Z-Average and Pdl obtained from 3 repeated measurements are reported in the table below. Z-Average is the intensity weighted mean diameter, Pdl is the polydispersity index.

| Condition | Z-Average (nm) | Pdl | Peak1 | | Peak2 | | Peak3 | |
|---|---|---|---|---|---|---|---|---|
| | | | nm | % | nm | % | nm | % |
| Not Filtered | 825,8 | 0,597 | 626,3 | 92,6 | 72,3 | 7,4 | - | - |
| 0,45$\mu$m - filtered | 81,6 | 0,484 | 152,6 | 78,8 | 26,6 | 26,6 | 4912,0 | 2,3 |

(continued)

| Condition | Z-Average (nm) | Pdl | Peak1 | | Peak2 | | Peak3 | |
|---|---|---|---|---|---|---|---|---|
| | | | nm | % | nm | % | nm | % |
| 0,1μm - filtered | 46,3 | 0,468 | 156,8 | 53,4 | 31,7 | 31,7 | - | - |

The results show that the aqueous dispersion contains large particles. In particular, filtration at 0.45 μm shows a polymodal distribution with a particle size signal larger than the filtration size (4912,0 nm). This also occurs with filtration at 0.10 μm, where a bimodal system is observed with a signal at 156.8 nm, exceeding the filter size. The data thus suggest the presence of supramolecular aggregates consisting of noncovalent intermolecular interactions. The data quality of the filtered samples is good.

Detection of exosomes in Product C Batch 1

Preparation of ultracentrifuged samples

The starting sample (Product C Batch 1) from which the ultracentrifugate is prepared was weighed and resuspended in a volume of VIB or vesicles isolation buffer (20 mM MES; 2 mM CaCl2; 100 mM NaCl, pH 6.0) maintaining the ratio of 5mL of buffer per 500 mg of sample. The sample was incubated under stirring at room temperature for 20-24 h to promote solubilisation. After incubation, several centrifugations were performed at 4°C and at increasing speeds to isolate particles between 30-500 nm in size. The T-1250 rotor (Thermo Fisher Scientific, 11718-5) and the Thermo Scientific™ Sorvall™ WX+ ultracentrifuge (Thermo Fisher Scientific™ 75000080, N°: 15342177) were used for the ultracentrifugation. The sample was centrifuged at 700 x g for 20 minutes; the pellet was discarded while the supernatant was filtered through a 0.45 μm filter and centrifuged at 10000 x g for 30 minutes. The supernatant was then moved to ultracentrifugation tubes and centrifuged at 40000 x g for 70 minutes. The supernatant was moved on a new ultracentrifuge tube and centrifuged again for 70 minutes at 100000 x g. Supernatant was discarded and the 100K pellet was resuspended in VIB buffer and centrifuged at the same conditions. The pellet was resuspended in 600 μL of 25 mM Trehalose (Merck, T0167) in PBS and stored at 4° C to be used within 24 h, or at -30° C for long-term storage.

Extracellular vesicles staining and flow cytometry analysis.

Extracellular vesicles from samples were stained with CellMask™ Green Plasma Membrane Stain (Thermo-Fisher Scientific #C37608) according to the manufacturer's instructions and quantified on a Attune NxT flow cytometer. Briefly, 27 μL of exosomes were added to 3 μL of CellMask™ Green Plasma Membrane Stain (10x) per sample for 30 minutes at 37°. Then, added 170 μL of PBS (double filtered at 0.22 μm) to each sample and read them. To remove any non-specific events in the flow cytometry analysis, PBS stained with CellMask™ Green Plasma Membrane Stain was used as a negative control and Fluorescent Exosome Standards (Novus Biologicals #NBP3-11691) was used as a positive control. Moreover, each sample was analyzed without staining to exclude any autofluorescence.

RESULTS

The sample Product C batch 1 was analyzed using Flow Cytometry as follows: the values of PBS marked by CellMask™ Green Plasma Membrane Stain were excluded from the area of interest because they were considered as blank samples. In the Fluorescent Exosome Standards, a large number of elements were identified in the same area being considered positive.

The samples then analyzed considering the number of elements present in the same area. The number of extracellular vesicles identified in the product C sample was $2.7 \times 10^6$

10. SPECTROSCOPY FTIR CHARACTERIZATION

Fourier Transform Infrared Spectroscopy (FTIR) is an analytical technique used to analyse the absorption or emission spectrum of a sample by examining the interaction between infrared radiation and matter. FTIR spectra can be influenced by weak interactions in plant matrices, such as hydrogen bonds, van der Waals forces, and hydrophobic interactions, which alter peak position, intensity, and shape. These interactions affect the absorption characteristics of functional groups. FTIR spectra are unique to each material, making it an effective method for studying the physico-chemical properties of plant matrices. FTIR is thus valuable for characterizing plant systems. FTIR instruments and set up.

Alpha spectrometer from BRUKER Optics. Instrument is equipped with a GLOBAR source emitting in the Far and Mid-Infrared regions, a ROCKSOLID interferometer (Michelson type), a KBr beamsplitter, and an RT-DLATGS detector.

- Resolution: 2 cm-1
- Spectral range: 5000-300 cm-1
- Background scans: 50
- Scans for sample acquisition: 50

Preparation of the sample

The Product C Batch 1 sample was transferred into the appropriate sample holder for ATR-FTIR analysis of solids and liquids. Approximately 10 mg of the sample were deposited and pressed onto the diamond crystal of the ATR support. Before recording the measurement, it was verified that the entire sample holder was properly covered.

Sample acquisition

The sample, at least three measurements were repeated to verify the reproducibility of the data. The replicates were then averaged to obtain a representative spectrum of the sample for characterization (Fig 14).

11. ISOTOPIC ABUNDANCE

Isotopic abundance analysis provides an atomic-level description of matter, highlighting the effects of isotopic substitution, such as the Geometric Isotope Effect (GIE) and the Kinetic Isotope Effect (KIE). The GIE involves changes in the geometric structure of molecules due to isotopic substitution, particularly affecting hydrogen bonding. These alterations can impact molecular geometry and influence physical, chemical, and biological properties. The KIE refers to the change in reaction rates caused by substituting isotopes in molecules. Isotopes have different masses, affecting bond vibrational energies and activation energies. The KIE is categorized into Primary KIE, where substitution directly affects the reaction's rate-determining step, and Secondary KIE, where substitution influences the reaction rate indirectly through changes in molecular geometry or electronic effects. Both effects are crucial for understanding isotopic influences on molecular behaviour.

Isotopic abundance analysis was first performed on Batch 1 of Product C

The sample was sent to Istituto San Michele all'Adige (Fondazione Edmund Mach) and tested for stable isotopes as follows:

- $\delta 18O$: Method PDP 7011:2010 REV. 0 (TC-IRMS), UNIT ‰ vs V-SMOW.
- $\delta 13C$: Method PDP 7009:2017 REV. 2 (EA-IRMS), UNIT vs ‰ V-PDB
- $\delta 15N$: Method PDP 7009:2017 REV. 2 (EA-IRMS), UNIT ‰ vs V-AIR.
- $\delta 34S$: Method PDP 7013:2010 REV. 0 (EA-IRMS), UNIT ‰ vs V-CDT.
- 14C- activity was also tested by Chelab (Tentamus Company):
- 14C-activity: Method ISO-16620-2;2015 (AMS), UNIT % modern carbon (pMC).

The results were as follows.

| Product | δ18O (‰) | δ13C (‰) | δ15N (‰) | | δ34S (‰) | | |
|---|---|---|---|---|---|---|---|
| | | | | | | | |
| Product C batch 1 (23D2227) | 29,2 ± 1 | -14,8 ± 0,3 | 4,8 ± 0,6 | 5,4 ± 1 | | | |

Thus, an isotopic characterization was carried out Product C Batch 1. In addition, the 14C activity was carried out on the Product C Batch 1 formulation excluding the raw materials that provide calcium carbonate. The measured value for the 14C activity of the sample of 99.82 ± 0,22 % percent modern carbon (pMC) corresponds to those for substances from purely bio-based carbon. There is no evidence of a synthetic source in the analyzed material.

BIODEGRADABILITY TEST ACCORDING TO OECD 301F:1992

Biodegradation is the breakdown of organic substances by microorganisms into simple natural components like $CO_2$, $H_2O$, and $NH_3$. Evaluating the biodegradability of chemicals is important in environmental risk assessment. Ready biodegradation tests (RBT), proposed by the OECD, are used to assess biodegradability by incubating chemicals in a mineral medium with microorganisms. Over 28 days, metabolic parameters like oxygen consumption and CO2 production are monitored. A chemical is considered readily biodegradable if it passes an RBT. Primary biodegradation refers to structural changes in a substance due to biological processes, which can be measured through chemical analysis. For example, the OECD 301F method evaluates biodegradability by measuring oxygen consumption in a respirometer under controlled conditions. The results are expressed as the percentage of oxygen consumed compared to theoretical oxygen demand (ThOD) or chemical oxygen demand (COD).

### Reagents and substances

This method involves working with the following reagents and substances:

A) Test substance Product C batch 1 in 100 mg/l dilution in mineral medium;
B) Mineral medium for solubilization of the test substance consisting of the following 4 solutions (A, B, C and D) brought to 1L with ultrapure water:

- 10 ml Solution A: 8.50 g KH2PO4 + 21.75 g K2HPO4 + 33.40g Na2HPO4 di-hydrate + 0.50 g NH4Cl brought to 1 lt with ultrapure water and with final pH at 7.4;
- 1 ml Solution B: 27.50g CaCl2 anhydrous made to 1 lt with ultrapure water;
- 1 ml Solution C: 22.50g MgSO4 heptahydrate brought to 1 lt with ultrapure water;
- 1 ml Solution D: 0.25g FeCl3 hexahydrate brought to 1 lt with ultrapure water.

C) Bacterial inoculum The test substance was added to appropriate inoculum and obtained by taking activated sludge in equal aliquots.
D) Chemical Standard for BOD determination at 5 to 28 days consisting of various solutions of Sodium Acetate anhydrous analytical purity.

### Assay execution

### Sample preparation

The sample was treated according to the procedure reported in the respirometric-manometric method no. 301F (OECD). To be able to have the correct registration by using the BOD sensor and enough material to perform the planned instrumental analysis. Sodium acetate was used as the reference substance. Tests were done at a constant temperature of 22 C.

### Inoculum preparation

The inoculum was prepared using activated sludge from different locations, mixed in equivalent volumes. The inoculum is oxygenated, stirred, and fed with glucose, peptone, and dibasic potassium phosphate, while the values of redox potential, oxygen consumption, and total dry matter are monitored daily. The dry matter was determined at 100°C to ensure the same quantity (30 mg/mL) in the vessels containing the test substance.

### Inoculum composition

The inoculum was obtained by mixing active sludge. The assembled inoculum was oxygenated, stirred and fed with glucose, peptone and monopotassium orthophosphate. Oxygen, redox, and total suspended solid values were monitored daily. Before the use of inoculum, the total dry matter is determined.
The composition of the microfauna was determined by optical microscope analysis.

### Reference substance

Manometric respirometric method also requires the conduct of another test with ultrapure water fortified with a standard (sodium acetate) to assess proper performance and instrumental reliability.

### Blank

Blank analysis was performed on inoculated mineral media to assess the contribution of the liquid and inoculum to the BOD value of the finished product.

Conditions of the Assay:

The containers are placed inside thermostat refrigerators set at 22±2°C and kept in constant agitation by mechanical movement of the anchor; all this is carried out for 28 days by measuring every 6h automatically and via wireless the oxygen depression value, which at 28 days will be the absolute biodegradability value of the sample under investigation.

Test Results

Product C batch 1:

| Sample name | Analysis duration [d] | Sampling time [h] | Last value of BOD [mg/l] | Average BOD [mg/l] | SD BOD [mg/l] |
|---|---|---|---|---|---|
| Product C 100mg-L m.resp.+ incoc. Test 1 | 28 | 6 | 51 | 51,25 | 1,8 |
| Product C 100mg-L m.resp.+ incoc. Test 2 | 28 | 6 | 51 | | |
| Product C 100mg-L m.resp.+ incoc. Test 3 | 28 | 6 | 49,3 | | |
| Product C 100mg-L m.resp.+ incoc. Test 4 | 28 | 6 | 53,7 | | |

Blank:

| name | Analysis duration [d] | Sampling time [h] | Last value of BOD [mg/l] | Average BOD [mg/l] | SD BOD [mg/l] |
|---|---|---|---|---|---|
| Mineral medium + inoculum Test 1 | 28 | 6 | 11 | 11,1 | 0,1 |
| Mineral medium + inoculum Test 2 | 28 | 6 | 11,2 | | |

The test shows a contribution to BOD between inoculum and mineral medium of 11.1 mg/l, a value that should be used for BOD correction of Product C batch1 100 mg/l mixture.
The mean value falls within the positive range of the test, which is between 10 and 50 mg/l.

Reference substance:

| Sample | ThOD (mg/l) | BOD (ppm) | | | | |
|---|---|---|---|---|---|---|
| | | Day 1 | Day 7 | Day 14 | Day 21 | Day 28 |
| Sodium Acetate 50 mg/L | 31 | 10,8 | 27,8 | 28,9 | 29,4 | 30,8 |

The positivity of the test results from the following evidence:

A) The obtained value (30,8 ppm Biochemical Oxygen Demand, BOD) of the chemical standard is within the positivity range of the test, which is 31±5 ppm Theoretical demand of Oxygen (ThOD) so the chemical control was positive.
B) After only 7 days, the BOD value is 89,68% of the total;
C) After 14 gg the BOD value is 93,22% of the total.
D) After 28 gg the BOD value stands at 99,35% of the total

The reference substance reached the threshold value within 14 days. It can therefore be stated that the test was correctly performed and that the ready biodegradation data collected on the substance under examination are reliable

The calculation of biodegradation is done at each sampling time for the reference substance, test sample and blank.

Total biodegradation of sample has been calculated using the equation:

BOD: (average of mg/L of $O_2$ consumed of the test substance) - (average of mg/L of $O_2$ consumed of the blank)/mg/L test substance in the container.

There is no evidence of nitrite and nitrate production, based on that no correction for nitrification/denitrification will be performed.

- mg/l $O_2$ consumed by the test substance: 51,25 mg/L.
- mg/l $O_2$ consumed by the blank: 11,1 mg/L.
- mg/l $O_2$ consumed by nitrification in the product : /
- mg/l test chemical in the container: 100 mg/L

BOD average calculated = 0,402 mg of $O_2$ for mg test substance.

In order to calculate the percentage degradation and thus the ready biodegradation, Chemical Oxygen Demand (COD) was evaluated by hot acid dichromate oxidation in ultrapure deionized water; tests were conducted on a 50 mg/l of Abo11 solution obtaining cod of 67.1 mg/l.

$$COD = (mg/l \ O_2 \ consumed \ by \ the \ test \ substance) \ / \ (mg/l \ test \ substance \ in \ the \ container)$$

$$COD = 0,467 \ mg \ of \ O_2 \ for \ mg \ of \ test \ substance.$$

Average of percentage degradation at 28 days: BOD average corrected by blank deduction/ COD pure substance = (0.402/0.467) *100 = 86,08%

Interpretation of results

Validity Criteria

The test is considered valid when:

- the average biodegradation rate of the reference substance is above 60% after 14 days of incubation;
- the difference of the extremes of the replicate values at the plateau at the end of the test is less than 20%;
- the oxygen demand of the blank is not more than 60 mg O2/L.

Interpretation

A substance is considered readily biodegradable when the level of biodegradation reached within 10 days after the start of degradation, considered to be the time when 10% of the substance has been degraded (10-day window), exceeds 60% and in addition the level of biodegradation reached at 28 days is >60%.

Results

- Validity criteria of the test are satisfied.
- The assessment regarding the biodegradability of the substance Product C bach 1 at concentration 100 mg/l is that during the conducted experiment the product showed ready biodegradability under the conditions applied in the manometric respirometric test developed according to ec regulation 440/2008 updated to reg. 640/2012 - part c: methods for determining ecotoxicity - method c.4. Part v - (method c.4-d) + OECD 301F:1992; in fact, the product exceeded 60% (62%) biodegradation within ten days after reaching 10%, meeting the validity criteria of the method.
- Product C batch 1 at 100 mg/L concentration showed no toxic effects on the activity of microorganisms at the tested concentration reaching 86,06% of the associated theoretical COD value and thus showing well-present biotic activity.

The substance under examination, tested at a concentration of 100 mg/L, was found to be "readily biodegradable" under aerobic conditions according to the conditions of the MANOMETRIC RESPIROMETRIC TEST (OECD 301F, EC REGULATION 440/2008 and subsequent updates, Method C.4-D). Indeed, the substance exceeded 60% biodegradation within ten days following the attainment of 10% degradation.

12. NETWORK ANALYSIS

A network analysis of the pathological state treated or ameliorated by product C was performed, the data obtained show how the natural matrices-based products tested are able to influence the body on a systemic scale.

Product C

- the condition in a pathological state (Figure 12 Panel A): dysfunctional and inflamed adipose tissue leads to an imbalance in bone homeostasis, negatively affecting the competition for mesenchymal stem cell reserves to induce osteoblast, osteoclast or adipocyte differentiation, and unphysiologically shifting this phenomenon towards osteoclast differentiation. This leads to a reduction in the number of mature osteoblasts, resulting in an inability to ensure proper mineralisation of the cellular matrix with loss of bone activity. In conditions of dysregulation of lipid metabolism and inflammation of the adipose tissue, a dysfunctional loop is established between the adipose tissue and the bone, leading to the accumulation of adipocytes and osteoclasts, disadvantaging the osteoblastic component and aggravating bone fragility. Bone is also an organ with endocrine activities and can therefore influence events in other tissues on a systemic level, for example through the secretion of osteocalcin (OCN), which promotes insulin secretion by the pancreas, insulin sensitivity in peripheral organs, such as muscle, and regulation of overall energy expenditure (Fukumoto S, Martin TJ. Bone as an endocrine organ. Trends Endocrinol Metab. 2009 Jul;20(5):230-6. Doi: 10.1016/j.tem.2009.02.001. Epub 2009 Jun 21. PMID: 19546009).
- the condition when treated with drug reference (Figure 12 Panel B): drug reference is only able to reduce the quantity of the adipocytes.
- the state when treated with product C (Figure 12 Panel C): product C, by interacting with the mesenchymal stem pool in bone and adipose tissue, is able to recapitulate all the necessary elements to restore the correct bone turnover, which is beneficial for the formation of a solid and functional bone structure, and, at a systemic level, to restore the balance of metabolic dysregulation and reduce inflammation.

13. DETAILED ANALYSIS OF THREE DIFFERENT BATCHES OF PRODUCT

To appreciate whether the final matrix constituting product C is characterized by the matrix effect, a series of analyses to grasp the product's features on different aspects was carried out on the three batches 1, 2 and 3 previously described. Figure 13 a and b shows that the 3 batches have the same desired therapeutic/beneicial effect on the In vitro cell based assay. A targeted metabolomics analysis capable of identifying most of such molecular components, was carried out on the vegetal matrices component of different batches of the product (see table below) together with the other analysis reported herein.

The product, as described above, consist of vegetal and other natural matrices assembled and resulting in a final new natural matrix. Several analytical techniques have been used to identify and quantify compounds belonging to the main classes present in plants so to assess the composition of the vegetal matrix component of the product. Although metabolomic analysis does not allow to appreciate the dynamic changes within the component of the matrix, it allows a "picture" of the composition in the moment the analysis is carried out.

Each individual plant metabolite was specifically researched through "targeted metabolomics". This analysis allows to capture a frame on the qualitative data, by determining the chemical compounds present in the material, and quantitative data, by defining the concentrations of each compound in the material.

For product C, a qualitative and quantitative characterization of as many primary and secondary metabolites as possible was carried out using an "omic" approach, the targeted metabolomics analysis, based on the use of multiple analytical methodologies. The analytical methods used for the chemical characterization of each batch are described below. The most appropriate analytical techniques have been adopted based on the chemical nature of the classes of compounds present. The analysis with chromatographic methods combined with different detection techniques (e.g., GC and LC each combined with a suitable detector), made it possible to identify and quantify, as appropriate, the organic compounds. The inductively coupled plasma analysis using a single quadrupole mass spectrometer (ICP-MS) or an optical emission spectrometer (ICP-OES) made it possible to establish the levels of elements present, while the anions were determined by ion chromatography and conductivity detector.

Batch 1: 23D2227

Batch 2: 24F0699

Batch 3: 23K3123

| METHOD | COMPOUNDS | Product C_23D2227 (%) | Product C_24F0699 (%) | Product C_23K3123 (%) | Product C_24F0699 (d%) | Product C_23K3123 (d%) |
|---|---|---|---|---|---|---|
| A | **MACROMOLECULES > 20000 (POLYSACCHARIDES, Buffer Method), Total** | 2,6828 | 2,643 | 2,5638 | 1,48 | 4,44 |
| | **PHENOLS, Total** | 0,0337903 | 0,03535877 | 0,03260014 | 4,64 | 3,52 |
| | **FLAVONOIDS, Total** | 0,0319882 | 0,0319143 | 0,02919242 | 0,23 | 8,74 |
| | **ANTHOCYANIDINS, Total** | 0,0072838 | 0,0060925 | 0,0056607 | 16,36 | 22,28 |
| B | Delphinidin | 0,0072838 | 0,0060925 | 0,0056607 | 16,36 | 22,28 |
| | **FLAVANONES, Total** | < LoQ | < LoQ | < LoQ | / | / |
| B | Hesperetin | ND | <LoQ | ND | / | / |
| B | Hesperidin | <LoQ | <LoQ | <LoQ | / | / |
| B | Naringenin & Pinobanksin (AS: Naringenin) | <LoQ | <LoQ | <LoQ | / | / |
| B | Naringenin-7-O-glucoside | <LoQ | <LoQ | <LoQ | / | / |
| B | **FLAVONES, Total** | 0,0099425 | 0,009862 | 0,00855152 | 0,81 | 13,99 |
| B | Genkwanin | <LoQ | <LoQ | <LoQ | / | / |
| B | Luteolin-4'-O-glucoside & Kaempferol-3-O-glucoside & Quercitrin (AS: Luteolin-4'-O-glucoside) | 0,0022669 | 0,0019026 | 0,0016835 | 16,07 | 25,74 |
| B | Luteolin-7-O-Rutinoside | <LoQ | <LoQ | <LoQ | / | / |
| B | Nobiletin | <LoQ | <LoQ | 0,00023362 | / | / |
| B | Orientin & Homoorientin (AS: Homoorientin) | <LoQ | <LoQ | <LoQ | / | / |
| B | Scutellarein tetramethyl ether | <LoQ | <LoQ | <LoQ | / | / |
| B | Sinensetin | <LoQ | <LoQ | <LoQ | / | / |
| B | Tangeretin | <LoQ | <LoQ | <LoQ | / | / |
| B | Vicenin-2 | 0,0076756 | 0,0079594 | 0,0066344 | 3,70 | 13,57 |
| B | **FLAVONOLS, Total** | 0,0147619 | 0,0159598 | 0,0149802 | 8,11 | 1,48 |
| B | Quercetin | ND | <LoQ | <LoQ | / | / |
| B | Quercetin-3-O-glucopyranoside (Isoquercetin) | 0,012504 | 0,014211 | 0,013361 | 13,65 | 6,85 |
| B | Quercetin-3-O-rutinoside-7-O-glucoside | <LoQ | <LoQ | <LoQ | / | / |
| B | Rutin | 0,0022579 | 0,0017488 | 0,0016192 | 22,55 | 28,29 |
| | **PHENOLIC ACIDS, Total** | < LoQ | < LoQ | < LoQ | / | / |

(continued)

| METHOD | COMPOUNDS | Product C_23D2227 (%) | Product C_24F0699 (%) | Product C_23K3123 (%) | Product C_24F0699 (d%) | Product C_23K3123 (d%) |
|---|---|---|---|---|---|---|
| B | alpha-Resorcylic acid & Protocatechuic acid (AS: Protocatechuic acid) | <LoQ | <LoQ | <LoQ | / | / |
| | **PHENYLPROPANOIDS, Total** | 0,0018021 | 0,00344447 | 0,00340772 | 91,14 | 89,10 |
| | **HYDROXYCINNAMIC ACIDS, Total** | 0,0018021 | 0,00344447 | 0,00340772 | 91,14 | 89,10 |
| B | Caffeic acid | <LoQ | 0,0011784 | 0,0015044 | / | / |
| B | Cryptochlorogenic acid & Chlorogenic acid (AS: Chlorogenic acid) | 0,0018021 | 0,0014587 | 0,0012007 | 19,06 | 33,37 |
| B | Ferulic acid | <LoQ | <LoQ | <LoQ | / | / |
| B | Isoacteoside | <LoQ | <LoQ | <LoQ | / | / |
| B | Neochlorogenic acid | <LoQ | 0,00080737 | <LoQ | / | / |
| B | Verbascoside | <LoQ | <LoQ | 0,00070262 | / | / |
| | **MONOLIGNOLS, Total** | < LoQ | < LoQ | < LoQ | / | / |
| B | Eleutheroside B | <LoQ | <LoQ | <LoQ | / | / |
| | **TANNINS, Total** | < LoQ | < LoQ | < LoQ | / | / |
| | **TANNIN MONOMERS, Total** | < LoQ | < LoQ | < LoQ | / | / |
| | **CONDENSED TANNINS MONOMERS, Total** | < LoQ | < LoQ | < LoQ | / | / |
| B | Catechin | <LoQ | <LoQ | <LoQ | / | / |
| | **TERPENES, Total** | 0,0222 | 0,0495 | 0,0368 | 122,97 | 65,77 |
| | **TRITERPENES, Total** | 0,0222 | 0,0495 | 0,0368 | 122,97 | 65,77 |
| | **PHYTOSTEROLS, Total** | 0,0222 | 0,0495 | 0,0368 | 122,97 | 65,77 |
| NEOTRON: HPLC-DAD | Ergosterol | 0,0222 | 0,0495 | 0,0368 | 122,97 | 65,77 |
| | **ORGANIC ACIDS, Total** | 8,39616 | 9,25083 | 9,0538 | 10,81 | 7,83 |
| | **MONOCARBOXYLIC ACIDS, Total** | < LoQ | < LoQ | < LoQ | / | / |
| C | Acetic acid | <LoQ | <LoQ | <LoQ | / | / |
| C | Lactic acid | <LoQ | <LoQ | <LoQ | / | / |
| | **DICARBOXYLIC ACIDS, Total** | 2,22076 | 2,42403 | 2,463 | 41,25 | 10,91 |
| C | Fumaric acid | 0,02916 | 0,04463 | 0,0291 | 53,05 | 0,21 |
| C | Malic acid | 2,1916 | 2,3794 | 2,4339 | 8,57 | 11,06 |
| C | Succinic acid | <LoQ | <LoQ | <LoQ | / | / |
| C | Tartaric acid | <LoQ | <LoQ | <LoQ | / | / |
| | **TRICARBOXYLIC ACIDS, Total** | 6,1754 | 6,8268 | 6,5908 | 10,55 | 6,73 |

(continued)

| METHOD | COMPOUNDS | Product C_23D2227 (%) | Product C_24F0699 (%) | Product C_23K3123 (%) | Product C_24F0699 (d%) | Product C_23K3123 (d%) |
|---|---|---|---|---|---|---|
| C | Citric acid | 6,1754 | 6,8268 | 6,5908 | 10,55 | 6,73 |
| | **SUGARS AND DERIVATIVES, Total** | 5,09549892 | 5,12836238 | 5,56704215 | 0,64 | 9,25 |
| | **MONOSACCHARIDES, Total** | 4,53164501 | 4,32757649 | 4,96553461 | 4,50 | 9,57 |
| D | Arabinose | <LoQ | <LoQ | <LoQ | / | / |
| D | Fructose | 2,21704215 | 1,95615311 | 2,36415214 | 11,77 | 6,64 |
| D | Galactose | <LoQ | <LoQ | <LoQ | / | / |
| D | Glucose | 2,31460286 | 2,37142338 | 2,60138248 | 2,45 | 12,39 |
| D | Mannose | <LoQ | <LoQ | <LoQ | / | / |
| D | Rhamnose | <LoQ | <LoQ | <LoQ | / | / |
| | **DISACCHARIDES, Total** | 0,56385391 | 0,80078588 | 0,60150754 | 124,88 | 6,68 |
| D | Lactose | <LoQ | <LoQ | <LoQ | / | / |
| D | Maltose | <LoQ | <LoQ | <LoQ | / | / |
| D | Sucrose | 0,56385391 | 0,80078588 | 0,60150754 | 42,02 | 6,68 |
| | **VITAMINS, Total** | 0,19148 | 0,25138 | 0,27496 | 31,28 | 43,60 |
| | **WATER SOLUBLE VITAMINS, Total** | 0,19148 | 0,25138 | 0,27496 | 31,28 | 43,60 |
| C | Ascorbic acid (Vitamin C) | 0,19148 | 0,25138 | 0,27496 | 31,28 | 43,60 |
| | **LIPIDS, Total** | < LoQ | < LoQ | < LoQ | / | / |
| | **FATTY ACIDS, Total** | < LoQ | < LoQ | < LoQ | / | / |
| E | Propionic acid | <LoQ | <LoQ | <LoQ | / | / |
| | **INORGANIC COMPOUNDS, Total** | 28,9817119 | 30,2765749 | 30,0347991 | 4,47 | 3,63 |
| | **ANIONS, Total** | 0,33454945 | 0,40160428 | 0,40745345 | 20,04 | 21,79 |
| F2 | Nitrate | <LoQ | <LoQ | <LoQ | / | / |
| F2 | Nitrite | <LoQ | <LoQ | <LoQ | / | / |
| F2 | Phosphate | 0,10151375 | 0,14300744 | 0,15248746 | 40,87 | 50,21 |
| F2 | Sulfate | 0,2330357 | 0,25859684 | 0,25496599 | 10,97 | 9,41 |
| | **MACROELEMENTS, Total** | 28,5455538 | 29,7835143 | 29,5246912 | 4,34 | 3,39 |
| F1 | Calcium | 27,1786219 | 28,1880826 | 28,0007519 | 3,71 | 3,02 |
| F2 | Chloride | 0,09940538 | 0,12997901 | 0,14231791 | 30,76 | 43,17 |
| F1 | Magnesium | 0,29963747 | 0,3068775 | 0,321387 | 2,42 | 7,26 |
| F1 | Phosphorus | 0,16727613 | 0,1327407 | 0,1606743 | 20,65 | 3,95 |
| F1 | Potassium | 0,741467 | 0,95538437 | 0,82954567 | 28,85 | 11,88 |
| F1 | Sodium | 0,05914598 | 0,07045016 | 0,07001442 | 19,11 | 18,38 |
| | **MICROELEMENTS, Total** | 0,01321692 | 0,00895736 | 0,01340341 | 32,23 | 1,41 |
| F1 | Chromium | 0,00029394 | 0,00033827 | 0,00038005 | 15,08 | 29,29 |
| F1 | Cobalt | < LoQ | < LoQ | < LoQ | / | / |

(continued)

| METHOD | COMPOUNDS | Product C_23D2227 (%) | Product C_24F0699 (%) | Product C_23K3123 (%) | Product C_24F0699 (d%) | Product C_23K3123 (d%) |
|---|---|---|---|---|---|---|
| F1 | Copper | 0,00034911 | 0,00031235 | 0,00043177 | 10,53 | 23,68 |
| F2 | Iodide | <LoQ | <LoQ | <LoQ | / | / |
| F1 | Iron | 0,01004836 | 0,00563629 | 0,01000417 | 43,91 | 0,44 |
| F1 | Manganese | 0,00153219 | 0,00181277 | 0,00160505 | 18,31 | 4,75 |
| F1 | Molybdenum | < LoQ | < LoQ | < LoQ | / | / |
| F1 | Nickel | 0,00026527 | 0,00025844 | 0,00027865 | 2,57 | 5,04 |
| F1 | Selenium | < LoQ | < LoQ | < LoQ | / | / |
| F1 | Tin | < LoQ | < LoQ | < LoQ | / | / |
| F1 | Vanadium | 0,00010867 | 0,00011214 | 0,00011181 | 3,20 | 2,89 |
| F1 | Zinc | 0,00061937 | 0,00048708 | 0,00059191 | 21,36 | 4,43 |
| | **OTHER ELEMENTS, Total** | 0,0883917 | 0,08249897 | 0,08925107 | 6,67 | 0,97 |
| F1 | Aluminum | 0,01264075 | 0,00822972 | 0,01317842 | 34,90 | 4,25 |
| F1 | Antimony | < LoQ | < LoQ | < LoQ | / | / |
| F1 | Arsenic | < LoQ | < LoQ | < LoQ | / | / |
| F1 | Barium | 0,0031083 | 0,00215448 | 0,00316116 | 30,69 | 1,70 |
| F1 | Boron | 0,00073173 | 0,00067518 | 0,00065108 | 7,73 | 11,02 |
| F2 | Bromide | <LoQ | <LoQ | <LoQ | / | / |
| F1 | Cadmium | < LoQ | < LoQ | < LoQ | / | / |
| F1 | Gadolinium | < LoQ | < LoQ | < LoQ | / | / |
| F1 | Gallium | < LoQ | < LoQ | < LoQ | / | / |
| F1 | Gold | < LoQ | < LoQ | < LoQ | / | / |
| F1 | Iridium | < LoQ | < LoQ | < LoQ | / | / |
| F1 | Lead | < LoQ | < LoQ | < LoQ | / | / |
| F1 | Lithium | < LoQ | < LoQ | < LoQ | / | / |
| F1 | Lutetium | < LoQ | < LoQ | < LoQ | / | / |
| F1 | Mercury | < LoQ | < LoQ | < LoQ | / | / |
| F1 | Palladium | < LoQ | < LoQ | < LoQ | / | / |
| F1 | Platinum | < LoQ | < LoQ | < LoQ | / | / |
| F1 | Rubidium | 0,00098574 | 0,00111759 | 0,00107559 | 13,38 | 9,11 |
| F1 | Ruthenium | < LoQ | < LoQ | < LoQ | / | / |
| F1 | Strontium | 0,02223713 | 0,02255957 | 0,02192484 | 1,45 | 1,40 |
| F1 | Tellurium | < LoQ | < LoQ | < LoQ | / | / |
| F1 | Thallium | < LoQ | < LoQ | < LoQ | / | / |
| F1 | Thorium | < LoQ | < LoQ | < LoQ | / | / |
| F1 | Titanium | 0,04868804 | 0,04776243 | 0,04925998 | 1,90 | 1,17 |
| F1 | Uranium | < LoQ | < LoQ | < LoQ | / | / |

(continued)

| METHOD | COMPOUNDS | Product C_23D2227 (%) | Product C_24F0699 (%) | Product C_23K3123 (%) | Product C_24F0699 (d%) | Product C_23K3123 (d%) |
|---|---|---|---|---|---|---|
| F1 | Ytterbium | < LoQ | < LoQ | < LoQ | / | / |

*Note 1. Gray boxes indicate chemical macro classes.*
*Note 2. %= compound concentration expressed as percentage of the whole matrix.*
*Note 3. (d%) = percentage deviation: (|Reference 23D2227 (%)-Test(%)|/(Reference 23D2227 (%) ) x 100).*
*Note 4. The term "Total" refers to the sum of the value of the various compounds which forms the corresponding group.*
*Note 5. <LdQ= below the limit of quantification.*
*Note 6. /= percentage deviation not quantifiable*

Qualitative miRNA characterization was also performed on ultracentrifuged samples from each batch and the results show high metabolomic complexity together with the presence of miRNAs typical of living matter.

| miRNA Family | miRNA | Batch 1 | Batch 2 | Batch 3 |
|---|---|---|---|---|
| miR166 | ath-miR166a-3p | x | x | x |
| | ath-miR166a-5p | | x | |
| | ath-miR166b-3p | x | x | x |
| | ath-miR166b-5p | | x | |
| | ath-miR166c | x | x | x |
| | ath-miR166d | x | x | x |
| | ath-miR166e-3p | x | x | x |
| | ath-miR166e-5p | | x | |
| | ath-miR166f | x | x | x |
| | ath-miR166g | x | x | x |
| miR8175 | pseudomiR8175 1 | x | | |
| | pseudomiR8175 2 | | x | |
| | pseudomiR8175 3 | x | x | |
| | pseudomiR8175 4 | x | x | x |
| | pseudomiR8175 5 | x | x | |
| | pseudomiR8175 6 | x | x | |
| | pseudomiR8175 7 | x | x | |
| | pseudomiR8175 8 | x | x | x |

The results obtained showed an appreciable composition variability of each vegetal matrix component of each batch and underline the impossibility to recapitulate the properties of the matrix as the sum of its single components. The work performed and reported herein (see cell-based assay results) together with the data below, demonstrates that the biological effect elicited by product cannot be recapitulated by the sum of the effects elicited by the single molecular components but is the result of interconnections and interactions among the components: the matrix effect. This translates into the impossibility to formally define a structure-activity relationship (SAR) according to the principles canonically applied to APIs.

The results of the targeted metabolomics highlighted more or less marked quantitative fluctuations of the individual chemical and the miRNA classes in the vegetal matrix component of the 3 batches of product C. These fluctuations, if taken as the reference parameter, would lead to an a priori expectation for these batches to have a different therapeutic or beneficial action. The analysis summarised in figure 13 demonstrates that, despite the biological activity is maintained across all the different batches assessed, none of the single molecular components identified would respect the criteria set for a single API, thus demonstrating that the matrix cannot be considered as a compilation of APIs.

As seen above, the same therapeutic action is preserved in every batch (functional resilience).

The product, through a physiological mechanism of action is capable to evoke, in biological systems, the same reaction relevant for the intended use.

This also highlights the fact that there are both structural and functional redundancy mechanisms maintained in products comprising or consisting of natural matrices, of a functional resilience (reaching the same result notwithstanding the individual differences among individuals of a same species) that is typical of the living matter.

Isotopic Abundance of all batches

In order to evaluate the isotopic ratio between different batches of Product C the analysis was performed on batches prepared from different starting materials. The samples were sent to Istituto San Michele all'Adige (Fondazione Edmund Mach) and tested for stable isotopes as follows:

- $\delta 18O$: Method PDP 7011:2010 REV. 0 (TC-IRMS), UNIT ‰ vs V-SMOW.
- $\delta 13C$: Method PDP 7009:2017 REV. 2 (EA-IRMS), UNIT vs ‰ V-PDB
- $\delta 15N$: Method PDP 7009:2017 REV. 2 (EA-IRMS), UNIT ‰ vs V-AIR.
- $\delta 34S$: Method PDP 7013:2010 REV. 0 (EA-IRMS), UNIT ‰ vs V-CDT.

The results were as follows.

$\delta$ ratio of the main isotopes of the Product C batches:

| Product | $\delta 18O$ | $\delta 13C$ (‰) | $\delta 15N$ (%o) | $\delta 34S$ (‰) |
|---|---|---|---|---|
| | | | | |
| **Product C batch 1 (23D2227)** | 29,2 $\pm$ 1 | -14,8 $\pm$ 0,3 | 4,8 $\pm$ 0,6 | 5,4 $\pm$ 1 |
| **Product C batch 2 (23K3123)** | 28,3 $\pm$ 1 | -14,9 $\pm$ 0,3 | 5,2 $\pm$ 0,6 | 5,3 $\pm$ 1 |
| **Product C batch 3 (24F0699)** | 28,5 $\pm$ 1 | -14,7 $\pm$ 0,3 | 5,8 $\pm$ 0,63 | 5,0 $\pm$ 1 |

The values $\delta 18O$, $\delta 15N$, $\delta 34S$ and $\delta 13C$ overlap between batches showing high reproducibility of the production process according to conservation of this parameter.

14. DEFINITION OF ARBITRARY UNIT OF PRODUCT C ACTIVITY (U-PRODUCT C):

The analysis of the reproducibility criteria of natural products led to the inventors focus on the reproducibility of the biological effect of the product. Through the analysis of different batches, significant chemical differences from batch to batch. However, notwithstanding said differences, the batches showed functional resilience, falling within the concept of maintaining biological activity. This approach is based on the principle of redundancy (functional resilience), ensuring that the product retains its biological efficacy despite variations. As a result, the product must be regulated in terms of units and is distinguished by a non-pharmacological mechanism. This places it outside the traditional concept of qSAR, highlighting a unique physiologic nature in its mechanism of action.

Product is a 100% natural, biodegradable matrix enriched with miRNA and exosomes derived from different batches. Said features are better managed by applying to product posology and batch release policy the concept of arbitrary unit of activity rather than weight or volume of a classical API.

A unit of Product C (U- Product C) is defined as one-tenth of the amount of product required to induce variations in the state of hADMSC, measured at the hallmark level using the method and adhering to the limits defined in figures 10, 11 and 13 or in the method of assessing whether a therapeutic product exerts its effect to treat a pathological state through a physiological mechanism of action disclosed in the specification..

In the case of batch 1, 10 U- Product C correspond to 50.4 mg of Product C, dissolved in 4 ml according to the experimental parameters described in the Methods and Materials section.

**Claims**

1. A method for determining the presence of native natural intelligence in a therapeutic or beneficial product, said product comprising or consisting of natural matrices, through the validation of its therapeutic or beneficial emerging properties, the method comprising the following steps

a. assessing the product or composition naturality by:

1. measuring the 14C activity in said product or composition with ISO-16620-2;2015 (AMS) method,
2. assessing the presence of miRNAs in said product or composition,
3. assessing the presence of exosomes in said product or composition,

b. assessing the presence of therapeutic or beneficial functional resilience between different batches of said product by comparing, batch to batch, the modulation of one or more biological activities underlying the product's desired therapeutic or beneficial effect on a relevant altered physiological state and/or on the pathological condition treated by said product or composition in a cell-based assay whose read-out is representative of the modulation of said one or more biological activities;
c. assessing from the read-out of said cell-based assay, whether the modulation of said biological activities underlying the desired therapeutic or beneficial effect results in the modulation of a whole altered physiological state or pathological condition; and

determining that said product or composition is itself a natural matrix, representing native natural intelligence, wherein native natural intelligence is present when
the measured value for the 14C activity is of $99.82 \pm 0.22$ % percent,
miRNAs, exosomes and therapeutic or functional resilience are detected, and
said modulation in c. results in the modulation of a whole altered physiological state or pathological condition.

2. The method of claim 1 further comprising: (1) providing a list of hallmarks representative of said altered metabolism and/or pathological state; (2) identifying for each of said hallmarks one or more biological activities modifications underlying said pathological state thereby pinpointing a network of biological activities whose modulation concurs to said pathological state and (3) identifying one or more parameters whose modulation concurs to the modulation of said one or more biological activities underlying the therapeutic effect of the product tested and determining the modulation trend in terms of up or down modulation of said one or more biological activities, in said network, concurring to said pathological state or to a healthy state.

3. The method of anyone of claims 1 or 2 wherein said altered physiological state is bone metabolism and/or said pathological condition is osteoporosis and said hallmarks are selected from: remodelling of bone, osteopenia, differentiation of osteoblasts, mineralization of bones, reduction of inflammation, reduction of adipose tissues.

4. The method according to claims 2 or 3 wherein the biological activities of (2) for the remodelling of bone hallmark are selected from the biological activities depicted in figure 9.

**Patentansprüche**

1. Verfahren zum Bestimmen des Vorhandenseins nativer natürlicher Intelligenz in einem therapeutischen oder vorteilhaften Produkt, das Produkt umfassend oder bestehend aus natürlichen Matrices, über die Validierung seiner therapeutischen oder vorteilhaften auftretenden Eigenschaften, das Verfahren umfassend die folgenden Schritte

a. Bewerten der Produkt- oder Zusammensetzungsnatürlichkeit durch:

1. Messen der 14C-Aktivität in dem Produkt oder der Zusammensetzung mit Verfahren ISO-16620-2;2015 (AMS),
2. Bewerten des Vorhandenseins von miRNAs in dem Produkt oder der Zusammensetzung,
3. Bewerten des Vorhandenseins von Exosomen in dem Produkt oder der Zusammensetzung,

b. Bewerten des Vorhandenseins einer therapeutischen oder vorteilhaften funktionellen Resilienz zwischen verschiedenen Chargen des Produkts durch Vergleichen, Charge zu Charge, der Modulation einer oder mehrerer biologischer Aktivitäten, die der gewünschten therapeutischen oder vorteilhaften Wirkung des Produkts auf einen relevanten veränderten physiologischen Status und/oder auf den pathologischen Zustand zugrunde liegen, die durch das Produkt oder die Zusammensetzung behandelt werden, in einem zellbasierten Assay, dessen Auslesung repräsentativ für die Modulation der einen oder der mehreren biologischen Aktivitäten ist;
c. Bewerten, von der Auslesung des zellbasierten Assays, ob die Modulation der biologischen Aktivitäten, die der gewünschten therapeutischen oder vorteilhaften Wirkung zugrunde liegen, zu der Modulation eines gesamten veränderten physiologischen Status oder pathologischen Zustands führt; und

Bestimmen, dass das Produkt oder die Zusammensetzung selbst eine natürliche Matrix, die die native natürliche Intelligenz repräsentiert, ist, wobei die native natürliche Intelligenz vorhanden ist, wenn der Messwert für die 14C-Aktivität 99,82 $\pm$ 0,22 % Prozent beträgt, miRNAs, Exosomen und therapeutische oder funktionelle Resilienz erfasst werden, und die Modulation in c. zu der Modulation eines gesamten veränderten physiologischen Status oder pathologischen Zustands führt.

**2.** Verfahren nach Anspruch 1, ferner umfassend: (1) Bereitstellen einer Liste von Kennzeichen, die für den veränderten Stoffwechsel und/oder pathologischen Status repräsentativ sind; (2) Identifizieren, für jedes der Kennzeichen, einer oder mehrerer Modifikationen der biologischen Aktivitäten, die dem pathologischen Status zugrunde liegen, wobei dadurch ein Netzwerk biologischer Aktivitäten lokalisiert wird, dessen Modulation mit dem pathologischen Status zusammenfällt, und (3) Identifizieren eines oder mehrerer Parameter, deren Modulation mit der Modulation der einen oder der mehreren biologischen Aktivitäten, die der therapeutischen Wirkung des getesteten Produkts zugrunde liegen, zusammenfällt, und Bestimmen des Modulationstrends in Bezug auf eine Modulation nach oben oder unten der einen oder der mehreren biologischen Aktivitäten in dem Netzwerk, die mit dem pathologischen Status oder mit einem gesunden Status zusammenfallen.

**3.** Verfahren nach einem der Ansprüche 1 oder 2, wobei der veränderte physiologische Status ein Knochenstoffwechsel ist und/oder der pathologische Zustand eine Osteoporose ist und die Kennzeichen ausgewählt sind aus: Knochenumbau, Osteopenie, Differenzierung von Osteoblasten, Mineralisierung von Knochen, Verringerung von Entzündungen, Verringerung von Fettgeweben.

**4.** Verfahren nach Anspruch 2 oder 3, wobei die biologischen Aktivitäten von (2) für den Knochenumbaukennzeichen aus den biologischen Aktivitäten, die in Figur 9 dargestellt sind, ausgewählt sind.

**Revendications**

**1.** Procédé permettant de déterminer la présence d'intelligence naturelle native dans un produit thérapeutique ou bénéfique, ledit produit comprenant des matrices naturelles ou en étant constitué, par la validation de ses propriétés émergentes thérapeutiques ou bénéfiques, le procédé comprenant les étapes suivantes

a. l'évaluation de la naturalité du produit ou de la composition par :

1. la mesure de l'activité du 14C dans ledit produit ou ladite composition avec la méthode de la norme ISO-16620-2 ;2015 (AMS),
2. l'évaluation de la présence de miARN dans ledit produit ou ladite composition,
3. l'évaluation de la présence d'exosomes dans ledit produit ou ladite composition,

b. l'évaluation de la présence d'une résilience fonctionnelle thérapeutique ou bénéfique entre différents lots dudit produit en comparant, lot à lot, la modulation d'une ou plusieurs activités biologiques sous-jacentes à l'effet thérapeutique ou bénéfique souhaité du produit sur un état physiologique modifié pertinent et/ou sur l'affection pathologique traitée par ledit produit ou ladite composition dans un essai cellulaire dont la lecture est représentative de la modulation de ladite ou desdites activités biologiques ;
c. le fait d'évaluer, à partir de la lecture dudit essai cellulaire, si la modulation desdites activités biologiques sous-jacentes à l'effet thérapeutique ou bénéfique souhaité entraîne la modulation d'un état physiologique ou d'une affection pathologique modifié dans son ensemble ; et

le fait de déterminer que ledit produit ou ladite composition est lui-même ou elle-même une matrice naturelle, représentant une intelligence naturelle native, dans lequel une intelligence naturelle native est présente lorsque la valeur mesurée pour l'activité du 14C est de 99,82 $\pm$ 0,22 %, des miARN, des exosomes, et une résilience thérapeutique ou fonctionnelle sont détectés, et ladite modulation en c. entraîne la modulation d'un état physiologique ou d'une affection pathologique modifié dans son ensemble.

**2.** Procédé selon la revendication 1, comprenant en outre : (1) la fourniture d'une liste de caractéristiques représentatives dudit métabolisme et/ou de ladite affection pathologique modifié ; (2) l'identification, pour chacune desdites caractéristiques, d'une ou plusieurs modifications d'activités biologiques sous-jacentes audit état pathologique, permettant ainsi de cerner un réseau d'activités biologiques dont la modulation concourt audit état pathologique, et (3) l'identification d'un ou plusieurs paramètres dont la modulation concourt à la modulation de ladite ou desdites activités biologiques sous-jacentes à l'effet thérapeutique du produit testé et la détermination de la tendance de modulation en termes de modulation à la hausse ou à la baisse de ladite ou desdites activités biologiques, dans ledit réseau, concourant audit état pathologique ou à un état sain.

**3.** Procédé selon l'une quelconque des revendications 1 ou 2, dans lequel ledit état physiologique modifié est un métabolisme osseux et/ou ladite affection pathologique est l'ostéoporose et lesdites caractéristiques sont choisies parmi : remodelage osseux, ostéopénie, différenciation des ostéoblastes, minéralisation des os, réduction de l'inflammation, réduction des tissus adipeux.

**4.** Procédé selon les revendications 2 ou 3, dans lequel les activités biologiques de (2) pour le remodelage de caractéristique osseuse sont choisies parmi les activités biologiques représentées sur la figure 9.

Fig 1

Fig 2

Fig 3

Fig 4

Fig 5

Fig 6

Fig 7

Fig 8

| OSTEOPOROSIS | | STATE OF THE ART | |
|---|---|---|---|
| Hallmarks of the pathology | Biological Activities | Pathological state modulation trend | Healthy physiological state |
| Remodelling of bone | Skeletal and Muscular System Development and Function → Remodelling → **Remodelling of bone** | ⬆ | ⬇ |
| | Skeletal and Muscular System Development and Function → Remodelling → **Resorption of bone** | ⬆ | ⬇ |
| | **SOST** | ⬆ | ⬇ |
| Osteopenia | Skeletal and Muscular Disorders → Osteopenia → **Osteopenia** | ⬆ | ⬇ |
| Differentiation of osteoblasts | Tissue Development → Differentiation → **Differentiation of mesenchymal stem cells** | ⬇ | ⬆ |
| | Connective Tissue Development and Function → Formation → **Formation of osteoblasts** | ⬇ | ⬆ |
| | Skeletal and Muscular System Development and Function → Quantity → **Quantity of bone cells** | ⬇ | ⬆ |
| | Skeletal and Muscular System Development and Function → Quantity → **Quantity of osteoblasts** | ⬇ | ⬆ |
| | RUNX2 | ⬇ | ⬆ |

Fig 9a

| OSTEOPOROSIS | | STATE OF THE ART | |
|---|---|---|---|
| Hallmarks of the pathology | Biological Activities | Pathological state modulation trend | Healthy physiological state |
| Mineralization | Skeletal and Muscular System Development and Function → Bone mineral density → **Bone mineral density** | ↓ | ↑ |
| | **Osteocalcin** | ↓ | ↑ |
| | **BMP8A** | ↓ | ↑ |
| Reduction of inflammation | Inflammatory Response → Inflammation → **Inflammation of adipose tissue** | ↑ | ↓ |
| | Inflammatory Response → Inflammation → **Inflammation of connective tissue** | ↑ | ↓ |
| | Inflammatory Response → Systemic inflammation → **Systemic inflammation** | ↑ | ↓ |
| | Inflammatory Response → Inflammation → **Inflammation of body cavity** | ↑ | ↓ |
| | Inflammatory Response → Inflammation → **Inflammation of soft tissue** | ↑ | ↓ |
| Reduction of adipose tissue | Endocrine System Development and Function → Glucose tolerance → **Glucose tolerance** | ↓ | ↑ |
| | Connective Tissue Development and Function → Quantity → **Quantity of adipocytes** | ↑ | ↓ |

Fig 9b

| OSTEOPOROSIS | | STATE OF THE ART | | TREATMENT | |
| --- | --- | --- | --- | --- | --- |
| | | | | CONTROL | PRODUCT SAMPLE |
| Hallmarks of the pathology | Biological Activities | Pathological state modulation trend | Healthy physiological state | Control Group | Product C |
| Remodelling of bone | Skeletal and Muscular System Development and Function → Remodelling → **Remodelling of bone** | ↑ | ↓ | 0 | -0.20 |
| | Skeletal and Muscular System Development and Function → Remodelling → **Resorption of bone** | ↑ | ↓ | 0 | -0.20 |
| | **SOST** | ↑ | ↓ | 0 | -0.30 |
| Osteopenia | Skeletal and Muscular Disorders → Osteopenia → **Osteopenia** | ↑ | ↓ | 0 | -0.20 |
| Differentiation of osteoblasts | Tissue Development → Differentiation → **Differentiation of mesenchymal stem cells** | ↓ | ↑ | 0 | 0.19 |
| | Connective Tissue Development and Function → Formation → **Formation of osteoblasts** | ↓ | ↑ | 0 | 0.21 |
| | Skeletal and Muscular System Development and Function → Quantity → **Quantity of bone cells** | ↓ | ↑ | 0 | 0.23 |
| | Skeletal and Muscular System Development and Function → Quantity → **Quantity of osteoblasts** | ↓ | ↑ | 0 | 0.22 |
| | **RUNX2** | ↓ | ↑ | 0 | 0.30 |

Fig 10a

| OSTEOPOROSIS | | STATE OF THE ART | | TREATMENT | |
| --- | --- | --- | --- | --- | --- |
| | | | | CONTROL | PRODUCT SAMPLE |
| Hallmarks of the pathology | Biological Activities | Pathological state modulation trend | Healthy physiological state | Control Group | Product C |
| Mineralization | Skeletal and Muscular System Development and Function → Bone mineral density → **Bone mineral density** | ⬇ | ⬆ | 0 | 0.15 |
| | **Osteocalcin** | ⬇ | ⬆ | 0 | 0.15 |
| | **BMP8A** | ⬇ | ⬆ | 0 | 0.30 |
| Reduction of inflammation | Inflammatory Response → Inflammation → **Inflammation of adipose tissue** | ⬆ | ⬇ | 0 | -0.31 |
| | Inflammatory Response → Inflammation → **Inflammation of connective tissue** | ⬆ | ⬇ | 0 | -0.31 |
| | Inflammatory Response → Systemic inflammation → **Systemic inflammation** | ⬆ | ⬇ | 0 | -0.18 |
| | Inflammatory Response → Inflammation → **Inflammation of body cavity** | ⬆ | ⬇ | 0 | -0.26 |
| | Inflammatory Response → Inflammation → **Inflammation of soft tissue** | ⬆ | ⬇ | 0 | -0.32 |
| Reduction of adipose tissue | Endocrine System Development and Function → Glucose tolerance → **Glucose tolerance** | ⬇ | ⬆ | 0 | 0.20 |
| | Connective Tissue Development and Function → Quantity → **Quantity of adipocytes** | ⬆ | ⬇ | 0 | -0.20 |

Fig 10b

| OSTEOPOROSIS | | STATE OF THE ART | | TREATMENT | | |
|---|---|---|---|---|---|---|
| | | | | CONTROL | PRODUCT SAMPLE | REFERENCE DRUG |
| Hallmarks of the pathology | Biological Activities | Pathological state modulation trend | Healthy physiological state | Control Group | Product C | DIBASE |
| Remodelling of bone | Skeletal and Muscular System Development and Function → Remodelling → **Remodelling of bone** | ⬆ | ⬇ | 0 | -0.20 | 0 |
| | Skeletal and Muscular System Development and Function → Remodelling → **Resorption of bone** | ⬆ | ⬇ | 0 | -0.20 | 0 |
| | **SOST** | ⬆ | ⬇ | 0 | -0.30 | 0.30 |
| Osteopenia | Skeletal and Muscular Disorders → Osteopenia → **Osteopenia** | ⬆ | ⬇ | 0 | -0.20 | 0.15 |
| Differentiation of osteoblasts | Tissue Development → Differentiation → **Differentiation of mesenchymal stem cells** | ⬇ | ⬆ | 0 | 0.19 | 0 |
| | Connective Tissue Development and Function → Formation → **Formation of osteoblasts** | ⬇ | ⬆ | 0 | 0.21 | -0.32 |
| | Skeletal and Muscular System Development and Function → Quantity → **Quantity of bone cells** | ⬇ | ⬆ | 0 | 0.23 | -0.15 |
| | Skeletal and Muscular System Development and Function → Quantity → **Quantity of osteoblasts** | ⬇ | ⬆ | 0 | 0.22 | -0.15 |
| | RUNX2 | ⬇ | ⬆ | 0 | 0.30 | 0 |

Fig 11a

| OSTEOPOROSIS | | STATE OF THE ART | | TREATMENT | | |
| | | | | CONTROL | PRODUCT SAMPLE | REFERENCE DRUG |
| Hallmarks of the pathology | Biological Activities | Pathological state modulation trend | Healthy physiological state | Control Group | Product C | DIBASE |
|---|---|---|---|---|---|---|
| Mineralization | Skeletal and Muscular System Development and Function → Bone mineral density → **Bone mineral density** | ↓ | ↑ | 0 | 0.15 | -0.20 |
| | Osteocalcin | ↓ | ↑ | 0 | 0.15 | -0.20 |
| | BMP8A | ↓ | ↑ | 0 | 0.30 | 0 |
| Reduction of inflammation | Inflammatory Response → Inflammation → **Inflammation of adipose tissue** | ↑ | ↓ | 0 | -0.31 | 0.15 |
| | Inflammatory Response → Inflammation → **Inflammation of connective tissue** | ↑ | ↓ | 0 | -0.31 | 0 |
| | Inflammatory Response → Systemic inflammation → **Systemic inflammation** | ↑ | ↓ | 0 | -0.18 | 0.22 |
| | Inflammatory Response → Inflammation → **Inflammation of body cavity** | ↑ | ↓ | 0 | -0.26 | 0 |
| | Inflammatory Response → Inflammation → **Inflammation of soft tissue** | ↑ | ↓ | 0 | -0.32 | 0.20 |
| Reduction of adipose tissue | Endocrine System Development and Function → Glucose tolerance → **Glucose tolerance** | ↓ | ↑ | 0 | 0.20 | 0 |
| | Connective Tissue Development and Function → Quantity → **Quantity of adipocytes** | ↑ | ↓ | 0 | -0.20 | -0.30 |

Fig 11b

Fig 12A

## Panel B

## Panel C

Fig 12 B and C

| OSTEOPOROSIS | | STATE OF THE ART | | TREATMENT | | | |
| | | | | CONTROL | PRODUCT SAMPLE | | |
| Hallmarks of the pathology | Biological Activities | Pathological state modulation trend | Healthy physiological state | Control Group | Product C batch 1 | Product C batch 2 | Product C batch 3 |
|---|---|---|---|---|---|---|---|
| Remodelling of bone | Skeletal and Muscular System Development and Function → Remodelling → **Remodelling of bone** | ⬆ | ⬇ | 0 | -0.20 | -0.22 | -0.20 |
| | Skeletal and Muscular System Development and Function → Remodelling → **Resorption of bone** | ⬆ | ⬇ | 0 | -0.20 | -0.24 | -0.20 |
| | SOST | ⬆ | ⬇ | 0 | -0.30 | -0.30 | -0.33 |
| Osteopenia | Skeletal and Muscular Disorders → Osteopenia → **Osteopenia** | ⬆ | ⬇ | 0 | -0.20 | -0.19 | -0.21 |
| Differentiation of osteoblasts | Tissue Development → Differentiation → **Differentiation of mesenchymal stem cells** | ⬇ | ⬆ | 0 | 0.19 | 0.23 | 0.20 |
| | Connective Tissue Development and Function → Formation → **Formation of osteoblasts** | ⬇ | ⬆ | 0 | 0.21 | 0.25 | 0.21 |
| | Skeletal and Muscular System Development and Function → Quantity → **Quantity of bone cells** | ⬇ | ⬆ | 0 | 0.23 | 0.23 | 0.27 |
| | Skeletal and Muscular System Development and Function → Quantity → **Quantity of osteoblasts** | ⬇ | ⬆ | 0 | 0.22 | 0.21 | 0.24 |
| | RUNX2 | ⬇ | ⬆ | 0 | 0.30 | 0.32 | 0.33 |

Fig 13a

| OSTEOPOROSIS | | STATE OF THE ART | | TREATMENT | | | |
|---|---|---|---|---|---|---|---|
| | | | | CONTROL | PRODUCT SAMPLE | | |
| Hallmarks of the pathology | Biological Activities | Pathological state modulation trend | Healthy physiological state | Control Group | Product C batch 1 | Product C batch 2 | Product C batch 3 |
| Mineralization | Skeletal and Muscular System Development and Function → Bone mineral density → Bone mineral density | ↓ | ↑ | 0 | 0.15 | 0.17 | 0.16 |
| | Osteocalcin | ↓ | ↑ | 0 | 0.15 | 0.20 | 0.20 |
| | BMP8A | ↓ | ↑ | 0 | 0.30 | 0.34 | 0.32 |
| Reduction of inflammation | Inflammatory Response → Inflammation → Inflammation of adipose tissue | ↑ | ↓ | 0 | -0.31 | -0.35 | -0.32 |
| | Inflammatory Response → Inflammation → Inflammation of connective tissue | ↑ | ↓ | 0 | -0.31 | -0.30 | -0.34 |
| | Inflammatory Response → Systemic inflammation → Systemic inflammation | ↑ | ↓ | 0 | -0.18 | -0.20 | -0.19 |
| | Inflammatory Response → Inflammation → Inflammation of body cavity | ↑ | ↓ | 0 | -0.26 | -0.29 | -0.26 |
| | Inflammatory Response → Inflammation → Inflammation of soft tissue | ↑ | ↓ | 0 | -0.32 | -0.35 | -0.34 |
| Reduction of adipose tissue | Endocrine System Development and Function → Glucose tolerance → Glucose tolerance | ↓ | ↑ | 0 | 0.20 | 0.22 | 0.23 |
| | Connective Tissue Development and Function → Quantity → Quantity of adipocytes | ↑ | ↓ | 0 | -0.20 | -0.24 | -0.22 |

Fig. 13b

Fig. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016024503 A1, KALLURI RAGHU

**Non-patent literature cited in the description**

- **MATTOLI LUISA et al.** Natural complex substances: From molecules to the molecular complexes. Analytical and technological advances for their definition and differentiation from the corresponding synthetic substances. *PHYTOCHEMISTRY*, 22 July 2023, vol. 215

- **ROMAGNOLI et al.** In Vitro Behaviour of Human Adipose Tissue-Derived Stem Cells on Poly($\varepsilon$-caprolactone) Film for Bone Tissue Engineering Applications. *BioMed Research International*, 2015, vol. 2015, 12, https://doi.org/10.1155/2015/323571
- **FUKUMOTO S ; MARTIN TJ**. Bone as an endocrine organ. *Trends Endocrinol Metab*, July 2009, vol. 20 (5), 230-6